# EUROPEAN PATENT SPECIFICATION

(11) **EP 1 141 017 B1**
(45) Date of publication and mention of the grant of the patent: **10.09.2008**
(21) Application number: 99964281.2
(22) Date of filing: 20.12.1999
(51) Int. Cl.: C07K 14/705, C12N 15/12, C12Q 1/68, C12N 5/10, C07K 16/28, G01N 33/53, A61K 38/17

(54) **CHARACTERIZATION OF THE SOC/CRAC CALCIUM CHANNEL PROTEIN FAMILY**
CHARAKTERISIERUNG DER PROTEINFAMILIE VON SOC/CRAC KALZIUMKANÄLEN
CARACTERISATION D'UNE FAMILLE DE CANAUX CALCIQUES

(30) Priority: 30.12.1998 US 114220 P; 29.01.1999 US 120018 P; 22.06.1999 US 140415 P
(43) Date of publication of application: 10.10.2001
(73) Proprietor: Beth Israel Deaconess Medical Center, Inc., Boston, MA 02215 (US)
(72) Inventor: SCHARENBERG, Andrew, M., Lexington, MA 02420 (US)
(74) Representative: HOFFMANN EITLE
(86) International application number: PCT/US1999/029996
(87) International publication number: WO 2000/040614

(56) References cited:
- WO-A-98/15657
- WO-A-98/37093
- WO-A-99/09166
- WO-A-99/09199
- DATABASE GENEMBL [Online] 16 February 1998 (1998-02-16) STRAUSBERG,R.: "ob70f05.s1 NCI_CGAP_GCB1 Homo sapiens cDNA clone IMAGE:1336737 3', mRNA sequence" XP002138823
- DATABASE GENEMBL [Online] 10 July 1998 (1998-07-10) MARRA ET AL.: "ub28d10.r1 Soares 2NbMT Mus musculus cDNA clone IMAGE:1379059 5' mRNA sequence" XP002149803
- DATABASE GENEMBL [Online] 19 July 1997 (1997-07-19) STRAUSBERG, R.: "ni64e11.s1 NCI_CGAP_Pr12 Homo sapiens cDNA clone IMAGE:981644 mRNA sequence" XP002148641
- DATABASE GENEMBL [Online] 18 November 1997 (1997-11-18) STRAUSBERG, R.: "nt76b07.s1 NCI_CGAP_Pr3 Homo sapiens cDNA clone IMAGE:1204405, mRNA" XP002148642
- DATABASE GENEMBL [Online] 30 November 1998 (1998-11-30) SHIMIZU, N.: "Homo sapiens mRNA complete cds." XP002148643 -& NAGAMINE ET AL.: "Molecular cloning of a novel putative Ca2+ channel protein (TRPC7) highly expressed in brain" GENOMICS, vol. 54, 15 November 1998 (1998-11-15), pages 124-131, XP000938744
- ZHU, XI ET AL: "Molecular cloning of a widely expressed human homologue for the Drosophila trp gene." FEBS LETTERS, (1995) VOL. 373, NO. 3, PP. 193-198., XP000907241
- HUNTER JOHN J ET AL: "Chromosomal localization and genomic characterization of the mouse melastatin gene (Mlsn1)." GENOMICS NOV. 15, 1998, vol. 54, no. 1, 15 November 1998 (1998-11-15), pages 116-123, XP000910696 ISSN: 0888-7543 cited in the application
- WES PAUL D ET AL: "TRPC1, a human homolog of a Drosophila store-operated channel." PROCEEDINGS OF THE NATIONAL ACADEMY OF SCIENCES OF THE UNITED STATES OF AMERICA 1995, vol. 92, no. 21, 1995, pages 9652-9656, XP002138820 ISSN: 0027-8424
- ZHU, XI ET AL: "Trp, A novel mammalian gene family essential for agonist-activated capacitative Ca-2+ entry." CELL, vol. 85, no. 5, 1996, pages 661-671, XP000907242
- GARCIA REYNALDO L ET AL: "Differential expression of mammalian TRP homologues across tissues and cell lines." BIOCHEMICAL AND BIOPHYSICAL RESEARCH COMMUNICATIONS 1997, vol. 239, no. 1, 1997, pages 279-283, XP002138822 ISSN: 0006-291X
- SINKINS WILLIAM G ET AL: "Functional expression of TrpC1: A human homologue of the Drosophila Trp channel." BIOCHEMICAL JOURNAL APRIL, 1998, vol. 331, no. 1, April 1998 (1998-04), pages 331-339, XP000864583 ISSN: 0264-6021
- PREUSS KLAUS-DIETER ET AL: "Expression and characterization of a trpl homolog from rat." BIOCHEMICAL AND BIOPHYSICAL RESEARCH COMMUNICATIONS NOV. 7, 1997, vol. 240, no. 1, 7 November 1997 (1997-11-07), pages 167-172, XP002138821 ISSN: 0006-291X
- OBUKHOV, ALEXANDER G. ET AL: "Direct activation of trpl cation channels by G-alpha-11 subunits." EMBO (EUROPEAN MOLECULAR BIOLOGY ORGANIZATION) JOURNAL, (1996) VOL. 15, NO. 21, PP. 5833-5838., XP000907243
- SCHARENBERG A M ET AL: "MLSN-1/SOC-1 defines a widely expressed Ca2+/cation channel family involved in Ca2+ homeostasis and store-operated Ca2+ signaling." FIFTY-THIRD ANNUAL MEETING OF THE SOCIETY OF GENERAL PHYSIOLOGISTS;WOODS HOLE, MASSACHUSETTS, USA; SEPTEMBER 9-11, 1999, vol. 114, no. 1, July 1999 (1999-07), page 14a XP000910708 Journal of General Physiology July, 1999 ISSN: 0022-1295

## Description

### Field of the Invention

This invention relates to nucleic acids coding for a novel family of calcium channel polypeptides, the encoded polypeptides, unique fragments of the foregoing, and methods of making and using same.

### Background of the Invention

Calcium channels are membrane-spanning, multi-subunit proteins that facilitate the controlled transport ("flux") of Ca²⁺ ions into and out of cells. Cells throughout the animal kingdom, and at least some bacterial, fungal and plant cells, possess one or more types of calcium channels. In general, "excitable" cells, such as neurons of the central nervous system, peripheral nerve cells, and muscle cells, including those of skeletal muscles, cardiac muscles, and venous and arterial smooth muscles, possess voltage-dependent calcium channels. In a voltage-dependent calcium channel, the transport of Ca²⁺ ions into and out of the cells requires a certain minimal level of depolarization (the difference in potential between the inside of the cell bearing the channel and the extracellular environment) with the rate of Ca²⁺ cell flux dependent on the difference in potential. In "non-excitable" cells, calcium influx is thought to occur predominantly in response to stimuli which cause the release of calcium from intracellular stores. This process, termed *store operated calcium influx*, is not well understood.

Characterization of a particular type of calcium channel by analysis of whole cells is complicated by the presence of mixed populations of different types of calcium channels in the majority of cells. Although single-channel recording methods can be used to examine individual calcium channels, such analysis does not reveal information related to the molecular structure or biochemical composition of the channel. Furthermore, in this type of analysis, the channel is isolated from other cellular constituents that might be important for the channel's natural functions and pharmacological interactions. To study the calcium channel structure-function relationship, large amounts of pure channel protein are needed. However, acquiring large amounts of pure protein is difficult in view of the complex nature of these multisubunit proteins, the varying concentrations of calcium channel proteins in tissue sources, the presence of mixed populations of calcium channel proteins in tissues, and the modifications of the native protein that can occur during the isolation procedure.

### Summary of the Invention

The invention is based on the identification of a novel family of calcium channel polypeptides and the molecular cloning and partial characterization of a novel member of this family that is expressed predominantly in human hematopoietic cells, liver, and kidney. This newly identified family of calcium channel polypeptides is designated, "SOC" or "CRAC" or "ICRAC", for Store Operated Channels or Calcium Release Activated Channels. Although not wishing to be bound to any particular theory or mechanism, it is believed that the SOC/CRAC calcium channel polypeptides are transmembrane polypeptides that modulate Ca²⁺ flux "into" and "out of" a cell, for example, in certain instances they may be activated upon depletion of Ca²⁺ from intracellular calcium stores, allowing Ca²⁺ influx into the cell. Accordingly, the compositions disclosed herein are believed to be useful for modulating calcium transport into and out of such intracellular stores and for the treatment of disorders that are characterized by aberrant calcium transport into and out of such intracellular stores. In particular, we believe that the SOC/CRAC calcium channel polypeptides disclosed herein play an important role in the influx of extracellular calcium by mediating the refilling of intracellular calcium stores following their depletion. Accordingly, we believe that the compositions for expressing functional SOC/CRAC calcium channel polypeptides in cells, as disclosed herein, are useful for treating patients having conditions that are characterized by reduced extracellular calcium influx into their SOC/CRAC-expressing cells. Additionally, the compositions of the invention are useful for delivering therapeutic and/or imaging agents to cells which preferentially express SOC/CRAC calcium channel polypeptides and, in particular, for delivering such agents to hematopoietic cells, liver, heart, spleen, and kidney to modulate proliferation and growth of these cells. Moreover, in view of the importance of cellular calcium levels to cell viability, we believe that SOC-2/CRAC-1, SOC-3/CRAC-2, and SOC-4/CRAC-3 as disclosed herein, and/or other members of the SOC/CRAC family of calcium channel polypeptides, represent an ideal target for designing and/or identifying (e.g., from molecular libraries) small molecule inhibitors that block lymphocyte proliferation, as well as other binding agents that selectively bind to SOC/CRAC polypeptides to which drugs or toxins can be conjugated for delivery to SOC/CRAC polypeptide expressing cells.

The invention is based, in part, on the molecular cloning and sequence analysis of the novel SOC/CRAC calcium channel molecules disclosed herein (also referred to as a "SOC-2/CRAC-1 molecule," a "SOC-3/CRAC-2 molecule," and/or "SOC-4/CRAC-3 molecule") that are predominantly expressed in human hematopoietic cells, liver, spleen, heart, and kidney (SOC-2/CRAC-1), kidney and colon (SOC-3/CRAC-2), and prostate (SOC-4/CRAC-3 molecule). As used herein, a "SOC/CRAC molecule" embraces a "SOC/CRAC calcium channel nucleic acid" (or "SOC/CRAC nucleic acid") and a "SOC/CRAC calcium channel polypeptide" (or "SOC/CRAC polypeptide"). Homologs and alleles also are embraced within the meaning of a SOC/CRAC calcium channel molecule.

According to one aspect of the invention, isolated SOC/CRAC nucleic acids which code for one or more member(s) of the SOC/CRAC family of calcium channel polypeptides are provided. The isolated nucleic acids refer to one or more of the following:
(a) nucleic acid molecules selected from the group consisting of SEQ ID NO:25 or SEQ ID NO:29, and which code for a SOC/CRAC polypeptide;
(b) nucleic acid molecules that differ from the nucleic acid molecules of (a) or (b) in codon sequence due to the degeneracy of the genetic code.

Described are isolated nucleic acid molecules selected from the group consisting of (a) a unique fragment of a nucleic acid molecule selected from the group consisting of SEQ ID NO:1, SEQ ID NO:3, SEQ ID NO:5, SEQ ID NO:7, SEQ ID NO:23, SEQ ID NO:25, SEQ ID NO:29, and SEQ ID NO:31, (b) complements of (a), provided that the unique fragment includes a sequence of contiguous nucleotides which is not identical to any sequence selected from a sequence group consisting of (1) sequences having the SEQ. ID NOS. or GenBank accession numbers of Table I, (2) complements of (1), and (3) fragments of (1) and (2).

According to yet another aspect of the invention, isolated SOC/CRAC polypeptides are provided. The isolated SOC/CRAC polypeptide molecules are encoded by one or more SOC/CRAC nucleic acid molecules of the invention. Preferably, the SOC/CRAC'polypeptide contains one or more polypeptides selected from the group consisting of the polypeptides having SEQ. ID Nos. 26, 30. In other embodiments, the isolated polypeptide may be a fragment or variant of the foregoing SOC/CRAC polypeptide molecules of sufficient length to represent a sequence unique within the human genome, and identifying with a polypeptide that functions as a calcium channel, provided that the fragment excludes a sequence of contiguous amino acids identified in Table II, and/or excludes a sequence of contiguous amino acids encoded for by a nucleic acid sequence identified in Table I. In another embodiment, immunogenic fragments of the polypeptide molecules described above are provided.

According to another aspect, isolated SOC/CRAC binding agents (e.g., polypeptides) are described which selectively bind to a SOC/CRAC molecule (e.g., a SOC/CRAC polypeptide encoded by the isolated nucleic acid molecules of the invention). Preferably, the isolated binding agents selectively bind to a polypeptide which comprises the sequence selected from the group consisting of SEQ ID NO:2, SEQ ID NO:4, SEQ ID NO:6, SEQ ID NO:8, SEQ ID NO:24, SEQ ID NO:26, SEQ ID NO:28, SEQ ID NO:30, and SEQ ID NO:32, or unique fragments thereof. In the preferred embodiments, the isolated binding polypeptides include antibodies and fragments of antibodies (e.g., Fab, F(ab)₂, Fd and antibody fragments which include a CDR3 region which binds selectively to a SOC/CRAC polypeptide). Preferably, the antibodies for human therapeutic applications are human antibodies.

According to another aspect of the invention, a pharmaceutical composition containing a pharmaceutically effective amount of an isolated SOC/CRAC nucleic acid, an isolated SOC/CRAC polypeptide, or an isolated SOC/CRAC binding polypeptide in a pharmaceutically acceptable carrier also is provided. The pharmaceutical compositions are useful in accordance with therapeutic methods disclosed herein.

According to yet another aspect of the invention, a method for isolating a SOC/CRAC molecule is provided. The method involves:
a) contacting a SOC/CRAC nucleic acid or a SOC/CRAC binding polypeptide with a sample that is believed to contain one or more SOC/CRAC molecules, under conditions to form a complex of the SOC/CRAC nucleic acid or the SOC/CRAC binding polypeptide and the SOC/CRAC molecule;
b) detecting the presence of the complex;
c) isolating the SOC/CRAC molecule from the complex; and
d) determining whether the isolated SOC/CRAC molecule has SOC/CRAC calcium channel activity. As used herein "SOC/CRAC calcium channel activity" refers to the transport of Ca²⁺ into and out of intracellular stores that is mediated by a SOC/CRAC polypeptide. In general, the SOC/CRAC calcium channel activity is initiated by a reduction or depletion of intracellular calcium stores.

Described is, the SOC/CRAC nucleic acid, e.g. SOC-2/CRAC-1 nucleic acid (e.g., a nucleic acid having SEQ. ID NO. 27, or complements thereof); in certain other embodiments, the SOC/CRAC nucleic acid is a SOC-3/CRAC-2 nucleic acid (e.g., a nucleic acid having SEQ ID NO. 29, or complements thereof); also described is a SOC-4/CRAC-3 nucleic acid (e.g., a nucleic acid having SEQ. ID NO. 31, or complements thereof). A SOC/CRAC polypeptide is a SOC-2/CRAC-1 binding polypeptide (e.g., an antibody that selectively binds to a SOC-2/CRAC-1 polypeptide). In yet further embodiments, the SOC/CRAC polypeptide is a SOC-3/CRAC-2 binding polypeptide (e.g., an antibody that selectively binds to a SOC-3/CRAC-2 polypeptide). Also described is the SOC/CRAC polypeptide is a SOC-4/CRAC-3 binding polypeptide (e.g., an antibody that selectively binds to a SOC-4/CRAC-3 polypeptide). Isolated binding polypeptides include antibodies and fragments of antibodies (e.g., Fab, F(ab)₂, Fd and antibody fragments which include a CDR3 region which binds selectively to a SOC-2/CRAC-1 (for comparative purposes), to a SOC-3/CRAC-2, and/or to a SOC-4/CRAC-3 (for comparative purposes) polypeptide). Preferably the isolated binding polypeptides or other binding agents selectively bind to a single SOC/CRAC molecule, i.e., are capable of distinguishing between different members of the SOC/CRAC family. Accordingly, one or more SOC/CRAC binding agents can be contained in a single composition (e.g., a pharmaceutical composition) to identify multiple SOC/CRAC molecules *in vivo* or *in vitro*.

According to yet another aspect of the invention, a method for identifying agents useful in the modulation of SOC/CRAC calcium channel activity is provided. The method involves:
a) contacting a SOC/CRAC polypeptide with a candidate agent suspected of modulating SOC/CRAC calcium channel activity, under conditions sufficient to allow the candidate agent to interact selectively with (e.g. bind to) the SOC/CRAC polypeptide;
b) detecting a Ca²⁺ concentration of step (b) associated with the SOC/CRAC calcium channel activity of the SOC/CRAC polypeptide in the presence of the candidate agent; and
c) comparing the Ca²⁺ concentration of step (b) with a control Ca²⁺ concentration of a SOC/CRAC polypeptide in the absence of the candidate agent to determine whether the candidate agent modulates (increases or decreases) SOC/CRAC calcium channel activity.

Also described is a method for identifying agents useful in the modulation of a SOC/CRAC polypeptide kinase activity is provided. The method involves:
a) contacting a SOC/CRAC polypeptide with kinase activity with a candidate agent suspected of modulating SOC/CRAC kinase activity, under conditions sufficient to allow the candidate agent to interact with the SOC/CRAC polypeptide and modulate its kinase activity;
b) detecting a kinase activity associated with the SOC/CRAC polypeptide in the presence of the candidate agent; and
c) comparing the kinase activity of step (b) with a control kinase activity of a SOC/CRAC polypeptide in the absence of the candidate agent to determine whether the candidate agent modulates (increases or decreases) SOC/CRAC kinase activity. In some embodiments the SOC/CRAC polypeptide comprises amino acids 999-1180 of the SOC-2/CRAC-1 polypeptide (SEQ ID NO:24), or a fragment thereof that retains the kinase activity.

According to yet another aspect of the invention, a method for determining the level of expression of a SOC/CRAC polypeptide in a subject is provided. The method involves:
a) measuring the expression of a SOC/CRAC polypeptide in a test sample, and
b) comparing the measured expression of the SOC/CRAC polypeptide in the test sample to the expression of a SOC/CRAC polypeptide in a control containing a known level of expression to determine the level of SOC/CRAC expression in the subject. Expression is defined as SOC/CRAC mRNA expression or SOC/CRAC polypeptide expression. Various methods can be used to measure expression. The preferred embodiments of the invention utilize PCR and Northern blotting for measuring mRNA expression, and monoclonal or polyclonal SOC/CRAC antisera as reagents for measuring SOC/CRAC polypeptide expression. In preferred embodiments, the SOC/CRAC molecule is SOC-3/CRAC-2. In certain embodiments, the test samples include biopsy samples and biological fluids such as blood. The method is useful, e.g., for assessing the presence or absence or stage of a proliferative disorder in a subject.

Also contemplated are kits comprising a package including assays for SOC/CRAC epitopes, SOC/CRAC nucleic acids, and instructions, and optionally related materials such as controls, for example, a number, color chart, or an epitope of the expression product of the foregoing isolated nucleic acid molecules of the invention for comparing, for example, the level of SOC/CRAC polypeptides or SOC/CRAC nucleic acid forms (wild-type or mutant) in a test sample to the level in a control sample having a known amount of a SOC/CRAC nucleic acid or SOC/CRAC polypeptide. This comparison can be used to assess in a subject a risk of developing a cancer or the progression of a cancer. The kits may also include assays for other known genes, and expression products thereof, associated with, for example, proliferative disorders (e.g., BRCA, p53, etc.). In a preferred embodiment, the kit comprises a package containing: (a) a binding agent that selectively binds to an isolated nucleic acid of the invention or an expression product thereof to obtain a measured test value, (b) a control containing a known amount of a SOC/CRAC nucleic acid or a SOC/CRAC polypeptide to obtain a measured control value, and (c) instructions for comparing the measured test value to the measured control value to determine the amount of SOC/CRAC nucleic acid or expression product thereof in a sample.

The invention provides isolated nucleic acid molecules, unique fragments thereof, expression vectors containing the foregoing, and host cells containing the foregoing. The invention also provides isolated binding polypeptides and binding agents which bind such polypeptides, including antibodies, and pharmaceutical compositions containing any of the compositions of the invention. The foregoing can be used, *inter alia*, in the diagnosis or treatment of conditions characterized by the aberrant expression levels and/or the presence of mutant forms of a SOC/CRAC nucleic acid or polypeptide. The invention also provides methods for identifying agents that alter the function of the SOC/CRAC polypeptide.

These and other aspects of the invention, as well as various advantages and utilities, will be more apparent with reference to the detailed description of the preferred embodiments.

### Brief Description of the Sequences

SEQ ID NO:1 is a partial nucleotide sequence of the human SOC-2/CRAC-1 cDNA.

SEQ ID NO:2 is the predicted amino acid sequence of the translation product of human SOC-2/CRAC-1 cDNA (SEQ ID NO:1).

SEQ ID NO:3 is a partial nucleotide sequence of the human SOC-2/CRAC-1 cDNA.

SEQ ID NO:4 is the predicted amino acid sequence of the translation product of human SOC-2/CRAC-1 cDNA (SEQ ID NO:3).

SEQ ID NO:5 is a partial nucleotide sequence of the human SOC-2/CRAC-1 cDNA.

SEQ ID NO:6 is the predicted amino acid sequence of the translation product of human SOC-2/CRAC-1 cDNA (SEQ ID NO:5).

SEQ ID NO:7 is a partial nucleotide sequence of the mouse homologue (mSOC-2/CRAC-1) of the human SOC-2/CRAC-1 cDNA.

SEQ ID NO:8 is the predicted amino acid sequence of the translation product of the mSOC-2/CRAC-1 cDNA (SEQ ID NO:7).

SEQ ID NO:9 is the nucleotide sequence of the mouse MLSN-1 (SOC-1) cDNA.

SEQ ID NO:10 is the predicted amino acid sequence of the translation product of the mouse MLSN-1 (SOC-1) cDNA (SEQ ID NO:9).

SEQ ID NO:11 is the nucleotide sequence of a human calcium channel cDNA with GenBank Acc. no.: AB001535.

SEQ ID NO:12 is the predicted amino acid sequence of the translation product of the human calcium channel cDNA with GenBank Acc. no.: AB001535 (SEQ ID NO:11).

SEQ ID NO:13 is the amino acid sequence of a *C*. *Elegans* polypeptide at the c05c12.3 locus.

SEQ ID NO:14 is the amino acid sequence of a *C*. *Elegans* polypeptide at the F54D1 locus.

SEQ ID NO:15 is the amino acid sequence of a *C*. *Elegans* polypeptide at the t01H8 locus.

SEQ ID NO:16 is the nucleotide sequence of a mouse kidney cDNA with GenBank Acc. no.: AI226731.

SEQ ID NO:17 is the predicted amino acid sequence of the translation product of the mouse kidney cDNA with GenBank Acc. no.: AI226731 (SEQ ID NO:16).

SEQ ID NO:18 is the nucleotide sequence of a human brain cDNA with GenBank Acc. no.: H18835.

SEQ ID NO:19 is the predicted amino acid sequence of the translation product of the human brain cDNA with GenBank Acc. no.: H18835 (SEQ ID NO:18).

SEQ ID NO:20 is the nucleotide sequence of the human EST with GenBank Acc. no.: AA419592.

SEQ ID NO:21 is the nucleotide sequence of the human EST with GenBank Acc. no.: AA419407.

SEQ ID NO:22 is the nucleotide sequence of the mouse EST with GenBank Acc. no.: AI098310.

SEQ ID NO:23 is a partial nucleotide sequence of the human SOC-2/CRAC-1 cDNA that contains the SOC-2/CRAC-1 sequences of SEQ ID NO:1, SEQ ID NO:3, and SEQ ID NO:5.

SEQ ID NO:24 is the predicted amino acid sequence of the translation product of human SOC-2/CRAC-1 cDNA (SEQ ID NO:23).

SEQ ID NO:25 is a partial nucleotide sequence of the human SOC-3/CRAC-2 cDNA.

SEQ ID NO:26 is the predicted amino acid sequence of the translation product of human SOC-3/CRAC-2 cDNA (SEQ ID NO:25).

SEQ ID NO:27 is the full nucleotide sequence of the human SOC-2/CRAC-1 cDNA.

SEQ ID NO:28 is the predicted amino acid sequence of the translation product of human SOC-2/CRAC-1 cDNA (SEQ ID NO:27).

SEQ ID NO:29 is the full nucleotide sequence of the human SOC-3/CRAC-2 cDNA.

SEQ ID NO:30 is the predicted amino acid sequence of the translation product of human SOC-3/CRAC-2 cDNA (SEQ ID NO:29).

SEQ ID NO:31 is the full nucleotide sequence of the human SOC-4/CRAC-3 cDNA.

SEQ ID NO:32 is the predicted amino acid sequence of the translation product of human SOC-4/CRAC-3 cDNA (SEQ ID NO:31).

### Brief Description of the Drawings

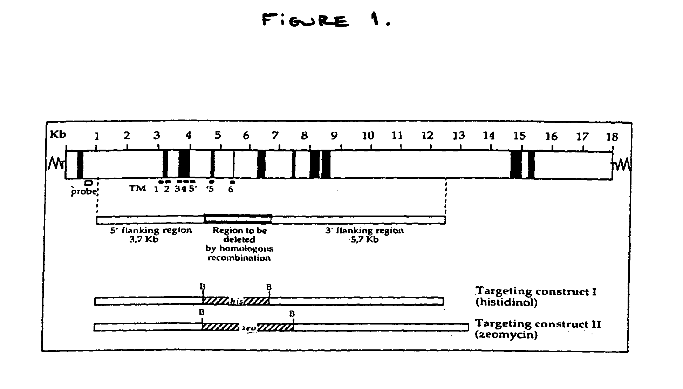

Figure 1 is a schematic depicting the intron/exon organization of the chicken SOC-2/CRAC-1 genomic sequence, as well as the putative transmembrane (TM) domains, and the targeting constructs utilized in the knockout experiments.

### Detailed Description of the Invention

One aspect of the invention involves the partial cloning of cDNAs encoding a member of a novel family of calcium channel polypeptides, referred to herein as "SOC/CRAC" (designated "SOC" or "CRAC" or "ICRAC", for Store Operated Channels or Calcium Release Activated Channels, or CECH). Although not intending to be bound to any particular mechanism or theory, we believe that a SOC/CRAC family member is a transmembrane calcium channel that modulates Ca²⁺ flux "into" and "out of a cell; in certain instances it may be activated upon depletion of Ca²⁺ from intracellular calcium stores, allowing Ca²⁺ influx into the cell.

The first three isolated SOC/CRAC members disclosed herein, define a new family of calcium channels which is distinct from previously described calcium channels, such as voltage gated calcium channels, ryanodine receptor/inositol-1,4,5-triphosphate receptor channels, and Transient Receptor Potential (TRP) channels. The SOC/CRAC family of calcium channels exhibits high selectivity (with a P_{Ca}/P_{Na} ratio near 1000), a unitary conductance below the detection level of the patch clamp method (the conductance estimated at approximately 0.2 picosiemens), and are subject to inhibition by high intracellular calcium levels. Although not intending to be bound to any particular mechanism or theory, we believe that SOC/CRAC calcium channels are responsible for the majority of, for example, calcium entry which occurs when intracellular calcium stores are depleted, and that SOC/CRAC currents are important for initiating various types of calcium-dependent processes. Thus, we believe that SOC/CRAC calcium channels play an important role in cellular calcium homeostasis by, e.g., modulating the supply of calcium to refill intracellular stores when depleted.

The isolated full-length sequence of a representative, first member of the SOC/CRAC family, human SOC/CRAC nucleic acid (cDNA), SOC-2/CRAC-1, is represented as the nucleic acid of SEQ ID NO:27. This nucleic acid sequence codes for the SOC-2/CRAC-1 polypeptide with the predicted amino acid sequence disclosed herein as SEQ ID NO:28. A homologous mouse cDNA sequence (>90% identity to the human at the nucleotide level) is represented as the nucleic acid of SEQ ID NO:7, and codes for a unique fragment of a mouse SOC-2/CRAC-1 polypeptide having the predicted, partial amino acid sequence represented as SEQ ID NO:8. Analysis of the SOC-2/CRAC-1 partial sequence by comparison to nucleic acid and protein databases show that SOC-2/CRAC-1 shares a limited homology to mouse MLSN-1 (SOC-1, SEQ ID NOs: 9 and 10). Limited homology is also shared between SOC-2/CRAC-1 and three C. *Elegans* polypeptides (SEQ ID NOs: 13, 14, and 15). We further believe that SOC-2/CRAC-1 plays a role in the regulation of cellular Ca²⁺ fluxing and, in particular, lymphocyte Ca²⁺ fluxing.

A second member of the human SOC/CRAC family of calcium channels, SOC-3/CRAC-2, is represented as the nucleic acid of SEQ ID NO:29, and codes for the human SOC-3/CRAC-2 polypeptide having the predicted amino acid sequence represented as SEQ ID NO:30 (this molecule may also be referred to as CECH2). SOC-3/CRAC-2 is predominantly expressed in human hematopoietic cells (including peripheral blood lymphocytes, liver, bone marrow, spleen, thymus, lymph nodes, heart, and kidney. Expression can also be detected (at lesser levels) in brain, skeletal muscle colon, small intestine, placenta, lung, and cells (cell lines) such as HL-60, HeLa, K562, MOLT-4, SW-480, A459, and G361.

A third member of the human SOC/CRAC family of calcium channels, SOC-4/CRAC-3, is represented as the nucleic acid of SEQ ID NO:31, and codes for the human SOC-4/CRAC-3 polypeptide having the predicted amino acid sequence represented as SEQ ID NO:32 (this molecule may also be referred to as CECH6). It specifically expressed in the prostate gland/cells.

As used herein, a SOC/CRAC calcium channel nucleic acid (also referred to herein as a "SOC/CRAC nucleic acid" refers to a nucleic acid molecule which: (1) hybridizes under stringent conditions to one or more of the nucleic acids having the sequences of SEQ. ID NOS. 7, 27, 29, and/or 31 (sequences of the mouse and human SOC-2/CRAC-1, human SOC-3/CRAC-2, and human SOC-4/CRAC-3 nucleic acids), and (2) codes for a SOC-2/CRAC-1, a SOC-3/CRAC-2 or a SOC-4/CRAC-3 calcium channel polypeptide, respectively, or unique fragments of said SOC-2/CRAC-1, SOC-3/CRAC-2, or SOC-4/CRAC-3 polypeptide.

As used herein, a SOC/CRAC calcium channel polypeptide (also referred to herein as a "SOC/CRAC polypeptide") refers to a polypeptide that is coded for by a SOC-2/CRAC-1 , a SOC-3/CRAC-2, and/or a SOC-4/CRAC-3 nucleic acid. Preferably, the above-identified SOC/CRAC polypeptides mediate transport of calcium into and out of a cell.

SOC/CRAC polypeptides also are useful as immunogenic molecules for the generation of binding polypeptides (e.g., antibodies) which bind selectively to SOC/CRAC (e.g., SOC-2/CRAC-1, SOC-3/CRAC-2, and/or SOC-4/CRAC-3) polypeptides. Such antibodies can be used in diagnostic assays to identify and/or quantify the presence of a SOC/CRAC polypeptide in a sample, such as a biological fluid or biopsy sample. SOC/CRAC polypeptides further embrace functionally equivalent fragments, variants, and analogs of the preferred SOC/CRAC polypeptides, provided that the fragments, variants, and analogs also are useful in mediating calcium transport into and out of intracellular calcium stores.

As used herein, "SOC/CRAC calcium channel activity" refers to Ca²⁺ transport ("Ca²⁺ fluxing") across the plasma membrane that is mediated by a SOC/CRAC calcium channel polypeptide. The SOC/CRAC calcium channel polypeptide typically has one or more of the following properties: high selectivity, a unitary conductance below the detection level of the patch clamp method,and are subject to inhibition by high intracellular calcium levels. Such activity can be easily detected using standard methodology well known in the art. See, e.g., the Examples and Neher, E., "Ion channels for communication between and within cells", Science, 1992; 256:498-502; and Hoth, M., and Penner, R., "Depletion of intracellular calcium stores activates a calcium current in mast cells", Nature, 1992; 355 (6358):353-6.

Isolated nucleic acid molecules which code for one or more member(s) of the SOC/CRAC family of calcium channel polypeptides are disclosed. The isolated nucleic acid molecules are selected from the following groups:
(a) nucleic acid molecules which hybridize under stringent conditions to one or more nucleic acid molecules selected from the group consisting of SEQ ID NO:1, SEQ ID NO:3, SEQ ID NO:5, SEQ ID NO:7, SEQ ID NO:23, SEQ ID NO:25, SEQ ID NO:27, SEQ ID NO:29, and SEQ ID NO:31, and which code for a SOC/CRAC polypeptide;
(b) deletions, additions and substitutions of (a) which code for a respective SOC/CRAC polypeptide;
(c) nucleic acid molecules that differ from the nucleic acid molecules of (a) or (b) in codon sequence due to the degeneracy of the genetic code, and
(d) complements of (a), (b) or (c).

The isolated nucleic acid molecule disclosed herein comprises one or more of nucleotides 1-1212 of SEQ ID NO:1; nucleotides 1-739 of SEQ ID NO:3; nucleotides 1-1579 of SEQ ID NO:5; nucleotides 1-5117 of SEQ ID NO:23; the mouse homolog for SOC-2/CRAC-1 corresponding to SEQ ID NO:7; nucleotides 1-2180 of SEQ ID NO:25; nucleotides 382-5976 of SEQ ID NO:27; nucleotides 73-3714 of SEQ ID NO:29; and nucleotides 23-3434 of SEQ ID NO:31. Moreover, the isolated nucleic acid molecule comprises a molecule which encodes a polypeptide having one or more sequences selected from the group consisting of SEQ ID NO:2, SEQ ID NO:4, SEQ ID NO:6, SEQ ID NO:8, SEQ ID NO:24, SEQ ID NO:26, SEQ ID NO:28, SEQ ID NO:30, and SEQ ID NO:32.

Isolated nucleic acid molecules are described which are selected from the group consisting of:
(a) a unique fragment of a nucleic acid molecule selected from the group consisting of SEQ ID NO:1, SEQ ID NO:3, SEQ ID NO:5, SEQ ID NO:7, SEQ ID NO:23, SEQ ID NO:25, SEQ ID NO:27, SEQ ID NO:29, and SEQ ID NO:31, (of sufficient length to represent a sequence unique within the human genome); and (b) complements of (a), provided that the unique fragment includes a sequence of contiguous nucleotides which is not identical to a sequence in the prior art as represented by the sequence group consisting of: (1) sequences having the SEQ ID NOs or GenBank accession numbers of Table I, (2) complements of (1), and (3) fragments of (1) and (2).

The sequence of contiguous nucleotides is selected from the group consisting of (1) at least two contiguous nucleotides nonidentical to the sequence group, (2) at least three contiguous nucleotides nonidentical to the sequence group, (3) at least four contiguous nucleotides nonidentical to the sequence group, (4) at least five contiguous nucleotides nonidentical to the sequence group, (5) at least six contiguous nucleotides nonidentical to the sequence group, (6) at least seven contiguous nucleotides nonidentical to the sequence group.

A unique fragment has a size selected from the group consisting of at least: 8 nucleotides, 10 nucleotides, 12 nucleotides, 14 nucleotides, 16 nucleotides, 18 nucleotides, 20, nucleotides, 22 nucleotides, 24 nucleotides, 26 nucleotides, 28 nucleotides, 30 nucleotides, 40 nucleotides, 50 nucleotides, 75 nucleotides, 100 nucleotides, 200 nucleotides, 1000 nucleotides and every integer length therebetween.

According to another aspect of the invention, expression vectors and host cells containing (e.g., transformed or transfected with) expression vectors comprising the nucleic acid molecules of the claims operably linked to a promoter are provided. In certain preferred embodiments, the host cells are eukaryotic cells.

The isolated nucleic acid molecules in the claims have various utilities, including their use as probes and primers to identify additional members of the SOC/CRAC family of calcium channels, as diagnostic reagents for identifying the presence of SOC/CRAC polypeptides in biological or other samples, and as agents.for generating SOC/CRAC binding polypeptides (e.g., antibodies) that can be used as reagents in diagnostic and therapeutic assays to identify the presence, absence, and/or amounts of a SOC/CRAC nucleic acid or polypeptide in a biological or other sample.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulatable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulatable by standard techniques known to those of ordinary skill in the art.

As used herein with respect to polypeptides (discussed below), the term "isolated" means separated from its native environment in sufficiently pure form so that it can be manipulated or used for any one of the purposes of the invention. Thus, isolated means sufficiently pure to be used (i) to raise and/or isolate antibodies, (ii) as a reagent in an assay, or (iii) for sequencing, etc.

Homologs and alleles of the SOC/CRAC nucleic acids can be identified by conventional techniques. Described are nucleic acid sequences which code for SOC/CRAC polypeptides and which hybridize to a nucleic acid molecule selected from a group consisting of the nucleic acid of SEQ ID NO:1, the nucleic acid of SEQ ID NO:3, the nucleic acid of SEQ ID NO:5, the nucleic acid of SEQ ID NO:7, the nucleic acid of SEQ ID NO:23, the nucleic acid of SEQ ID NO:25, the nucleic acid of SEQ ID NO:27, the nucleic acid of SEQ ID NO:29, and the nucleic acid of SEQ ID NO:31, under stringent conditions. The term "stringent conditions" as used herein refers to parameters with which the art is familiar. Nucleic acid hybridization parameters may be found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. More specifically, stringent conditions, as used herein, refers, for example, to hybridization at 65°C in hybridization buffer (3.5 x SSC, 0.02% Ficoll, 0.02% polyvinyl pyrolidone, 0.02% Bovine Serum Albumin, 2.5mM NaH₂PO₄(pH7), 0.5% SDS, 2mM EDTA). SSC is 0.15M sodium chloride/0.15M sodium citrate, pH7; SDS is sodium dodecyl sulphate; and EDTA is ethylenediaminetetracetic acid. After hybridization, the membrane upon which the DNA is transferred is washed at 2 x SSC at room temperature and then at 0.1 x SSC/0.1 x SDS at temperatures up to 68°C.

There are other conditions, reagents, and so forth which can be used, and would result in a similar degree of stringency. The skilled artisan will be familiar with such conditions, and thus they are not given here. It will be understood, however, that the skilled artisan will be able to manipulate the conditions in a manner to permit the clear identification of homologs and alleles of the SOC/CRAC nucleic acids of the invention. The skilled artisan also is familiar with the methodology for screening cells and libraries for expression of such molecules which then are routinely isolated, followed by isolation of the pertinent nucleic acid molecule and sequencing.

In general homologs and alleles typically will share at least 40% nucleotide identity and/or at least 50% amino acid identity to SEQ ID NO:1, SEQ ID NO:3, SEQ ID NO:5, SEQ ID NO:7, SEQ ID NO:23, SEQ ID NO:25, SEQ ID NO:27, SEQ ID NO:29, and/or SEQ ID NO:31, and SEQ ID NO:2, SEQ ID NO:4, SEQ ID NO:6, SEQ ID NO:8, SEQ ID NO:24, SEQ ID NO:26, SEQ ID NO:28, SEQ ID NO:30, and/or SEQ ID NO:32, respectively. In some instances sequences will share at least 50% nucleotide identity and/or at least 65% amino acid identity and in still other instances sequences will share at least 60% nucleotide identity and/or at least 75% amino acid identity. The homology can be calculated using various, publicly available software tools developed by NCBI (Bethesda, Maryland) that can be obtained through the internet (ftp:/ncbi.nlm.nih.gov/pub/). Exemplary tools include the BLAST system available at http://www.ncbi.nlm.nih.gov. Pairwise and ClustalW alignments (BLOSUM30 matrix setting) as well as Kyte-Doolittle hydropathic analysis can be obtained using the MacVetor sequence analysis software (Oxford Molecular Group). Watson-Crick complements of the foregoing nucleic acids also are embraced by the invention.

In screening for SOC/CRAC related genes, such as homologs and alleles of SOC-2/CRAC-1 and/or SOC-3/CRAC-2, a Southern blot may be performed using the foregoing conditions, together with a radioactive probe. After washing the membrane to which the DNA is finally transferred, the membrane can be placed against X-ray film or a phosphoimager plate to detect the radioactive signal.

Given that the expression of the SOC/CRAC gene is prominent in certain human tissues (e.g., SOC-2/CRAC-1: lymphoid tissue/heart, SOC-3/CRAC-2: kidney/colon, SOC-4/CRAC-3: prostate), and given the teachings herein of partial human SOC/CRAC cDNA clones, full-length and other mammalian sequences corresponding to the human SOC/CRAC partial nucleic acid sequences can be isolated from, for example, a cDNA library prepared from one or more of the tissues in which SOC-2/CRAC-1 expression is prominent, SOC-3/CRAC-2 is prominent, and/or SOC-4/CRAC-3 expression is prominent, using standard colony hybridization techniques.

Also described are degenerate nucleic acids which include alternative codons to those present in the native materials. For example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide triplets may be employed to direct the protein synthesis apparatus, *in vitro* or *in vivo*, to incorporate a serine residue into an elongating SOC/CRAC polypeptide. Similarly, nucleotide sequence triplets which encode other amino acid residues include, but are not limited to: CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences. Thus, the invention embraces degenerate nucleic acids that differ from the biologically isolated nucleic acids in codon sequence due to the degeneracy of the genetic code.

Also described are isolated unique fragments of an isolated nucleic acid molecule selected from the group consisting of SEQ ID NO:1, SEQ ID NO:3, SEQ ID NO:5, SEQ ID NO:7, SEQ ID NO:23, SEQ ID NO:25, SEQ ID NO:27, SEQ ID NO:29, and SEQ ID NO:31. A unique fragment is one that is a 'signature' for the larger nucleic acid. For example, the unique fragment is long enough to assure that its precise sequence is not found in molecules within the human genome outside of the SOC/CRAC nucleic acids defined above (and human alleles). Those of ordinary skill in the art may apply no more than routine procedures to determine if a fragment is unique within the human genome.

Unique fragments, however, exclude fragments completely composed of the nucleotide sequences of any of GenBank accession numbers and SEQ ID NOs listed in Table I (SEQ ID NO:9, AB001535, AI226731, H18835, AA419592, AA261842, AA419407, AI098310, AA592910, D86107, AF071787, Z77132, Z83117, Z68333, AA708532, AA551759, AA932133, R47363, N31660, AC005538, AA654650, AA370110, AA313170, AA493512, AI670079, AI671853, AC005538, AA654650, AA370110, AA313170, AA493512, AI670079, AI671853), or other previously published sequences as of the filing date of this application.

A fragment which is completely composed of the sequence described in the foregoing GenBank deposits and SEQ ID NO:9, is one which does not include any of the nucleotides unique to the sequences of the invention. Thus, a unique fragment must contain a nucleotide sequence other than the exact sequence of those in GenBank or fragments thereof. The difference may be an addition, deletion or substitution with respect to the GenBank sequence, or it may be a sequence wholly separate from the GenBank sequence.

Unique fragments can be used as probes in Southern and Northern blot assays to identify such nucleic acids, or can be used in amplification assays such as those employing PCR. As known to those skilled in the art, large probes such as 200, 250, 300 or more nucleotides are preferred for certain uses such as Southern and Northern blots, while smaller fragments will be preferred for uses such as PCR. Unique fragments also can be used to produce fusion proteins for generating antibodies or determining binding of the polypeptide fragments, as demonstrated in the Examples, or for generating immunoassay components. Likewise, unique fragments can be employed to produce nonfused fragments of the SOC/CRAC polypeptides, useful, for example, in the preparation of antibodies, immunoassays or therapeutic applications. Unique fragments further can be used as antisense molecules to inhibit the expression of SOC/CRAC nucleic acids and polypeptides, respectively.

As will be recognized by those skilled in the art, the size of the unique fragment will depend upon its conservancy in the genetic code. Thus, some regions of SEQ ID NO:1, SEQ ID NO:3, SEQ ID NO:5, SEQ ID NO:7, SEQ ID NO:23, SEQ ID NO:25, SEQ ID NO:27, SEQ ID NO:29, and SEQ ID NO:31, and complements thereof, will require longer segments to be unique while others will require only short segments, typically between 12 and 32 nucleotides long (e.g. 12, 13, 14, 15, 16, 17, 18, 19, 20, 21, 22, 23, 24, 25, 26, 27, 28, 29, 30, 31 and 32 bases) or more, up to the entire length of the disclosed sequence. As mentioned above, this disclosure intends to embrace each and every fragment of each sequence, beginning at the first nucleotide, the second nucleotide and so on, up to 8 nucleotides short of the end, and ending anywhere from nucleotide number 8, 9, 10 and so on for each sequence, up to the very last nucleotide, (provided the sequence is unique as described above). Virtually any segment of the region of SEQ ID NO:1 beginning at nucleotide 1 and ending at nucleotide 1212, or SEQ ID NO:3 beginning at nucleotide 1 and ending at nucleotide 739, or SEQ ID NO:5 beginning at nucleotide 1 and ending at nucleotide 1579, or SEQ ID NO:7 beginning at nucleotide 1 and ending at nucleotide 3532, or SEQ ID NO:23 beginning at nucleotide 1 and ending at nucleotide 5117, SEQ ID NO:25 beginning at nucleotide 1 and ending at nucleotide 2180, SEQ ID NO:27 beginning at nucleotide 1 and ending at nucleotide 7419, or SEQ ID NO:29 beginning at nucleotide 1 and ending at nucleotide 4061, or SEQ ID NO:31 beginning at nucleotide 1 and ending at nucleotide 4646, or complements thereof, that is 20 or more nucleotides in length will be unique. Those skilled in the art are well versed in methods for selecting such sequences, typically on the basis of the ability of the unique fragment to selectively distinguish the sequence of interest from other sequences in the human genome of the fragment to those on known databases typically is all that is necessary, although *in vitro* confirmatory hybridization and sequencing analysis may be performed

As mentioned above, antisense oligonucleotides are disclosed that selectively bind to a nucleic acid molecule encoding a SOC/CRAC polypeptide, to decrease SOC/CRAC calcium channel activity as defined in the claims. When using antisense preparations of the invention, slow intravenous administration is preferred.

As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon SEQ ID NO:1, SEQ ID NO:3, SEQ ID NO:5, SEQ ID NO:7, SEQ ID NO:23, SEQ ID NO:25, SEQ ID NO:27, SEQ ID NO:29, and SEQ ID NO:31, or upon allelic or homologous genomic and/or cDNA sequences, one of skill in the art can easily choose and synthesize any of a number of appropriate antisense molecules for use in accordance with the present invention. In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nat. Med. 1(11):1116-1118, 1995). Most preferably, the antisense oligonucleotides comprise a complementary sequence of 20-30 bases. Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted by antisense oligonucleotides. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., Cell Mol. Neurobiol. 14(5):439-457, 1994) and at which proteins are not expected to bind. Finally, although, SEQ ID No:1 discloses a cDNA sequence, one of ordinary skill in the art may easily derive the genomic DNA corresponding to this sequence. Described are antisense oligonucleotides which are complementary to the genomic DNA corresponding to SEQ ID NO:1, SEQ ID NO:3, SEQ ID NO:5,SEQ ID NO:7, SEQ ID NO:23, SEQ ID NO:25, SEQ ID NO:27, SEQ ID NO:29, and SEQ ID NO:31. Similarly, antisense to allelic or homologous SOC/CRAC cDNAs and genomic DNAs are enabled without undue experimentation.

In one set of embodiments, the antisense oligonucleotides may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose. The present invention, thus, contemplates pharmaceutical preparations containing modified antisense molecules that are complementary to and hybridizable with, under physiological conditions, nucleic acids encoding. SOC/CRAC polypeptides, together with pharmaceutically acceptable carriers. Antisense oligonucleotides may be administered as part of a pharmaceutical composition. Such a pharmaceutical composition may include the antisense oligonucleotides in combination with any standard physiologically and/or pharmaceutically acceptable carriers which are known in the art. The compositions should be sterile and contain a therapeutically effective amount of the antisense oligonucleotides in a unit of weight or volume suitable for administration to a patient. The term "pharmaceutically acceptable" means a non-toxic material that does not interfere with the effectiveness of the biological activity of the active ingredients. The term "physiologically acceptable" refers to a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, solubilizers, and other materials which are well known in the art.

As defined in the claims, the invention also involves expression vectors coding for SOC/CRAC proteins and fragments and variants thereof and host cells containing those expression vectors. Virtually any cells, prokaryotic or eukaryotic, which can be transformed with heterologous DNA or RNA and which can be grown or maintained in culture, may be used in the practice of the invention. Examples include bacterial cells such as E.coli and eukaryotic cells such as mouse, hamster, pig, goat, primate, yeast, xenopous, etc. They may be of a wide variety of tissue types, including mast cells, fibroblasts, oocytes and lymphocytes, and they may be primary cells or cell lines. Specific examples include CHO cells and COS cells. Cell-free transcription systems also may be used in lieu of cells.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase or alkaline phosphatase), and genes which visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined.

As used herein, a coding sequence and regulatory sequences are said to be "operably" joined when they are covalently linked in such a way_ as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Especially, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

According to yet another aspect of the invention, isolated SOC/CRAC polypeptides are provided. Preferably, the isolated SOC/CRAC polypeptides are encoded by the isolated SOC/CRAC nucleic acid molecules disclosed herein. The isolated SOC/CRAC polypeptides of the invention have an amino acid sequence selected from the group consisting of SEQ ID Nos. 26 or 30. Preferably, the isolated SOC/CRAC polypeptides are of sufficient length to represent a sequence unique within the human genome. Thus, the preferred embodiments include a sequence of contiguous amino acids which is not identical to a prior art sequence as represented by the sequence group consisting of the contiguous amino acids identified in Table II (SEQ ID NO:10, SEQ ID NO:13, SEQ ID NO:14, SEQ ID NO:15, SEQ ID NO:17, SEQ ID NO:19 and GenBank Acc. Nos. AB001535, AA592910, D86107, AF071787, Z77132, Z83117, Z68333, AA708532, AA551759, AA932133, R47363, N31660, NP003298, CAB00861, NP002411, CAA92726, CAB05572).

In certain embodiments, the isolated SOC/CRAC polypeptides are immunogenic and can be used to generate binding polypeptides (e.g., antibodies) for use in diagnostic and therapeutic applications. Such binding polypeptides also are useful for detecting the presence, absence, and/or amounts of a SOC/CRAC nucleic acid or polypeptide in a sample such as a biological fluid or biopsy sample. Preferably, the SOC/CRAC polypeptides that are useful for generating binding polypeptides are unique polypeptides and, therefore, binding of the antibody to a SOC/CRAC polypeptide in a sample is selective for the SOC/CRAC polypeptide.

Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA) encoding a SOC/CRAC polypeptide or fragment or variant thereof. The heterologous DNA (RNA) is placed under operable control of transcriptional elements to permit the expression of the heterologous DNA in the host cell.

Preferred systems for mRNA expression in mammalian cells are those such as pRc/CMV (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen, Carlsbad, CA), which contains an Epstein Barr virus (EBV) origin of replication, facilitating the maintenance of plasmid as a multicopy extrachromosomal element. Another expression vector is the pEF-BOS plasmid containing the promoter of polypeptide Elongation Factor 1α, which stimulates efficiently transcription *in vitro.* The plasmid is described by Mishizuma and Nagata (Nuc. Acids Res. 18:5322, 1990), and its use in transfection experiments is disclosed by, for example, Demoulin (Mol. Cell. Biol. 16:4710-4716, 1996). Still another preferred expression vector is an adenovirus, described by Stratford-Perricaudet, which is defective for E1 and E3 proteins (J. Clin. Invest. 90:626-630, 1992). The use of the adenovirus as an Adeno.P1A recombinant is disclosed by Wamier et al., in intradermal injection in mice for immunization against P1A (Int. J. Cancer, 67:303-310, 1996).

The invention also embraces so-called expression kits, which allow the artisan to prepare a desired expression vector or vectors. Such expression kits include at least separate portions of each of the previously discussed coding sequences. Other components may be added, as desired, as long as the previously mentioned sequences, which are required, are included.

It will also be recognized that the invention embraces the use of the above described, SOC/CRAC cDNA sequence containing expression vectors, to transfect host cells and cell lines, by these prokaryotic (e.g., E. coli), or eukaryotic (e.g., CHO cells, COS cells, yeast expression systems and recombinant baculovirus expression in insect cells). Especially useful are mammalian cells such as mouse, hamster, pig, goat, primate, etc. They may be of a wide variety of tissue types, and include primary cells and cell lines. Specific examples include dendritic cells, U293 cells, peripheral blood leukocytes, bone marrow stem cells and embryonic stem cells. The invention also permits the construction of SOC/CRAC gene "knock-outs" in cells and in animals, providing materials for studying certain aspects of SOC/CRAC calcium channel activity.

The invention also provides isolated polypeptides (including whole proteins and partial proteins), encoded by the foregoing SOC/CRAC nucleic acids, and include the polypeptides of SEQ ID NO: 26, 30. Such polypeptides are useful, for example, to regulate calcium transport-mediated cell growth, differentiation and proliferation, to generate antibodies, as components of immunoassays, etc. Polypeptides can be isolated from biological samples including tissue or cell homogenates, and can also be expressed recombinantly in a variety of prokaryotic and eukaryotic expression systems by constructing an expression vector appropriate to the expression system, introducing the expression vector into the expression system, and isolating the recombinantly expressed protein. Short polypeptides, including antigenic peptides (such as are presented by MHC molecules on the surface of a cell for immune recognition) also can be synthesized chemically using well-established methods of peptide synthesis.

A unique fragment of a SOC/CRAC polypeptide, in general, has the features and characteristics of unique fragments as discussed above in connection with nucleic acids. As will be recognized by those skilled in the art, the size of the unique fragment will depend upon factors such as whether the fragment constitutes a portion of a conserved protein domain. Thus, some regions of SEQ ID NO:2, SEQ ID NO:4, SEQ ID NO:6, SEQ ID NO:8, SEQ ID NO:24, SEQ ID NO:26, SEQ ID NO:28, SEQ ID NO:30, and/or SEQ ID NO:32, will require longer segments to be unique while others will require only short segments, typically between 5 and 12 amino acids (e.g. 5, 6, 7, 8, 9, 10, 11 and 12 amino acids long or more, including each integer up to the full length, >1,000 amino acids long). Virtually any segment of SEQ ID NO:2, SEQ ID NO:4, SEQ ID NO:6, SEQ ID NO:8, SEQ ID NO:24, SEQ ID NO:26, SEQ ID NO:28, SEQ ID NO:30, and/or SEQ ID NO:32, excluding the ones that share identity with it (the polypeptides identified in Table II - SEQ ID NO:10, SEQ ID NO:13, SEQ ID NO:14, SEQ ID NO:15, SEQ ID NO:17, SEQ ID NO:19, and GenBank Acc. Nos. AB001535, AA592910, D86107, AF071787, Z77132, Z83117, Z68333, AA708532, AA551759, AA932133, R47363, N31660, NP003298, CAB00861, NP002411, CAA92726, CAB05572) that is 9 or more amino acids in length will be unique.

Unique fragments of a polypeptide are fragments which retain a distinct functional capability of the polypeptide. Functional capabilities which can be retained in a unique fragment of a polypeptide include Ca²⁺ fluxing, high selectivity, a unitary conductance below the detection level of the patch clamp method, and/or and are subject to inhibition by high intracellular calcium levels.

One important aspect of a unique fragment is its ability to act as a signature for identifying the polypeptide. Optionally, another aspect of a unique fragment is its ability to provide an immune response in an animal. Those skilled in the art are well versed in methods for selecting unique amino acid sequences, typically on the basis of the ability of the unique fragment to selectively distinguish the sequence of interest from non-family members. A comparison of the sequence of the fragment to those on known databases typically is all that is necessary.

The invention embraces variants of the SOC/CRAC polypeptides as defined in the claims. As used herein, a "variant" of a SOC/CRAC polypeptide is a polypeptide which contains one or more modifications to the primary amino acid sequence of a SOC/CRAC polypeptide. Modifications which create a SOC/CRAC polypeptide variant are typically made to the nucleic acid which encodes the SOC/CRAC polypeptide, and can include deletions, point mutations, truncations, amino acid substitutions and addition of amino acids or non-amino acid moieties to: 1) reduce or eliminate a calcium channel activity of a SOC/CRAC polypeptide; 2) enhance a property of a SOC/CRAC polypeptide, such as protein stability in an expression system or the stability of protein-protein binding; 3) provide a novel activity or property to a SOC/CRAC polypeptide, such as addition of an antigenic epitope or addition of a detectable moiety; or 4) to provide equivalent or better binding to a SOC/CRAC polypeptide receptor or other molecule. Alternatively, modifications can be made directly to the polypeptide, such as by cleavage, addition of a linker molecule, addition of a detectable moiety, such as biotin, addition of a fatty acid, and the like. Modifications also embrace fusion proteins comprising all or part of the SOC/CRAC amino acid sequence. One of skill in the art will be familiar with methods for predicting the effect on protein conformation of a change in protein sequence, and can thus "design" a variant SOC/CRAC polypeptide according to known methods. One example of such a method is described by Dahiyat and Mayo in Science 278:82-87, 1997, whereby proteins can be designed *de novo*. The method can be applied to a known protein to vary only a portion of the polypeptide sequence. By applying the computational methods of Dahiyat and Mayo, specific variants of a SOC/CRAC calcium channel polypeptide can be proposed and tested to determine whether the variant retains a desired conformation.

Variants can include SOC/CRAC polypeptides which are modified specifically to alter a feature of the polypeptide unrelated to its physiological activity. For example, cysteine residues can be substituted or deleted to prevent unwanted disulfide linkages. Similarly, certain amino acids can be changed to enhance expression of a SOC/CRAC polypeptide by eliminating proteolysis by proteases in an expression system (e.g., dibasic amino acid residues in yeast expression systems in which KEX2 protease activity is present).

Mutations of a nucleic acid which encodes a SOC/CRAC polypeptide preferably preserve the amino acid reading frame of the coding sequence and, preferably, do not create regions in the nucleic acid which are likely to hybridize to form secondary structures, such as hairpins or loops, which can be deleterious to expression of the variant polypeptide.

Mutations can be made by selecting an amino acid substitution, or by random mutagenesis of a selected site in a nucleic acid which encodes the polypeptide. Variant polypeptides are then expressed and tested for one or more activities to determine which mutation provides a variant polypeptide with the desired properties. Further mutations can be made to variants (or to non-variant SOC/CRAC polypeptides) which are silent as to the amino acid sequence of the polypeptide, but which provide preferred codons for translation in a particular host. The preferred codons for translation of a nucleic acid in, e.g., E. coli, are well known to those of ordinary skill in the art. Still other mutations can be made to the noncoding sequences of a SOC/CRAC gene or cDNA clone to enhance expression of the polypeptide.

The skilled artisan will realize that conservative amino acid substitutions may be made in SOC/CRAC polypeptides to provide functionally equivalent variants of the foregoing polypeptides, i.e, the variants retain the functional capabilities of the SOC/CRAC polypeptides. As used herein, a "conservative amino acid substitution" refers to an amino acid substitution which does not alter the relative charge or size characteristics of the protein in which the amino acid substitution is made. Variants can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Exemplary functionally equivalent variants of the SOC/CRAC polypeptides include conservative amino acid substitutions of SEQ ID NO:2, SEQ ID NO:4, SEQ ID NO:6, SEQ ID NO:8, SEQ ID NO:24, SEQ ID NO:26, SEQ ID NO:28, SEQ ID NO:30, and/or SEQ ID NO:32. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

Thus functionally equivalent variants of SOC/CRAC polypeptides, i.e., variants of SOC/CRAC polypeptides which retain the function of the natural SOC/CRAC polypeptides, are described. Conservative amino-acid substitutions in the amino acid sequence of SOC/CRAC polypeptides to produce functionally equivalent variants of SOC/CRAC polypeptides typically are made by alteration of a nucleic acid encoding SOC/CRAC polypeptides (e.g., SEQ ID NOs:1, 3, 5, 7, 23, 25, 27, 29, 31). Such substitutions can be made by a variety of methods known to one of ordinary skill in the art. For example, amino acid substitutions may be made by PCR-directed mutation, site-directed mutagenesis according to the method of Kunkel (Kunkel, Proc. Nat. Acad Sci. U.S.A. 82: 488-492, 1985), or by chemical synthesis of a gene encoding a SOC/CRAC polypeptide. The activity of functionally equivalent fragments of SOC/CRAC polypeptides can be tested by cloning the gene encoding the altered SOC/CRAC polypeptide into a bacterial or mammalian expression vector, introducing the vector into an appropriate host cell, expressing the altered SOC/CRAC polypeptide, and testing for a functional capability of the SOC/CRAC polypeptides as disclosed herein (e.g., SOC/CRAC calcium channel activity).

The invention as described herein has a number of uses, some of which are described elsewhere herein. First, the invention permits isolation of SOC/CRAC polypeptides, including the isolation of the complete SOC/CRAC polypeptide. A variety of methodologies well-known to the skilled practitioner can be utilized to obtain isolated SOC/CRAC molecules. The polypeptide may be purified from cells which naturally produce the polypeptide by chromatographic means or immunological recognition. Alternatively, an expression vector may be introduced into cells to cause production of the polypeptide. In another method, mRNA transcripts may be microinjected or otherwise introduced into cells to cause production of the encoded polypeptide. Translation of SOC/CRAC mRNA in cell-free extracts such as the reticulocyte lysate system also may be used to produce SOC/CRAC polypeptides. Those skilled in the art also can readily follow known methods for isolating SOC/CRAC polypeptides. These include, but are not limited to, immunochromatography, HPLC, size-exclusion chromatography, ion-exchange chromatography and immune-affinity chromatography.

The invention also provides, in certain embodiments, "dominant negative" polypeptides derived from SOC/CRAC polypeptides. A dominant negative polypeptide is an inactive variant of a protein, which, by interacting with the cellular machinery, displaces an active protein from its interaction with the cellular machinery or competes with the active protein, thereby reducing the effect of the active protein. For example, a dominant negative receptor which binds a ligand but does not transmit a signal in response to binding of the ligand can reduce the biological effect of expression of the ligand. Likewise, a dominant negative inactive SOC/CRAC calcium channel which interacts normally with the cell membrane but which does not mediate calcium transport can reduce calcium transport in a cell. Similarly, a dominant negative transcription factor which binds to a promoter site in the control region of a gene but does not increase gene transcription can reduce the effect of a normal transcription factor by occupying promoter binding sites without increasing transcription.

The end result of the expression of a dominant negative polypeptide in a cell is a reduction in function of active proteins. One of ordinary skill in the art can assess the potential for a dominant negative variant of a protein, and using standard mutagenesis techniques to create one or more dominant negative variant polypeptides. See, e.g., U.S. Patent No. 5,580,723 and Sambrook et al.; 1989, Molecular Cloning: A Laboratory Manual, Second Edition, Cold Spring Harbor Laboratory Press. The skilled artisan then can test the population of mutagenized polypeptides for diminution in a selected and/or for retention of such an activity. Other similar methods for creating and testing dominant negative variants of a protein will be apparent to one of ordinary skill in the art.

Also described is a method for isolating a SOC/CRAC molecule having SOC/CRAC calcium channel activity. The method involves contacting a binding molecule that is a SOC/CRAC nucleic acid or a SOC/CRAC binding polypeptide with a sample containing one or more SOC/CRAC molecules under conditions that allow such binding (see earlier discussion) to form a complex, detecting the presence of the complex, isolating the SOC/CRAC molecule from the complex, and determining whether the isolated SOC/CRAC molecule has SOC/CRAC calcium channel activity. Thus, the invention is useful for identifying and isolating full length complementary (cDNA) or genomic nucleic acids encoding SOC/CRAC polypeptides having SOC/CRAC calcium channel activity. Identification and isolation of such nucleic acids and polypeptides may be accomplished by hybridizing/binding, under appropriate conditions well known in the art, libraries and/or restriction enzyme-digested human nucleic acids, with a labeled SOC/CRAC molecular probe. As used herein, a "label" includes molecules that are incorporated into, for example, a SOC/CRAC molecule (nucleic acid or peptide), that can be directly or indirectly detected. A wide variety of detectable labels are well known in the art that can be used, and include labels that provide direct detection (e.g., radioactivity, luminescence, optical or electron density, etc), or indirect detection (e.g., epitope tag such as the FLAG epitope, enzyme tag such as horseseradish peroxidase, etc.). The label may be bound to a SOC/CRAC binding partner, or incorporated into the structure of the binding partner.

A variety of methods may be used to detect the label, depending on the nature of the label and other assay components. For example, the label may be detected while bound to the solid substrate or subsequent to separation from the solid substrate. Labels may be directly detected through optical or electron density, radioactive emissions, nonradioactive energy transfers, etc. or indirectly detected with antibody conjugates, strepavidin-biotin conjugates, etc. Methods for detecting the labels are well known in the art. Once a library clone or hybridizing fragment is identified in the hybridization/binding reaction, it can be further isolated by employing standard isolation/cloning techniques known to those of skill in the art. See, generally, Sambrook et al., 1989, Molecular Cloning: A Laboratory Manual, 2nd Edition, Cold Spring Harbor Laboratory Press. In addition, nucleic acid amplification techniques well known in the art, may also be used to locate splice variants of calcium channel (or calcium channel subunits) with SOC/CRAC calcium channel activity. Size and sequence determinations of the amplification products can reveal splice variants.

The foregoing isolated nucleic acids and polypeptides may then be compared to the nucleic acids and polypeptides of the present invention in order to identify homogeneity or divergence of the sequences, and be further characterized functionally to determine whether they belong to a family of molecules with SOC/CRAC calcium channel activity (for methodology see under the Examples section).

The isolation of the SOC/CRAC cDNA and/or partial sequences thereof also makes it possible for the artisan to diagnose a disorder characterized by an aberrant expression of SOC/CRAC. These methods involve determining expression of the SOC/CRAC gene, and/or SOC/CRAC polypeptides derived therefrom. In the former situation, such determinations can be carried out via any standard nucleic acid determination assay, including the polymerase chain reaction, or assaying with labeled hybridization probes as exemplified below. In the latter situation, such determination can be carried out via any standard immunological assay using, for example, antibodies which bind to the SOC/CRAC protein.

The invention also embraces isolated peptide binding agents which, for example, can be antibodies or fragments of antibodies ("binding polypeptides"), having the ability to selectively bind to SOC/CRAC polypeptides. Antibodies include polyclonal and monoclonal antibodies, prepared according to conventional methodology.

Exclude are binding agents (e.g., antibodies) that bind to the polypeptides encoded by the nucleic acids of SEQ ID NOs: 10, 12, 13, 14, 15, 17, and 19.

Significantly, as is well-known in the art, only a small portion of an antibody molecule, the paratope, is involved in the binding of the antibody to its epitope (see, in general, Clark, W.R. (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. Thus, for example, PCT International Publication Number WO 92/04381 teaches the production and use of humanized murine RSV antibodies in which at least a portion of the murine FR regions have been replaced by FR regions of human origin. Such antibodies, including fragments of intact antibodies with antigen-binding ability, are often referred to as "chimeric" antibodies.

Thus, as will be apparent to one of ordinary skill in the art, also described are F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The antibodies include so-called single chain antibodies.

Thus, the description relates to binding polypeptides of numerous size and type that bind selectively to SOC/CRAC polypeptides, and complexes containing SOC/CRAC polypeptides. These binding polypeptides also may be derived also from sources other than antibody technology. For example, such polypeptide binding agents can be provided by degenerate peptide libraries which can be readily prepared in solution, in immobilized form, as bacterial flagella peptide display libraries or as phage display libraries. Combinatorial libraries also can be synthesized of peptides containing one or more amino acids. Libraries further can be synthesized of peptides and non-peptide synthetic moieties.

Phage display can be particularly effective in identifying binding peptides useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to the SOC/CRAC polypeptide or a complex containing a SOC/CRAC polypeptide. This process can be repeated through several cycles of reselection of phage that bind to the SOC/CRAC polypeptide or complex. Repeated rounds lead to enrichment of phage bearing particular sequences. DNA sequence analysis can be conducted to identify the sequences of the expressed polypeptides. The minimal linear portion of the sequence that binds to the SOC/CRAC polypeptide or complex can be determined. One can repeat the procedure using a biased library containing inserts containing part or all of the minimal linear portion plus one or more additional degenerate residues upstream or downstream thereof. Yeast two-hybrid screening methods also may be used to identify polypeptides that bind to the SOC/CRAC polypeptides. Thus, the SOC/CRAC polypeptides of the invention, or a fragment thereof, or complexes of SOC/CRAC can be used to screen peptide libraries, including phage display libraries, to identify and select peptide binding polypeptides that selectively bind to the SOC/CRAC polypeptides of the invention. Such molecules can be used, as described, for screening assays, for purification protocols, for interfering directly with the functioning of SOC/CRAC and for other purposes that will be apparent to those of ordinary skill in the art.

A SOC/CRAC polypeptide, or a fragment thereof, also can be used to isolate naturally occurring, polypeptide binding partners which may associate with the SOC/CRAC polypeptide in the membrane of a cell. Isolation of binding partners may be performed according to well-known methods. For example, isolated SOC/CRAC polypeptides can be attached to a substrate, and then a solution suspected of containing an SOC/CRAC binding partner may be applied to the substrate. If the binding partner for SOC/CRAC polypeptides is present in the solution, then it will bind to the substrate-bound SOC/CRAC polypeptide. The binding partner then may be isolated. Other proteins which are binding partners for SOC/CRAC, may be isolated by similar methods without undue experimentation.

The invention also describes kits which could be used to measure the levels of the nucleic acids of the invention, expression products of the invention or anti-SOC/CRAC antibodies. In the case of nucleic acid detection, pairs of primers for amplifying SOC/CRAC nucleic acids can be included. The preferred kits would include controls such as known amounts of nucleic acid probes, SOC/CRAC epitopes (such as SOC/CRAC expression products) or anti-SOC/CRAC antibodies, as well as instructions or other printed material. In certain embodiments the printed material can characterize risk of developing a disorder that is characterized by aberrant SOC/CRAC polypeptide expression based upon the outcome of the assay. The reagents may be packaged in containers and/or coated on wells in predetermined amounts, and the kits may include standard materials such as labeled immunological reagents (such as labeled anti-IgG antibodies) and the like. One kit is a packaged polystyrene microtiter plate coated with a SOC/CRAC polypeptide and a container containing labeled anti-human IgG antibodies. A well of the plate is contacted with, for example, serum, washed and then contacted with the anti-IgG antibody. The label is then detected. A kit embodying features of the present invention is comprised of the following major elements: packaging an agent of the invention, a control agent, and instructions. Packaging is a box-like structure for holding a vial (or number of vials) containing an agent of the invention a vial (or number of vials) containing a control agent, and instructions. Individuals skilled in the art can readily modify packaging to suit individual needs.

Also claimed is a method for determining the level of SOC/CRAC expression in a subject. As used herein, a subject is a human, non-human primate, cow, horse, pig, sheep, goat, dog, cat or rodent. In all embodiments, human subjects are preferred. Expression is defined either as SOC/CRAC mRNA expression or SOC/CRAC polypeptide expression. Various methods can be used to measure expression. Preferred embodiments of the invention include PCR and Northern blotting for measuring mRNA expression, and monoclonal or polyclonal SOC/CRAC antisera as reagents to measure SOC/CRAC polypeptide expression. In certain embodiments, test samples such as biopsy samples, and biological fluids such as blood, are used as test samples. SOC/CRAC expression in a test sample of a subject is compared to SOC/CRAC expression in control sample to, e.g., assess the presence or absence or stage of a proliferative disorder (e.g., a lymphocyte proliferative disorder) in a subject.

SOC/CRAC polypeptides preferably are produced recombinantly, although such polypeptides may be isolated from biological extracts. Recombinantly produced SOC/CRAC polypeptides include chimeric proteins comprising a fusion of a SOC/CRAC protein with another polypeptide, e.g., a polypeptide capable of providing or enhancing protein-protein binding, sequence specific nucleic acid binding (such as GAL4), enhancing stability of the SOC/CRAC polypeptide under assay conditions, or providing a detectable moiety, such as green fluorescent protein. A polypeptide fused to a SOC/CRAC polypeptide or fragment may also provide means of readily detecting the fusion protein, e.g., by immunological recognition or by fluorescent labeling.

The generation of transgenic non-human animals is also possible. As used herein, "transgenic non-human animals" includes non-human animals having one or more exogenous nucleic acid molecules incorporated in germ line cells and/or somatic cells. Thus the transgenic animal include "knockout" animals having a homozygous or heterozygous gene disruption by homologous recombination, animals having episomal or chromosomally incorporated expression vectors, etc. Knockout animals can be prepared by homologous recombination using embryonic stem cells as is well known in the art. The recombination may be facilitated using, for example, the cre/lox system or other recombinase systems known to one of ordinary skill in the art. In certain embodiments, the recombinase system itself is expressed conditionally, for example, in certain tissues or cell types, at certain embryonic or post-embryonic developmental stages, inducibly by the addition of a compound which increases or decreases expression, and the like. In general, the conditional expression vectors used in such systems use a variety of promoters which confer the desired gene expression pattern (e.g., temporal or spatial). Conditional promoters also can be operably linked to SOC/CRAC nucleic acid molecules to increase expression of SOC/CRAC in a regulated or conditional manner. *Trans*-acting negative regulators of SOC/CRAC calcium channel activity or expression also can be operably linked to a conditional promoter as described above. Such *trans*-acting regulators include antisense SOC/CRAC nucleic acids molecules, nucleic acid molecules which encode dominant negative SOC/CRAC molecules, ribozyme molecules specific for SOC/CRAC nucleic acids, and the like. The transgenic non-human animals are useful in experiments directed toward testing biochemical or physiological effects of diagnostics or therapeutics for conditions characterized by increased or decreased SOC/CRAC expression. Other uses will be apparent to one of ordinary skill in the art.

The invention further provides efficient methods of identifying agents or lead compounds for agents active at the level of a SOC/CRAC polypeptide (e.g., a SOC/CRAC polypeptide) or SOC/CRAC fragment dependent cellular function. In particular, such functions include interaction with other polypeptides or fragments thereof, and selective binding to certain molecules (e.g., agonists and antagonists). Generally, the screening methods involve assaying for compounds which interfere with SOC/CRAC calcium channel activity, although compounds which enhance SOC/CRAC calcium channel activity also can be assayed using the screening methods. Such methods are adaptable to automated, high throughput screening of compounds. The target therapeutic indications for pharmacological agents detected by the screening methods are limited only in that the target cellular function be subject to modulation by alteration of the formation of a complex comprising a SOC/CRAC polypeptide or fragment thereof and one or more SOC/CRAC binding targets. Target indications include cellular processes modulated by SOC/CRAC such as Ca²⁺ fluxing, and affected by SOC/CRAC ability to form complexes with other molecules and polypeptides as, for example, may be present in the cell membrane.

A wide variety of assays for pharmacological agents are provided, including, expression assays, labeled *in vitro* protein-protein binding assays, electrophoretic mobility shift assays, immunoassays, cell-based assays such as calcium transport assays, etc. For example, two-hybrid screens are used to rapidly examine the effect of transfected nucleic acids on the intracellular binding of SOC/CRAC or SOC/CRAC fragments to specific intracellular targets (e.g. a tyrosine kinase). The transfected nucleic acids can encode, for example, combinatorial peptide libraries or cDNA libraries. Convenient reagents for such assays, e.g., GAL4 fusion proteins, are known in the art. An exemplary cell-based assay involves transfecting a cell with a nucleic acid encoding a SOC/CRAC polypeptide fused to a GAL4 DNA binding domain and a nucleic acid encoding a reporter gene operably linked to a gene expression regulatory region, such as one or more GAL4 binding sites. Activation of reporter gene transcription occurs when the SOC/CRAC and reporter fusion polypeptides bind such as to enable transcription of the reporter gene. Agents which modulate a SOC/CRAC polypeptide mediated cell function are then detected through a change in the expression of reporter gene. Methods for determining changes in the expression of a reporter gene are known in the art.

In an expression system, for example, a SOC/CRAC polypeptide is attached to a membrane, the membrane preferably separating two fluid environments and being otherwise not permeable to Ca²⁺. Such separation is preferred so that a change in Ca²⁺ concentration on either side of the membrane is mediated only through the attached SOC/CRAC polypeptide. Preferably, a SOC/CRAC polypeptide is expressed in an intact cell and is present on the cell-membrane (as in physiologic conditions). The cell expressing the SOC/CRAC polypeptide is preferably a eukaryotic cell, and the SOC/CRAC polypeptide is preferably recombinantly expressed, although cells naturally expressing a SOC/CRAC polypeptide may also be used. Synthetic membranes, however, containing SOC/CRAC polypeptides may also be used. See, e.g., K. Kiselyov, et al., Functional interaction between InsP3 receptors and store-operated Htrp3 channels, Nature 396, 478-82 (1998).

The cell expressing the SOC/CRAC polypeptide is incubated under conditions which, in the absence of the candidate agent, permit calcium flux into the cell and allow detection of a reference calcium concentration. For example, depletion of intracellular calcium stores with thapsigargin or other agents (Putney, J.W. Jr., in Capacitative Calcium Entry, R.G. Landes Co. and Chapman & Hall, 1997) would produce a given level of SOC/CRAC channel activation and a given reference calcium concentration. Detection of a decrease in the foregoing activities (i.e., a decrease in the intracellular calcium concentration) relative to the reference calcium concentration indicates that the candidate agent is a lead compound for an agent to inhibit SOC/CRAC calcium channel activity. Preferred SOC/CRAC polypeptides include the polypeptides of claim 15.

SOC/CRAC fragments used in the methods, when not produced by a transfected nucleic acid are added to an assay mixture as an isolated polypeptide. SOC/CRAC polypeptides preferably are produced recombinantly, although such polypeptides may be isolated from biological extracts or chemically synthesized. Recombinantly produced SOC/CRAC polypeptides include chimeric proteins comprising a fusion of a SOC/CRAC protein with another polypeptide, e.g., a polypeptide capable of providing or enhancing protein-protein binding, sequence specific nucleic acid binding (such as GAL4), enhancing stability of the SOC/CRAC polypeptide under assay conditions, or providing a detectable moiety, such as green fluorescent protein or Flag epitope.

The assay mixture is comprised of a SOC/CRAC polypeptide binding target (candidate agent) capable of interacting with a SOC/CRAC polypeptide. While natural SOC/CRAC binding targets may be used, it is frequently preferred to use portions (e.g., peptides or nucleic acid fragments) or analogs (i.e., agents which mimic the SOC/CRAC binding properties of the natural binding target for purposes of the assay) of the SOC/CRAC binding target so long as the portion or analog provides binding affinity and avidity to the SOC/CRAC polypeptide (or fragment thereof) measurable in the assay.

The assay mixture also comprises a candidate agent (binding target, e.g., agonist/antagonist). Typically, a plurality of assay mixtures are run in parallel with different agent concentrations to obtain a different response to the various concentrations. Typically, one of these concentrations serves as a negative control, i.e., at zero concentration of agent or at a concentration of agent below the limits of assay detection. Candidate agents encompass numerous chemical classes, although typically they are organic compounds. Preferably, the candidate agents are small organic compounds, i.e., those having a molecular weight of more than 50 yet less than about 2500, preferably less than about 1000 and, more preferably, less than about 500. Candidate agents comprise functional chemical groups necessary for structural interactions with polypeptides and/or nucleic acids, and typically include at least an amine, carbonyl, hydroxyl or carboxyl group, preferably at least two of the functional chemical groups and more preferably at least three of the functional chemical groups. The candidate agents can comprise cyclic carbon or heterocyclic structure and/or aromatic or polyaromatic structures substituted with one or more of the above-identified functional groups. Candidate agents also can be biomolecules such as peptides, saccharides, fatty acids, sterols, isoprenoids, purines, pyrimidines, derivatives or structural analogs of the above, or combinations thereof and the like. Where the agent is a nucleic acid, the agent typically is a DNA or RNA molecule, although modified nucleic acids as defined herein are also contemplated.

Candidate agents are obtained from a wide variety of sources including libraries of synthetic or natural compounds. For example, numerous means are available for random and directed synthesis of a wide variety of organic compounds and biomolecules, including expression of randomized oligonucleotides, synthetic organic combinatorial libraries, phage display libraries of random peptides, and the like. Alternatively, libraries of natural compounds in the form of bacterial, fungal, plant and animal extracts are available or readily produced. Additionally, natural and synthetically produced libraries and compounds can be readily modified through conventional chemical, physical, and biochemical means. Further, known agents may be subjected to directed or random chemical modifications such as acylation, alkylation, esterification, amidification, etc. to produce structural analogs of the agents. Non-SOC/CRAC calcium channel agonists and antagonists, for example, include agents such as dihydropyridines (DHPs), phenylalkylamines, omega conotoxin (omega.-CgTx) and pyrazonoylguanidines.

A variety of other reagents also can be included in the mixture. These include reagents such as salts, buffers, neutral proteins (e.g., albumin), detergents, etc. which may be used to facilitate optimal protein-protein, protein-nucleic acid, and/or protein/membrane component binding association. Such a reagent may also reduce non-specific or background interactions of the reaction components. Other reagents that improve the efficiency of the assay such as protease, inhibitors, nuclease inhibitors, antimicrobial agents, and the like may also be used.

The mixture of the foregoing assay materials is incubated under conditions whereby, but for the presence of the candidate agent, the SOC/CRAC polypeptide specifically binds the cellular binding target, a portion thereof or analog thereof. The order of addition of components, incubation temperature, time of incubation, and other perimeters of the assay may be readily determined. Such experimentation merely involves optimization of the assay parameters, not the fundamental composition of the assay. Incubation temperatures typically are between 4°C and 40°C. Incubation times preferably are minimized to facilitate rapid, high throughput screening, and typically are between 0.1 and 10 hours.

After incubation, the presence or absence of specific binding between the SOC/CRAC polypeptide and one or more binding targets is detected by any convenient method available to the user. For cell free binding type assays, a separation step is often used to separate bound from unbound components. The separation step may be accomplished in a variety of ways. Conveniently, at least one of the components is immobilized on a solid substrate, from which the unbound components may be easily separated. The solid substrate can be made of a wide variety of materials and in a wide variety of shapes, e.g., microtiter plate, microbead, dipstick, resin particle, etc. The substrate preferably is chosen to maximum signal to noise ratios, primarily to minimize background binding, as well as for ease of separation and cost.

Separation may be effected for example, by removing a bead or dipstick from a reservoir, emptying or diluting a reservoir such as a microtiter plate well, rinsing a bead, particle, chromotograpic column or filter with a wash solution or solvent. The separation step preferably includes multiple rinses or washes. For example, when the solid substrate is a microtiter plate, the wells may be washed several times with a washing solution, which typically includes those components of the incubation mixture that do not participate in specific bindings such as salts, buffer, detergent, non-specific protein, etc. Where the solid substrate is a magnetic bead, the beads may be washed one or more times with a washing solution and isolated using a magnet.

Detection may be effected in any convenient way for cell-based assays such as two- or three-hybrid screens. The transcript resulting from a reporter gene transcription assay of SOC/CRAC polypeptide interacting with a target molecule typically encodes a directly or indirectly detectable product, e.g., β-galactosidase activity, luciferase activity, and the like. For cell-free binding assays, one of the components usually comprises, or is coupled to, a detectable label. A wide variety of labels can be used, such as those that provide direct detection (e.g., radioactivity, luminescence, optical or electron density, etc.) or indirect detection (e.g., epitope tag such as the FLAG epitope, enzyme tag such as horseseradish peroxidase, etc.). The label may be bound to a SOC/CRAC binding partner, or incorporated into the structure of the binding partner.

A variety of methods may be used to detect the label, depending on the nature of the label and other assay components. For example, the label may be detected while bound to the solid substrate or subsequent to separation from the solid substrate. Labels may be directly detected through optical or electron density, radioactive emissions, nonradiative energy transfers, etc. or indirectly detected with antibody conjugates, strepavidin-biotin conjugates, etc. Methods for detecting the labels are well known in the art.

Of particular importance in any of the foregoing assays and binding studies is the use of a specific sequence motif identified in the SOC-2/CRAC-1 polypeptide sequence as a kinase catalytic domain. Amino acids 999-1180 of the SOC-2/CRAC-1 polypeptide (SEQ ID NO:24) (or a fragment thereof), show a localized homology with the catalytic domains of eukaryotic elongation factor-2 kinase (eEF-2 kinase, GenBank Acc. no. U93850) and *Dictyostelium* myocin heavy chain kinase A (MHCK A, GenBank Acc. no. U16856), as disclosed in Ryazanov AG, et al., Proc Natl Acad Sci U S A, 1997, 94(10):4884-4889. Therefore, a method for identifying agents useful in the modulation of SOC/CRAC polypeptide kinase activity is described. The method involves contacting a SOC/CRAC polypeptide with kinase activity, that includes, for example, amino acids 999-1180 of the SOC-2/CRAC-1 polypeptide (SEQ ID NO:24) with a candidate agent suspected of modulating SOC/CRAC kinase activity, under conditions sufficient to allow the candidate agent to interact with the SOC/CRAC polypeptide and modulate its kinase activity; detecting a kinase activity associated with the SOC/CRAC polypeptide in the presence of the candidate agent; and comparing the kinase activity in the previous step with a control kinase activity of a SOC/CRAC polypeptide in the absence of the candidate agent to determine whether the candidate agent modulates (increases or decreases) SOC/CRAC kinase activity. Other controls for kinase activity can also be performed at the same time, for example, by utilizing eEF-2 kinase and/or *Dictyostelium* MHC Kinase A, in a similar manner to the SOC/CRAC member. Methods for performing such kinase activity assays are well known in the art.

Also provided are SOC/CRAC-specific binding agents, methods of identifying and making such agents, and their use in diagnosis, therapy and pharmaceutical development. For example, SOC/CRAC-specific agents are useful in a variety of diagnostic and therapeutic applications, especially where disease or disease prognosis is associated with altered SOC/CRAC and SOC/CRAC calcium channel fluxing characteristics. Novel SOC/CRAC-specific binding agents include SOC/CRAC-specific antibodies and other natural intracellular and extracellular binding agents identified with assays such as two hybrid screens, and non-natural intracellular and extracellular binding agents identified in screens of chemical libraries and the like.

In general, the specificity of SOC/CRAC binding to a specific molecule is determined by binding equilibrium constants. Targets which are capable of selectively binding a SOC/CRAC polypeptide preferably have binding equilibrium constants of at least about 10⁷ M⁻¹, more preferably at least about 10⁸ M⁻¹, and most preferably at least about 10⁹ M⁻¹. The wide variety of cell based and cell free assays may be used to demonstrate SOC/CRAC-specific binding. Cell based assays include one, two and three hybrid screens, assays in which SOC/CRAC-mediated transcription is inhibited or increased, etc. Cell free assays include SOC/CRAC-protein binding assays, immunoassays, etc. Other assays useful for screening agents which bind SOC/CRAC polypeptides include fluorescence resonance energy transfer (FRET), and electrophoretic mobility shift analysis (EMSA).

Various techniques may be employed for introducing nucleic acids of the invention into cells, depending on whether the nucleic acids are introduced *in vitro or in vivo* in a host. Such techniques include transfection of nucleic acid-CaPO₄ precipitates, transfection of nucleic acids associated with DEAE, transfection with a retrovirus including the nucleic acid of interest, liposome mediated transfection, and the like. For certain uses, it is preferred to target the nucleic acid to particular cells. In such instances, a vehicle used for delivering a nucleic acid of the invention into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. For example, where liposomes are employed to deliver the nucleic acids of the invention, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids.

Other delivery systems can include time-release, delayed release or sustained release delivery systems. Such systems can avoid repeated administrations of the anti-inflammatory agent, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono- di- and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for treatment of chronic conditions. Long-term release, are used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

The application also contemplates gene therapy. The procedure for performing *ex vivo* gene therapy is outlined in U.S. Patent 5,399,346 and in exhibits submitted in the file history of that patent, all of which are publicly available documents. In general, it involves introduction *in vitro* of a functional copy of a gene into a cell(s) of a subject which contains a defective copy of the gene, and returning the genetically engineered cell(s) to the subject. The functional copy of the gene is under operable control of regulatory elements which permit expression of the gene in the genetically engineered cell(s). Numerous transfection and transduction techniques as well as appropriate expression vectors are well known to those of ordinary skill in the art, some of which are described in PCT application WO95/00654. *In vivo* gene therapy using vectors such as adenovirus, retroviruses, herpes virus, and targeted liposomes also is contemplated according to the invention. See, e.g., U.S. Patent Nos. 5,670,488, entitled "Adenovirus Vector for Gene Therapy", issued to Gregory et al., and 5,672,344, entitled "Viral-Mediated Gene Transfer System", issued to Kelley et al.

The invention will be more fully understood by reference to the following examples. These examples, however, are merely intended to illustrate the embodiments of the invention and are not to be construed to limit the scope of the invention.

### Examples

As an initial approach to identifying SOC/CRAC channels, we considered publicly available data and hypothesized that the following characteristics are likely to be exhibited by SOC/CRAC calcium channels: i) SOC/CRAC calcium channels would be integral membrane proteins related (probably distantly) to one of the known calcium channel families (e.g. voltage gated, ligand gated, Trp), and therefore should have a pore region formed by a tetramer of 6-7 transmembrane (TM) regions; ii) high calcium selectivity was likely to come at the price of complexity, and therefore these were likely to be large proteins; iii) the high calcium selectivity of this type of channel was likely to be useful and, therefore, highly conserved; and iv) these channels should be expressed in one or more types of lymphocytes, since ICRAC is best defined in those cell types. Since the full genome of the nematode C. *elegans* is nearing completion, and IP3-dependent calcium signals have recently been shown to be required for one or more aspects of C. *elegans* development, we took the set of proteins encoded by this genome (at the time this search was initiated WORMPEP14 was the available predicted protein set) and began searching for proteins which fit the criteria above. This search began by proceeding in alphabetical order through WORMPEP 14 and arbitrarily excluding all proteins below approximately 1000 amino acids in size, followed by focusing on remaining proteins with clear TM spanning regions similar to those of other calcium channels. We stopped this screen on encountering a protein designated CO5C12.3, a predicted protein of 1816 amino acids (SEQ ID NO:13). COSC12.3 was noteable because its central pore region had some sequence similarity to but was clearly distinct from members of the Trp family of calcium channels, and the hydrophobicity plot of this region showed a characteristically wide spacing between the fifth and sixth TM regions for the amino acid residues which are thought to line the channel pore region and mediate the calcium selectivity of the channels. In addition, it lacked any ankyrin repeats in the region amino-terminal to its pore region, further distinguishing it from other Trp family proteins.

We then used COSC12.3 for BLAST alignment screening of the rest of the C. *elegans* genome and also mammalian databases for homologous proteins, revealing two other C. *elegans* homologues (SEQ ID NO:14 and SEQ ID NO:15), and also a recently cloned mammalian protein named melastatin-1 (MLSN-1-/SOC-1, SEQ ID NOs:9 and 10, and GenBank Acc. No. AF071787). Using these sequences, we subsequently performed an exhaustive screening of publicly accessible EST databases in search of lymphocyte homologues, but were unsuccessful in detecting any homologous transcripts in any lymphocyte lines. Since MLSN-1 (SEQ ID NOs:9 and 10) was expressed exclusively in melanocytes and retina by Northern blot hybridization and by EST database searching, there was no evidence that this type of channel was expressed in the type of cell in which ICRAC-like currents were best defined. Subsequent BLAST searches picked up mouse EST sequence AI098310 (SEQ ID NO:22) from a monocyte cell line. The I.M.A.G.E. consortium clone containing the above-identified EST was then purchased from ATCC (clone ID. 1312756, Manassas, VA) and was further characterized. Using other portions of this sequence in EST searches, we subsequently picked up similar sequences in human B-cells (SEQ ID NOs:20 and 21), and other cell types as well (SEQ ID NOs: 11, 12, 16, 17, 18, and 19). Most of these sequences were subsequently identified to be part of the 3'-UTR or of the carboxy terminal region of the proteins, which are not readily identifiable as Trp channels, providing an explanation for the art's inability to detect any type of Trp related transcripts in lymphocytes. Partial sequences from the 5' and/or 3' ends of the above identified clones were then used to screen leukocyte and kidney cDNA libraries to extend the original sequences more toward the 5' and/or 3' ends.

In view of the foregoing, it was concluded that channels of this type were expressed in many types of lymphocytes, and therefore were members of a new family of SOC/CRAC calcium channels.

### Experimental Procedures

### Screening of the cDNA libraries

Leukocyte and kidney cDNA libraries from Life Technologies (Gaithersburg, MD) were screened using the Gene Trapper II methodology (Life Technologies) according to manufacturer's recommendation, using the inserts of I.M.A.G.E. clone ID nos. 1312756 and 1076485 from ATCC (Manassas, VA), under stringent hybridization conditions. Using standard methodology ( Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York), individual cDNA clones were subjected to 3-4 rounds of amplification and purification under the same hybridization conditions.

After excision from the vector and subcloning of inserts into the plasmid forms, several clones were sequenced by the Beth Israel Deaconess Medical Center's Automated Sequencing Facility. Molecular biological techniques such as restriction enzyme treatment, subcloning, DNA extraction, bacterial culture and purification of DNA fragments were performed according to methods well known in the art. Computer analyses of protein and DNA sequences was done using "Assemblylign" (Oxford Molecular, Cambell, CA). Multiple alignments of the SOC/CRAC family members were produced using the CLUSTAL facility of the MacVector program. Restriction endonucleases, expression vectors, and modifying enzymes were purchased from commercial sources (Gibco-BRL). Sequencing vectors for DNA were purchased from Stratagene (La Jolla, CA).

Once the first members of what appeared to be a novel family of calcium channel receptors were identified and characterized, additional BLAST alignments were performed with the newly characterized nucleic acid sequences. An initial match was with genomic DNA fragment NH0332L11 (Genbank Acc. No. AC005538). Using this genomic sequence, promers were designed and a number of cDNA libraries was surveyed by PCR. A prostate specific message was identified and characterized, leading to the isolation and characterization of SOC-4/CRAC-3 (SEQ ID NOs: 31 and 32).

### Functional Assays

### Transient Expression of SOC/CRAC

In our initial transient expression experiments, we expressed or expect to express a SOC/CRAC molecule transiently in RBL-2H3 mast cells, Jurkat T cells, and A20 B-lymphocytes using both electroporation and vaccinia virus-driven expression, and measured the calcium influx produced by depletion of intracellular calcium stores with thapsigargin. Each of the foregoing techniques is well known to those of ordinary skill in the art and can be performed using various methods (see, e.g., Current Methods in Molecular Biology, eds. Ausubal, F.M., et al. 1987, Green Publishers and Wiley Interscience, N.Y., N.Y.). Exemplary methods are described herein.

Depletion of intracellular calcium stores is accomplished by treating the cells with 1 micromolar thapsigargin; alternative agents which function to deplete intracellular stores are described in by Putney, J.W. Jr., in Capacitative Calcium Entry, R.G. Landes Co. and Chapman & Hall, 1997 and include, for example, ionomycin, cyclopiazonic acid, and DBHQ.

Calcium influx is determined by measuring cytoplasmic calcium as indicated using the fura-2 fluorescent calcium indicator (see, e.g., G. Grynkiewicz, M. Poenie, R. Y. Tsien, A new generation of Ca2+ indicators with greatly improved fluorescence properties, J. Biol Chem 260, 3440-50 (1985), and M. Poenie, R. Tsien, Fura-2: a powerful new tool for measuring and imaging [Ca2+]i in single cells, Prog Clin Biol Res 210, 53-6 (1986)).

### Patch Clamp Analysis and Determining Selectivity of SOC/CRAC

*Patch clamp* analysis of cells injected with SOC/CRAC cRNA is performed by using the general patch technique as described in Neher, E., "Ion channels for communication between and within cells", Science, 1992; 256:498-502. Specific techniques for applying the patch clamp analysis to RBL cells are described in Hoth, M., and Penner, R., "Depletion of intracellular calcium stores activates a calcium current in mast cells", Nature, 1992; 355:3535-355. Additional protocols for applying the patch clamp technique to other cell types are described in Putney, J.W. Jr., in Capacitative Calcium Entry, R.G. Landes Co. and Chapman & Hall, 1997

An exemplary protocol for patch clamp analysis of SOC/CRAC molecule expressed in RBL-2H3 mast cells using a recombinant vaccinia virus is as follows. The currents elicited by store depletion are determined using the whole cell configuration (Neher, E., Science, 1992; 256:498-502). Currents in SOC/CRAC expressing cells are compared to currents in control cells expressing an irrelevant protein or a classic Trp family calcium channel known as VR1 (M. J. Caterina, et al., The capsaicin receptor: a heat-activated ion channel in the pain pathway [see comments], Nature 389, 816-24 (1997)) in order to assess the contribution of SOC/CRAC expression. In addition, the magnitude of whole cell currents in the presence of extracellular calcium (10 mM), barium (10 mM), or magnesium (10 mM) are compared to determine the relative permeability of the channels to each of these ions (Hoth, M., and Penner, R., Nature, 1992; 355:3535-355) and, thereby, determine the ionic selectivity.

### Pharmacologic Behaviour of SOC/CRAC

For analysis of the pharmacologic behavior of a SOC/CRAC molecule, a SOC/CRAC molecule is expressed in RBL-2H3 mast cells using a recombinant vaccinia virus, and the degree of calcium influx elicited by store depletion is monitored using a bulk spectrofluorimeter or a fluorescence microscope and the calcium sensitive dye fura-2 (G. Grynkiewicz, M. Poenie, R. Y. Tsien, A new generation of Ca2+ indicators with greatly improved fluorescence properties, J Biol Chem 260, 3440-50 (1985) and M. Poenie, R. Tsien, Fura-2: a powerful new tool for measuring and imaging [Ca2+]i in single cells, Prog Clin Biol Res 210, 53-6 (1986)). The level of cytoplasmic calcium in SOC/CRAC expressing cells is compared to the level achieved in control cells expressing an irrelevant protein or a classic Trp. family calcium channels known as VR1 (M. J. Caterina, et al., The capsaicin receptor: a heat-activated ion channel in the pain pathway [see comments], Nature 389, 816-24 (1997)). These cells then are pre-incubated with the desired pharmacologic reagent, and again the response to store depletion is monitored. Comparison of the effect of depleting stores in SOC/CRAC expressing cells relative to controls in the presence or absence of the pharmacologic reagent is used to assess the ability of that reagent to modulate SOC/CRAC activity. Sphingosine is an exemplary molecule that can be used as pharmacologic reagents for pharmacologic characterization of SOC/CRAC calcium channels. See, e.g., Mathes, C., et al., Calcium release activated calcium current as a direct target for sphingosine, J Biol Chem 273(39):25020-25030 (1998). Other non-specific calcium channel inhibitors that can be used for this purpose include SKR96365 (Calbiochem) and Lanthanum.

### Bulk Calcium Assays

Bulk calcium assays can be performed in a PTI Deltascan bulk spectrofluorometer using fura-2 as described in Scharenberg AM, et al., EMBO J, 1995, 14(14):3385-94.

### Gene Targeting

The method (and reagents) described by Buerstedde JM et al, (Cell, 1991, Oct 4;67(1):179-88), was used to generate "knockouts" in cells. Briefly, part of the chicken SOC-2/CRAC-1 genomic sequence coding for the transmembrane region was cloned utilizing the human sequence as the probe in a chicken library screen. Chicken SOC-2/CRAC-1 clones were isolated and characterized using standard methodology. The putative exon and domain arrangement of the chicken SOC-2/CRAC-1, is depicted in Figure 1. The exons coding for TM5 (pore region) and TM6, were replaced with promoter/antibiotic cassettes (see Figure 1). These targeting vectors were then used to target (and replace) the endogenous gene in DT-40 cells (chicken B lymphocyte cells).

### Results

### Example 1: Transient Expression of SOC/CRAC

In the above-identified cell lines and using both of the foregoing expression techniques, SOC/CRAC expression enhances thapsigargin-dependent influx. In addition, SOC/CRAC expression also enhances the amount of intracellular calcium stores. That this effect is likely due to SOC/CRAC acting as a plasma membrane calcium channel can be confirmed by producing an in-frame carboxy-terminal translational fusion with green fluorescent protein followed by confocal microscopy, revealing that SOC/CRAC is expressed predominantly as a plasma membrane calcium channel.

### Example 2: Patch Clamp Analysis

The biophysical characteristics of SOC/CRAC enhanced currents when expressed in Xenopus oocytes are determined. SOC/CRAC cRNA injection is able to enhance thapsigargin-dependent whole cell currents. In addition, SOC/CRAC does not alter the reversal potential of these currents and the determination of the P_{ca}/P_{Na} ratio shows that SOC/CRAC channels are highly calcium selective.

### Example 3: Pharmacologic Behavior of SOC/CRAC

The pharmacologic behavior of SOC/CRAC is evaluated as described above. SOC/CRAC-enhanced influx is inhibited by sphingosine in a manner that is substantially the same as that of endogenous thapsigargin-dependent calcium influx.

### Example 4: Gene targeting

Transfection of DT-40 cells with the foregoing targeting vectors, selection for antibiotic resistance, and screening, is collectivelly refered to, herein, as a round of targeting. For the first round of targeting SOC-2/CRAC-1, 18/24 clones with homologous recombination of the targeting construct into one of the endogenous SOC-2/CRAC-1 alleles were obtained. On the second round of targeting (in order to target the second allele and therefore generate a homozygous SOC-2/CRAC-1 mutant cell), 0/48 clones were obtained. These results indicate that a "null" SOC-2/CRAC-1 mutation is detrimental to DT-40 cells, and that SOC-2/CRAC-1 is required for cell viability.

**Table I. Nucleotide Sequences with homologies to SOC/CRAC nucleic acids**

| Sequences with SEQ ID NOs and GenBank accession numbers: |
|---|
| SEQ ID NO:9, AB001535, AI226731, H18835, AA419592, AA261842, AA419407, |
| AA592910, D86107, AI098310, AF071787, Z77132, Z83117, Z68333, AA708532, |
| AA551759, AA932133, R47363, N31660, AC005538, AA654650, AA370110, AA313170, |
| AA493512, AI670079, AI671853. |

**Table II. Amino Acid Sequences with homologies to SOC/CRAC polypeptides**

| Sequences with SEQ ID NOs and GenBank accession numbers: |
|---|
| SEQ ID NO:10, SEQ ID NO:13, SEQ ID NO:14, SEQ ID NO:15, SEQ ID NO:17, SEQ ID |
| NO:19, AB001535, AA592910, D86107, AF071787, Z77132, Z83117, Z68333, |
| AA708532, AA551759, AA932133, R47363, N31660, NP003298, CAB00861, NP002411, |
| CAA92726, CAB05572. |

## Claims

1. An isolated nucleic acid molecule, comprising:
(a) a nucleic acid molecule of SEQ.ID NO:29, or
(b) a nucleic acid sequence that differs from the nucleic acid sequence of (a) in codon sequence due to the degeneracy of the genetic code and which encodes a polypeptide of SEQ ID NO:30.

2. An isolated nucleic acid molecule comprising
(a) a nucleic acid molecule of SEQ ID NO:25 , or
(b) a nucleic acid sequence that differs from the nucleic acid sequence of (a) in codon sequence due to the degeneracy of the genetic code and which encodes a polypeptide of SEQ ID NO:26.

3. An isolated nucleic acid molecule comprising
(a) nucleotides 73-3714 of SEQ ID NO:29, or
(b) a nucleic acid sequence that differs from the nucleic acid sequence of (a) in codon sequence due to the degeneracy of the genetic code, and which encodes the same polypeptide as nucleotides 73-3714 of SEQ ID NO:29.

4. A full-length complement of the isolated nucleic acid of any of claims 1-3.

5. An expression vector comprising the isolated nucleic acid molecule of any of claims 1-3 operably linked to a promoter.

6. A host cell transformed or transfected with the expression vector of claim 5.

7. An isolated polypeptide, wherein the isolated polypeptide comprises a polypeptide having the sequence of amino acids set forth as SEQ ID NO:26 or SEQ ID NO:30.

8. A method for identifying agents useful in the modulation of Ca²⁺ flux, comprising:
(a) contacting a cell comprising a polypeptide encoded by the isolated nucleic acid of any of claims 1-3 with a candidate agent suspected of modulating SOC3/CRAC-2 activity, under conditions sufficient to allow the polypeptide to interact selectively with the candidate agent;
(b) detecting a Ca²⁺ concentration in the cell in the presence of the candidate agent; and
(c) comparing the Ca²⁺ concentration of step (b) with a control Ca²⁺ concentration in the cell in the absence of the candidate agent to determine whether the candidate agent modulates SOC-3/CRAC-2 activity.

9. A method for determining the level of SOC-3/CRAC-2 expression in a subject, comprising:
(a) measuring the expression of a polypeptide encoded by the isolated nucleic acid of any of claims 1-3 in a test sample obtained from the subject, and
(b) comparing the measured expression of the polypeptide in the test sample to the expression of the polypeptide-in a control to determine the level of SOC-3/CRAC-2 expression in the subject.

10. The method of claim 9, wherein the expression of the SOC-3/CRAC-2 in (b) is SOC-3/CRAC-2 mRNA expression.

11. The method of claim 9, wherein the expression of SOC-3/CRAC-2 in (b) is SOC-3/CRAC-2 polypeptide expression.

12. The method of claim 9, wherein the test sample is tissue.

13. The method of claim 9, wherein the test sample is a biological fluid.

14. The method of claim 10, wherein SOC-3/CRAG-2 mRNA expression is measured using the Polymerase Chain Reaction (PCR).

15. The method of claim 10, wherein SOC-3/CRAC-2 mRNA expression is measured using a method selected from the group consisting of northern blotting, monoclonal antisera to the polypeptide, and polyclonal antisera to the polypeptide.

16. A pharmaceutical composition comprising:
a pharmaceutically effective amount of an agent comprising an isolated nucleic acid molecule of any of claims 1-3 or an expression product thereof that encodes a polypeptide of SEQ ID NO:26 or 30, and a pharmaceutically acceptable carrier.

17. The pharmaceutical composition of claim 16, wherein the agent is an expression product of the isolated nucleic acid molecule of claim 1 that encodes a polypeptide of SEQ ID NO:30.

## Patentansprüche

1. Isoliertes Nukleinsäuremolekül umfassend:
(a) ein Nukleinsäuremolekül gemäß SEQ ID Nr. 29 oder
(b) eine Nukleinsäuresequenz, die sich von der Nukleinsäuresequenz in (a) in der Codonsequenz aufgrund der Degeneriertheit des genetischen Codes unterscheidet und ein Polypeptid nach SEQ ID Nr. 30 codiert.

2. Isoliertes Nukleinsäuremolekül umfassend:
(a) ein Nukleinsäuremolekül gemäß SEQ ID Nr. 25 oder
(b) eine Nukleinsäuresequenz, die sich von der Nukleinsäuresequenz in (a) in der Codonsequenz aufgrund der Degeneriertheit des genetischen Codes unterscheidet und ein Polypeptid nach SEQ ID Nr. 26 codiert.

3. Isoliertes Nukleinsäuremolekül umfassend:
(a) Nukleotide 73-3714 der SEQ ID Nr. 29 oder
(b) eine Nukleinsäuresequenz, die sich von der Nukleinsäuresequenz in (a) in der Codonsequenz aufgrund der Degeneriertheit des genetischen Codes unterscheidet und die das gleiche Polypeptid wie die Nukleotide 73-3714 in SEQ ID Nr. 29 codiert.

4. Komplement vollständiger Länge der isolierten Nukleinsäuren gemäß irgendeinem der Ansprüche 1 bis 3.

5. Expressionsvektor umfassend das isolierte Nukleinsäuremolekül gemäß irgendeinem der Ansprüche 1 bis 3, das operativ mit einem Promotor verbunden ist.

6. Wirtszelle, die mit dem Expressionsvektor gemäß Anspruch 5 transformiert oder transfiziert ist.

7. Isoliertes Polypeptid, wobei das isolierte Polypeptid ein Polypeptid umfaßt, das die Sequenz der Aminosäuren hat, die in SEQ ID Nr. 26 oder SEQ ID Nr. 30 aufgeführt sind.

8. Verfahren zum Identifizieren von Mitteln, die bei der Modulierung des Ca²⁺-Flußes nützlich sind, umfassend:
(a) Kontaktieren einer Zelle, die ein Polypeptid umfaßt, welches durch die isolierte Nukleinsäure gemäß irgendeinem der Ansprüche 1 bis 3 codiert wird, mit einer Kandidatensubstanz, von der vermutet wird, daß sie die SOC-3/CRAC-2-Aktivität unter Bedingungen moduliert, die ausreichen, um dem Polypeptid zu ermöglichen, selektiv mit der Kandidatensubstanz zu interagieren,
(b) Nachweisen einer Ca²⁺-Konzentration in der Zelle in Gegenwart der Kandidatensubstanz und
(c) Vergleichen der Ca²⁺-Konzentration des Schritts (b) mit einer Kontroll-Ca²⁺-Konzentration in der Zelle in Abwesenheit der Kandidatensubstanz, um festzustellen, ob die Kandidatensubstanz die SOC-3/CRAC-2-Aktivität moduliert.

9. Verfahren zum Bestimmen des Niveaus der SOC-3/CRAC-2-Expression in einem Individuum, umfassend:
(a) Messen der Expression eines Polypeptids, welches von der isolierten Nukleinsäure gemäß irgendeinem der Ansprüche 1 bis 3 codiert wird, in einer Testprobe, die von dem Individuum gewonnen wurde, und
(b) Vergleichen der gemessenen Expression des Polypeptids in der Testprobe mit der Expression des Polypeptids in einer Kontrolle, um das Niveau der SOC-3/CRAC-2-Expression in dem Individuum zu bestimmen.

10. Verfahren gemäß Anspruch 9, wobei die Expression von SOC-3/CRAC-2 in (b) die SOC-3/CRAC-2-mRNA-Expression ist.

11. Verfahren gemäß Anspruch 9, wobei die Expression von SOC-3/CRAC-2 in (b) die SOC-3/CRAC-2-Polypeptidexpression ist.

12. Verfahren gemäß Anspruch 9, wobei die Testprobe Gewebe ist.

13. Verfahren gemäß Anspruch 9, wobei die Testprobe eine biologische Flüssigkeit ist.

14. Verfahren gemäß Anspruch 10, wobei die SOC-3/CRAC-2-2mRNA-Expression unter Verwendung der Polymerase-Kettenreaktion (PCR) gemessen wird.

15. Verfahren nach Anspruch 10, wobei die SOC-3/CRAC-2-mRNA-Expression unter Verwendung eines Verfahrens gemessen wird, das aus der Gruppe ausgewählt ist, die aus Northern Blot, monoklonalen Antiseren gegen das Polypeptid und polyklonalen Antiseren gegen das Polypeptid besteht.

16. Pharmazeutische Zusammensetzung umfassend:
eine pharmazeutisch wirksame Menge einer Substanz, welche ein isoliertes Nukleinsäuremolekül gemäß irgendeinem der Ansprüche 1 bis 3 umfaßt oder ein Expressionsprodukt davon, welches ein Polypeptid nach SEQ ID Nr. 26 oder 30 codiert, und einen pharmazeutisch annehmbaren Träger.

17. Pharmazeutische Zusammensetzung gemäß Anspruch 16, wobei die Substanz ein Expressionsprodukt des isolierten Nukleinsäuremoleküls nach Anspruch 1 ist, welches ein Polypeptid der SEQ ID Nr. 30 codiert.

## Revendications

1. Molécule d'acide nucléique isolée, comprenant :
(a) une molécule d'acide nucléique de SEQ ID n° 29, ou
(b) une séquence d'acide nucléique qui diffère de la séquence d'acide nucléique de (a) dans la séquence des codons à cause de la dégénérescence du code génétique et qui code un polypeptide de SEQ ID n° 30.

2. Molécule d'acide nucléique isolée comprenant
(a) une molécule d'acide nucléique de SEQ ID n° 25, ou
(b) une séquence d'acide nucléique qui diffère de la séquence d'acide nucléique de (a) dans la séquence des codons à cause de la dégénérescence du code génétique et qui code un polypeptide de SEQ ID n° 26.

3. Molécule d'acide nucléique isolée comprenant
(a) les nucléotides 73 à 3714 de SEQ ID n° 29, ou
(b) une séquence d'acide nucléique qui diffère de la séquence d'acide nucléique de (a) dans la séquence des codons à cause de la dégénérescence du code génétique et qui code le même polypeptide que les nucléotides 73 à 3714 de SEQ ID n° 29.

4. Complémentaire pleine longueur de l'acide nucléique isolé selon l'une quelconque des revendications 1 à 3.

5. Vecteur d'expression comprenant la molécule d'acide nucléique isolée selon l'une quelconque des revendications 1 à 3 liée de manière fonctionnelle à un promoteur.

6. Cellule hôte transformée ou transfectée avec le vecteur d'expression selon la revendication 5.

7. Polypeptide isolé, où le polypeptide isolé comprend un polypeptide ayant la séquence d'acides aminés représentée par SEQ ID n° 26 ou SEQ ID n° 30.

8. Procédé d'identification d'agents utiles dans la modulation du flux de Ca²⁺, comprenant :
(a) la mise en contact d'une cellule comprenant un polypeptide codé par l'acide nucléique isolé selon l'une quelconque des revendications 1 à 3 avec un agent candidat suspecté de moduler l'activité de SOC3/CRAC-2, dans des conditions suffisantes pour permettre au polypeptide d'interagir sélectivement avec l'agent candidat ;
(b) la détection de la concentration de Ca²⁺ dans la cellule en présence de l'agent candidat ; et
(c) la comparaison de la concentration de Ca²⁺ de l'étape (b) avec une concentration de Ca²⁺ témoin dans la cellule en l'absence de l'agent candidat pour déterminer si l'agent candidat module l'activité de SOC-3/CRAC-2.

9. Procédé de détermination du taux d'expression de SOC-3/CRAC-2 chez un sujet, comprenant :
(a) la mesure de l'expression d'un polypeptide codé par l'acide nucléique isolé selon l'une quelconque des revendications 1 à 3 dans un échantillon à tester obtenu à partir du sujet, et
(b) la comparaison de l'expression mesurée du polypeptide dans l'échantillon à tester avec l'expression du polypeptide dans un témoin pour déterminer le taux d'expression de SOC-3/CRAC-2 chez le sujet.

10. Procédé selon la revendication 9, dans lequel l'expression de SOC-3/CRAC-2 dans (b) est l'expression de l'ARNm de SOC-3/CRAC-2.

11. Procédé selon la revendication 9, dans lequel l'expression de SOC-3/CRAC-2 dans (b) est l'expression du polypeptide de SOC-3/CRAC-2.

12. Procédé selon la revendication 9, dans lequel l'échantillon à tester est du tissu.

13. Procédé selon la revendication 9, dans lequel l'échantillon à tester est un liquide biologique.

14. Procédé selon la revendication 10, dans lequel l'expression de l'ARNm de SOC-3/CRAC-2 est mesurée en utilisant la réaction en chaîne de la polymérase (PCR).

15. Procédé selon la revendication 10, dans lequel l'expression de l'ARNm de SOC-3/CRAC-2 est mesurée en utilisant un procédé choisi dans le groupe constitué d'une analyse Northern blot, d'antisérums monoclonaux dirigés contre le polypeptide, et d'antisérums polyclonaux dirigés contre le polypeptide.

16. Composition pharmaceutique comprenant :
une quantité pharmaceutiquement efficace d'un agent comprenant une molécule d'acide nucléique isolé selon l'une quelconque des revendications 1 à 3 ou d'un produit d'expression de celui-ci qui code un polypeptide de SEQ ID n° 26 ou 30, et un support pharmaceutiquement acceptable.

17. Composition pharmaceutique selon la revendication 16, dans laquelle l'agent est un produit d'expression de la molécule d'acide nucléique isolée selon la revendication 1 qui code un polypeptide de SEQ ID n° 30.


### SEQUENCE LISTING

<110> Beth Israel Deaconess Medical Center, Inc. Scharenberg, Andrew
<120> CHARACTERIZATION OF A CALCIUM CHANNEL FAMILY
<130> B0662/7026WO/ERP/KA
<150> U.S. 60/114,220
   <151> 1998-12-30
<150> U.S. 60/120,018
   <151> 1999-01-29
<150> U.S. 60/140,415
   <151> 1999-06-22
<160> 32
<170> FastSEQ for Windows Version 3.0
<210> 1
   <211> 1212
   <212> DNA
   <213> Homo Sapiens
<400> 1
   gcacgaggca aattttttgt tagtacacca tctcagccaa gttgcaaaag ccacttggaa     60
   actggaacca aagatcaaga aactgtttgc tctaaagcta cagaaggaga taatacagaa    120
   tttggagcat ttgtaggaca cagagatagc atggatttac agaggtttaa agaaacatca    180
   aacaagataa aaatactatc caataacaat acttctgaaa acactttgaa acgagtgagt    240
   tctcttgctg gatttactga ctgtcacaga acttccattc ctgttcattc aaaacaagaa    300
   aaaatcagta gaaggccatc taccgaagac actcatgaag tagattccaa agcagcttta    360
   ataccggttt gtagatttca actaaacaga tatatattat taaatacatt aaactttttt     420
   agataagatc tacaaagtgg tgatatttgg gactatatca aaaattcaaa aaaatttttc    480
   ttaagaaaac tgactttagc atagtagcag ttacagaaaa gtttcttaca gtgaatagtc    540
   aggaatttta aagaaaaatt tatgcagaat aaaggcagga atctcttttt gtttgaattg    600
   aagctaatta tatgaactca tttccagcta actgcgataa tgattgattt tgcaaattcc    660
   ctttaaaagc acacactgac aagacaaaaa gctcaggaaa aggcagaaaa attactcctt    720
   tataatcaag tattatatat aagtcagtgc tcataatttt gctcaagaaa atattgactt    780
   acattcatat atatctgttc tggcatagag agattatgtt gttaaaatca tgttattgaa    840
   aaaagttatt tcagtgggga aagaggttag ttaacaaaga gattcacagt aacaaatcct    900
   cctttctgga gggactcttc ctgaccctga gctgcacaac tttgcaacaa attaaagcct    960
   aaccgaagat gacctcacaa tggcaattta gaactcatgg gagtcaactt acataaacgg   1020
   tatttgattt ctgataagat agtggaatta ttggttatag atgacaaaat aagtatgttt   1080
   aaagtgatga tggacataaa aaagttttaa atataaaaca tgagaaaaga aggagatact   1140
   attcaaaaag actggcaaat ttgaaaaact agaaataaaa aaaaaaaaaa aaaatgagcg   1200
   gccgcaagct tt                                                        1212
<210> 2
   <211> 141
   <212> PRT
   <213> Homo Sapiens
<400> 2
   Ala Arg Gly Lys Phe Phe Val Ser Thr Pro Ser Gln Pro Ser Cys Lys
   1               5                   10                  15
   Ser His Leu Glu Thr Gly Thr Lys Asp Gln Glu Thr Val Cys Ser Lys
               20                  25                  30
   Ala Thr Glu Gly Asp Asn Thr Glu Phe Gly Ala Phe Val Gly His Arg
           35                  40                  45
   Asp Ser Met Asp Leu Gln Arg Phe Lys Glu Thr Ser Asn Lys Ile Lys
       50                  55                  60
   Ile Leu Ser Asn Asn Asn Thr Ser Glu Asn Thr Leu Lys Arg Val Ser
   65                  70                  75                  80
   Ser Leu Ala Gly Phe Thr Asp Cys His Arg Thr Ser Ile Pro Val His
                   85                  90                  95
   Ser Lys Gln Glu Lys Ile Ser Arg Arg Pro Ser Thr Glu Asp Thr His
               100                 105                 110
   Glu Val Asp Ser Lys Ala Ala Leu Ile Pro Val Cys Arg Phe Gln Leu
           115                 120                 125
   Asn Arg Tyr Ile Leu Leu Asn Thr Leu Asn Phe Phe Arg
       130                 135                 140
<210> 3
   <211> 739
   <212> DNA
   <213> Homo Sapiens
<220>
   <221> unsure
   <222> (5)...(5)
   <223> UNKNOWN
<221> unsure
   <222> (21)...(22)
   <223> UNKNOWN
<221> unsure
   <222> (29)...(29)
   <223> UNKNOWN
<400> 3
   tcgantaggg gtcttccacc nncatactng gatgatggtt ggtgaagtct atgcatacga      60
   aattgatgtg tgtgcaaacg attctgttat ccctcaaatc tgtggtcctg ggacgtggtt     120
   gactccattt cttcaagcag tctacctctt tgwacagtat atcattatgg ttaatcttct     180
   tattgcattt ytcaacaatg tgtatttaca agtgaaggca atttccaata ttgyatggaa     240
   gtaccagcgt tatcatttta ttatggctta tcatgagaaa ccagttctgc ctcctccact     300
   tatcattctt agccatatag tttctctgtt ttgctgcata tgtaagagaa gaaagaaaga     360
   taagacttcc gatggaccaa aacttttctt aacagaagaa gatcaaaaga aacttcatga     420
   ttttgaagag cagtgtgttg aaatgtattt caatgaaaaa gatgacaaat ttcattctgg     480
   gagtgaagag agaattcgtg tcacttttga aagagtggaa cagatgtgca ttcagattaa     540
   agaagttgga gatccgtgtc aactacataa aaagatcatt acaatcatta gattctcaaa     600
   ttggccattt gcaagatctt tcagccctga cggtagatac attaaaaaca ctcactggcc     660
   aaaagcgtcg gaagctagca aagttcataa tgaaatcaca cgagaactga gcatttccaa     720
   acacttggct caaaacctt                                                739
<210> 4
   <211> 235
   <212> PRT
   <213> Homo Sapiens
<220>
   <221> UNSURE
   <222> (41)...(41)
   <223> UNKNOWN
<221> UNSURE
   <222> (54)...(54)
   <223> UNKNOWN
<221> UNSURE
   <222> (68)...(68)
   <223> UNKNOWN
<400> 4
   Met Met Val Gly Glu Val Tyr Ala Tyr Glu Ile Asp Val Cys Ala Asn
    1               5                  10                  15
   Asp Ser Val Ile Pro Gln Ile Cys Gly Pro Gly Thr Trp Leu Thr Pro
               20                  25                  30
   Phe Leu Gln Ala Val Tyr Leu Phe Xaa Gln Tyr Ile Ile Met Val Asn
           35                  40                  45
   Leu Leu Ile Ala Phe Xaa Asn Asn Val Tyr Leu Gln Val Lys Ala Ile
       50                  55                  60
   Ser Asn Ile Xaa Trp Lys Tyr Gln Arg Tyr His Phe Ile Met Ala Tyr
   65                  70                  75                  80
   His Glu Lys Pro Val Leu Pro Pro Pro Leu Ile Ile Leu Ser His Ile
                   85                  90                  95
   Val Ser Leu Phe Cys Cys Ile Cys Lys Arg Arg Lys Lys Asp Lys Thr
               100                 105                 110
   Ser Asp Gly Pro Lys Leu Phe Leu Thr Glu Glu Asp Gln Lys Lys Leu
           115                 120                 125
   His Asp Phe Glu Glu Gln Cys Val Glu Met Tyr Phe Asn Glu Lys Asp
       130                 135                 140
   Asp Lys Phe His Ser Gly Ser Glu Glu Arg Ile Arg Val Thr Phe Glu
   145                 150                 155                 160
   Arg Val Glu Gln Met Cys Ile Gln Ile Lys Glu Val Gly Asp Pro Cys
                   165                 170                 175
   Gln Leu His Lys Lys Ile Ile Thr Ile Ile Arg Phe Ser Asn Trp Pro
               180                 185                 190
   Phe Ala Arg Ser Phe Ser Pro Asp Gly Arg Tyr Ile Lys Asn Thr His
           195                 200                 205
   Trp Pro Lys Ala Ser Glu Ala Ser Lys Val His Asn Glu Ile Thr Arg
       210                 215                 220
   Glu Leu Ser Ile Ser Lys His Leu Ala Gln Asn
   225                 230                 235
<210> 5
   <211> 1579
   <212> DNA
   <213> Homo Sapiens
<220>
   <221> unsure
   <222> (368)...(368)
   <223> g or c
<221> unsure
   <222> (372)...(372)
   <223> g or c
<221> unsure
   <222> (374)...(374)
   <223> g or a
<221> unsure
   <222> (375)...(375)
   <223> g or c
<221> unsure
   <222> (387)...(387)
<221> unsure
   <222> (482)...(482)
<400> 5
   acgtcgcctg caggtaccgg tccggaattc ccgggtcgac ccacgcgtcc ggcatggtgt 60
   tgtaaataca cttagctcct ctcttcctca aggtgatctt gaaagtaata atccttttca 120
   ttgtaatatt ttaatgaaag atgacaaaga tccccagtgt aatatatttg gtcaagactt 180
   acctgcagta ccccagagaa aagaatttaa ttttccagag gctggttcct cttctggtgc 240
   cttattccca agtgctgttt cccctccaga actgcgacag agactacatg gggtagaact 300
   cttaaaaata tttaataaaa atcaaaaatt aggcagttca tctactagca taccacatct 360
   gtcatccsca csarscaaat tttttgntag tacaccatct cagccaagtt gcaaaagcca 420
   cttggaaact ggaaccaaag atcaagaaac tgtttgctct aaagctacag aaggagataa 480
   tncagaattt ggagcatttg taggacacag agatagcatg gatttacaga ggtttaaaga 540
   aacatcaaac aagataaaaa tactatccaa taacaatact tctgaaaaca ctttgaaacg 600
   agtgagttct cttgctggat ttactgactg tcacagaact tccattcctg ttcattcaaa 660
   acaagaaaaa atcagtagaa ggccatctac cgaagacact catgaagtag attccaaagc 720
   agctttaata ccggtttgta gatttcaact aaacagatat atattattaa atacattaaa 780
   cttttttaga taagatctac aaagtggtga tatttgggac tatatcaaaa attcaaaaaa 840
   atttttctta agaaaactga ctttagcata gtagcagtta cagaaaagtt tcttacagtg 900
   aatagtcagg aattttaaag aaaaatttat gcagaataaa ggcaggaatc tctttttgtt 960
   tgaattgaag ctaattatat gaactcattt ccagctaact gcgataatga ttgattttgc 1020
   aaattccctt taaaagcaca cactgacaag acaaaaagct caggaaaagg cagaaaaatt 1080
   actcctttat aatcaagtat tatatataag tcagtgctca taattttgct caagaaaata 1140
   ttgacttaca ttcatatata tctgttctgg catagagaga ttatgttgtt aaaatcatgt 1200
   tattgaaaaa agttatttca gtggggaaag aggttagtta acaaagagat tcacagtaac 1260
   aaatcctcct ttctggaggg actcttcctg accctgagct gcacaacttt gcaacaaatt 1320
   aaagcctaac cgaagatgac ctcacaatgg caatttagaa ctcatgggag tcaacttaca 1380
   taaacggtat ttgatttctg ataagatagt ggaattattg gttatagatg acaaaataag 1440
   tatgtttaaa gtgatgatgg acataaaaaa gttttaaata taaaacatga gaaaagaagg 1500
   agatactatt caaaaagact ggcaaatttg aaaaactaga aataaaaaaa aaaaaaaaaa 1560
   atgagcggcc gcaagcttt 1579
<210> 6
   <211> 243
   <212> PRT
   <213> Homo Sapiens
<220>
   <221> UNSURE
   <222> (103)...(105)
   <223> UNKNOWN
<221> UNSURE
   <222> (109)...(109)
   <223> UNKNOWN
<221> UNSURE
   <222> (141)...(141)
   <223> UNKNOWN
<400> 6
   Val Asn Thr Leu Ser Ser Ser Leu Pro Gln Gly Asp Leu Glu Ser Asn
   1 5 10 15
   Asn Pro Phe His Cys Asn Ile Leu Met Lys Asp Asp Lys Asp Pro Gln
   20 25 30
   Cys Asn Ile Phe Gly Gln Asp Leu Pro Ala Val Pro Gln Arg Lys Glu
   35 40 45
   Phe Asn Phe Pro Glu Ala Gly Ser Ser Ser Gly Ala Leu Phe Pro Ser
       50                  55                  60
   Ala Val Ser Pro Pro Glu Leu Arg Gln Arg Leu His Gly Val Glu Leu
   65                  70                  75                  80
   Leu Lys Ile Phe Asn Lys Asn Gln Lys Leu Gly Ser Ser Ser Thr Ser
                   85                  90                  95
   Ile Pro His Leu Ser Ser Xaa Xaa Xaa Lys Phe Phe Xaa Ser Thr Pro
               100                 105                 110
   Ser Gln Pro Ser Cys Lys Ser His Leu Glu Thr Gly Thr Lys Asp Gln
           115                 120                 125
   Glu Thr Val Cys Ser Lys Ala Thr Glu Gly Asp Asn Xaa Glu Phe Gly
       130                 135                 140
   Ala Phe Val Gly His Arg Asp Ser Met Asp Leu Gln Arg Phe Lys Glu
   145                 150                 155                 160
   Thr Ser Asn Lys Ile Lys Ile Leu Ser Asn Asn Asn Thr Ser Glu Asn
                   165                 170                 175
   Thr Leu Lys Arg Val Ser Ser Leu Ala Gly Phe Thr Asp Cys His Arg
               180                 185                 190
   Thr Ser Ile Pro Val His Ser Lys Gln Glu Lys Ile Ser Arg Arg Pro
           195                 200                 205
   Ser Thr Glu Asp Thr His Glu Val Asp Ser Lys Ala Ala Leu Ile Pro
       210                 215                 220
   Val Cys Arg Phe Gln Leu Asn Arg Tyr Ile Leu Leu Asn Thr Leu Asn
   225                 230                 235                 240
   Phe Phe Arg
<210> 7
   <211> 3532
   <212> DNA
   <213> Mus Musculus
<220>
   <221> unsure
   <222> (2420)...(2420)
   <223> unknown
<221> unsure
   <222> (2434)...(2434)
   <223> unknown
<221> unsure
   <222> (2461)...(2461)
   <223> unknown
<221> unsure
   <222> (2466)...(2466)
   <223> unknown
<221> unsure
   <222> (2470)...(2470)
   <223> unknown
<400> 7
   attatggctt atcatgaaaa accagtcctg cctcctcctc ttatcatcct cagccatata      60
   gtttcactgt tttgctgtgt atgcaaaaga agaaagaaag ataagacttc cgatgggcca     120
   aaacttttct taacagaaga agatcaaaag aaactccatg attttgaaga gcagtgtgtt     180
   gagatgtact ttgatgagaa agatgacaaa ttcaattctg ggagtgaaga gagaatccgg     240
   gtcacttttg aaagagtgga gcagatgagc attcagatta aagaagttgg agatcgtgtc     300
   aactacataa aaagatcatt acagtcttta gattctcaaa ttggtcatct gcaagatctc     360
   tcagccctaa cagtagatac attgaaaaca cttacagccc agaaagcttc agaagctagt     420
   aaagtgcaca atgagatcac acgagaattg agtatttcca aacacttggc tcagaatctt     480
   attgatgatg ttcctgtaag acctttgtgg gaagaaccta gtgctgtaaa cacactgagt     540
   tcctctcttc ctcaaggtga tcgggaaagt aataatcctt ttctttgtaa tatttttatg     600
   aaagatgaaa aagaccccca atataatctg tttggacaag atttgcccgt gataccccag     660
   agaaaagaat tcaacattcc agaggctggt tcctcctgtg gtgccttatt cccaagtgct     720
   gtttctcccc cagaattacg acagagacga catggggtag aaatgttaaa aatatttaat     780
   aaaaatcaaa aattaggcag ttcacctaat agttcaccac atatgtcctc cccaccaacc     840
   aaattttctg tgagtacccc atcccagcca agttgcaaaa gtcacttgga atccacaacc     900
   aaagatcaag aacccatttt ctataaagct gcagaagggg ataacataga atttggagca     960
   tttgtgggac acagagatag tatggactta cagaggttta aagaaacatc aaacaaaata    1020
   agagaactgt tatctaatga tactcctgaa aacactctga aacatgtggg tgctgctgga    1080
   tatagtgaat gttgtaagac ttctacttct cttcactcgg tgcaagcaga aagctgtagt    1140
   agaagagcgt cgacggaaga ctctccagaa gtcgattcta aagcagcttt gttaccggat    1200
   tggttacgag atagaccatc aaacagagaa atgccatctg aaggaggaac attaaatggt    1260
   cttgcttctc catttaagcc cgttttggat acaaattact attattcagc tgtggaaaga    1320
   aataacctga tgaggttgtc acagagtatt cccttcgttc ctgtacctcc acgaggcgag    1380
   cctgtcacag tgtaccgtct ggaggagagt tctcccagta tactgaataa cagcatgtct    1440
   tcatggtctc agctaggcct ctgtgccaaa attgagtttt taagtaaaga ggaaatggaa    1500
   ggtggtttac gaagagcagt caaagtgctg tgtacctggt cagagcacga tatcctgaag    1560
   tcagggcatc tctatatcat taagtcattt cttcctgagg tgataaacac atggtcaagc    1620
   atttataaag aagatacggt tctacatctc tgtctcagag aaatacaaca acagagagca    1680
   gcacaaaagc tcacatttgc ctttaatcag atgaaaccca aatccatacc atattctcca    1740
   aggttccttg aagttttcct gttgtactgc cattcagcag ggcagtggtt tgctgtagaa    1800
   gagtgcatga ctggtgaatt tagaaaatac aacaacaata atggtgatga aatcattcct    1860
   acaaatactc tagaagagat catgctagcc tttagccact ggacctatga atataccaga    1920
   ggggagttac tggtacttga cttacaagga gtgggagaaa acttgactga cccatctgta    1980
   ataaaagctg aagaaaaaag atcctgtgac atggtttttg gccctgccaa tctaggagaa    2040
   gatgcaataa aaaacttcaa gagccaaaca tccactgtaa ttcttgctgt cgaaagctta    2100
   aacttcccag atttgaagag gaatgactac acgcccttga taaaattata tttcctcagg    2160
   atgagtcatc agatttgaat cttcaatctg gaaattccac caaagaatca gaagcaacaa    2220
   attctgttcg tctgatgtta tagtgctgag tcattggttt ttgcctacac ttcacaaaag    2280
   tgtaactgtc agttttcctt tcgggggaat tgatgatata ggaagatgtg tgcaaaatga    2340
   gcttgctggc cccacacata gtctagaggt aatgttctca ttgaaaaacg cctggaggtg    2400
   gaggctgcag atgccagtgn aaagtgctag ctgncagaga gtcagtgctc tcgggctggt    2460
   naaggncggn acccttgctg ctgagagtgg tggttctctt cacctggtgc aggaccatta    2520
   accaaagtca agtcttcaga tttgattggc tgctcagtca cagcccattc agctaaggaa    2580
   actaaattgc gcagcttttt aaatggctga agtcttcctc agtttgtgct ctatgataat    2640
   gatgttagct ctcaactagg tgtttgtggc cacgggagaa ctactcctta caattttgct    2700
   tcacaggcat gttacaaagc ctgcactgaa aaccgtttgt cttccctctc tccctccctc    2760
   ttttccctgt agtattgagg atcaaaccca gggcctcatg aagaccattt tctaagagac    2820
   attttattta agaatcaact atagagtcta tgtttatgga tacagccagt ttttgttaaa    2880
   caaaacctga attgtgcaaa agggtttttt aacatttatc aatgttaagt aaaagaaagc    2940
   catgataaat aagaattaac tcactgttca atgggtgttt cctgtgagga aggttacagt    3000
   tgtaacagcc tgcagttgca tacatctcca aagatttaca gacttagtgt atcaaatcag    3060
   agtgtcatgt gagctctcac attgaaaatt ctataggaat gtgtcaatgt gaattctatt    3120
   tctggtactt aagaaatcag ttgttggatt atccttatac agtataggga gatcacaata    3180
   caactttatg ccaataaaat ctaacttaat tgcccagata tttttgcata tttagcaaca    3240
   agaaaagctt atcatttgac tcaagtttta tgctttctct ttcttttcat ttcctaggta    3300
   ctaattttaa tttttatttg gaaggagcag tgtaaagctt acttgtattc aatagtgtat    3360
   ctcatagata cagacaaggc cgcagagata agctgttaaa tagtgtttaa tgttgatgtg    3420
   gagagaaagg tgtattactt aaaaatacta taccatatac gttttgtata tcattaaatc    3480
   tttaaaagaa attaaattta ttcttgttta aaaaaaaaaa aaaaaaaaaa aa            3532
<210> 8
   <211> 475
   <212> PRT
   <213> Mus Musculus
<400> 8
   Ile Met Ala Tyr His Glu Lys Pro Val Leu Pro Pro Pro Leu Ile Ile
    1              5                   10                  15
   Leu Ser His Ile Val Ser Leu Phe Cys Cys Val Cys Lys Arg Arg Lys
               20                  25                  30
   Lys Asp Lys Thr Ser Asp Gly Pro Lys Leu Phe Leu Thr Glu Glu Asp
           35                  40                  45
   Gln Lys Lys Leu His Asp Phe Glu Glu Gln Cys Val Glu Met Tyr Phe
       50                  55                  60
   Asp Glu Lys Asp Asp Lys Phe Asn Ser Gly Ser Glu Glu Arg Ile Arg
   65                  70                  75                  80
   Val Thr Phe Glu Arg Val Glu Gln Met Ser Ile Gln Ile Lys Glu Val
                   85                  90                  95
   Gly Asp Arg Val Asn Tyr Ile Lys Arg Ser Leu Gln Ser Leu Asp Ser
               100                 105                 110
   Gln Ile Gly His Leu Gln Asp Leu Ser Ala Leu Thr Val Asp Thr Leu
           115                 120                 125
   Lys Thr Leu Thr Ala Gln Lys Ala Ser Glu Ala Ser Lys Val His Asn
       130                 135                 140
   Glu Ile Thr Arg Glu Leu Ser Ile Ser Lys His Leu Ala Gln Asn Leu
   145                 150                 155                 160
   Ile Asp Asp Val Pro Val Arg Pro Leu Trp Glu Glu Pro Ser Ala Val
                   165                 170                 175
   Asn Thr Leu Ser Ser Ser Leu Pro Gln Gly Asp Arg Glu Ser Asn Asn
               180                 185                 190
   Pro Phe Leu Cys Asn Ile Phe Met Lys Asp Glu Lys Asp Pro Gln Tyr
           195                 200                 205
   Asn Leu Phe Gly Gln Asp Leu Pro Val Ile Pro Gln Arg Lys Glu Phe
       210                 215                 220
   Asn Ile Pro Glu Ala Gly Ser Ser Cys Gly Ala Leu Phe Pro Ser Ala
   225                 230                 235                 240
   Val Ser Pro Pro Glu Leu Arg Gln Arg Arg His Gly Val Glu Met Leu
                   245                 250                 255
   Lys Ile Phe Asn Lys Asn Gln Lys Leu Gly Ser Ser Pro Asn Ser Ser
               260                 265                 270
   Pro His Met Ser Ser Pro Pro Thr Lys Phe Ser Val Ser Thr Pro Ser
           275                 280                 285
   Gln Pro Ser Cys Lys Ser His Leu Glu Ser Thr Thr Lys Asp Gln Glu
       290                 295                 300
   Pro Ile Phe Tyr Lys Ala Ala Glu Gly Asp Asn Ile Glu Phe Gly Ala
   305                 310                 315                 320
   Phe Val Gly His Arg Asp Ser Met Asp Leu Gln Arg Phe Lys Glu Thr
                   325                 330                 335
   Ser Asn Lys Ile Arg Glu Leu Leu Ser Asn Asp Thr Pro Glu Asn Thr
               340                 345                 350
   Leu Lys His Val Gly Ala Ala Gly Tyr Ser Glu Cys Cys Lys Thr Ser
           355                 360                 365
   Thr Ser Leu His Ser Val Gln Ala Glu Ser Cys Ser Arg Arg Ala Ser
       370                 375                 380
   Thr Glu Asp Ser Pro Glu Val Asp Ser Lys Ala Ala Leu Leu Pro Asp
   385                 390                 395                 400
   Trp Leu Arg Asp Arg Pro Ser Asn Arg Glu Met Pro Ser Glu Gly Gly
                   405                 410                 415
   Thr Leu Asn Gly Leu Ala Ser Pro Phe Lys Pro Val Leu Asp Thr Asn
               420                 425                 430
   Tyr Tyr Tyr Ser Ala Val Glu Arg Asn Asn Leu Met Arg Leu Ser Gln
           435                 440                 445
   Ser Ile Pro Phe Val Pro Val Pro Pro Arg Gly Glu Pro Val Thr Val
       450                 455                 460
   Tyr Pro Ser Gly Gly Arg Val Leu Pro Val Tyr
   465                 470                 475
<210> 9
   <211> 5433
   <212> DNA
   <213> Mus Musculus
<220>
   <221> unsure
   <222> (5094)...(5094)
   <223> unknown
<400> 9
   ggctgaaaga gcctgagctg tgcctctcca ttccactgct gtggcagggt cagaaatctt   60
   ggatagagaa aaccttttgc aaacgggaat gtatctttgt aattcctagc acgaaagact  120
   ctaacaggtg ttgctgtggc cagttcacca accagcatat cccccctctg ccaagtgcaa   180
   cacccagcaa aaatgaagag gaaagcaaac aggtggagac tcagcctgag aaatggtctg  240
   ttgccaagca cacccagagc tacccaacag attcctatgg agttcttgaa ttccagggtg  300
   gcggatattc caataaagcc atgtatatcc gtgtatccta tgacaccaag ccagactcac  360
   tgctccatct catggtgaaa gattggcagc tggaactccc caagctctta atatctgtgc  420
   atggaggcct ccagaacttt gagatgcagc ccaagctgaa acaagtcttt gggaaaggcc  480
   tgatcaaggc tgctatgacc accggggcct ggatcttcac cgggggtgtc agcacaggtg  540
   ttatcagcca cgtaggggat gccttgaaag accactcctc caagtccaga ggccgggttt  600
   gtgctatagg aattgctcca tggggcatcg tggagaataa ggaagacctg gttggaaagg  660
   atgtaacaag agtgtaccag accatgtcca accctctaag taagctctct gtgctcaaca  720
   actcccacac ccacttcatc ctggctgaca atggcaccct gggcaagtat ggcgccgagg  780
   tgaagctgcg aaggctgctg gaaaagcaca tctccctcca gaagatcaac acaagactgg  840
   ggcagggcgt gcccctcgtg ggtctcgtgg tggagggggg ccctaacgtg gtgtccatcg  900
   tcttggaata cctgcaagaa gagcctccca tccctgtggt gatttgtgat ggcagcggac  960
   gtgcctcgga catcctgtcc tttgcgcaca agtactgtga agaaggcgga ataataaatg  1020
   agtccctcag ggagcagctt ctagttacca ttcagaaaac atttaattat aataaggcac  1080
   aatcacatca gctgtttgca attataatgg agtgcatgaa gaagaaagaa ctcgtcactg  1140
   tgttcagaat gggttctgag ggccagcagg acatcgagat ggcaatttta actgccctgc  1200
   tgaaaggaac aaacgtatct gctccagatc agctgagctt ggcactggct tggaaccgcg  1260
   tggacatagc acgaagccag atctttgtct ttgggcccca ctggacgccc ctgggaagcc  1320
   tggcaccccc gacggacagc aaagccacgg agaaggagaa gaagccaccc atggccacca  1380
   ccaagggagg aagaggaaaa gggaaaggca agaagaaagg gaaagtgaaa gaggaagtgg  1440
   aggaagaaac tgacccccgg aagatagagc tgctgaactg ggtgaatgct ttggagcaag  1500
   cgatgctaga tgctttagtc ttagatcgtg tcgactttgt gaagctcctg attgaaaacg  1560
   gagtgaacat gcaacacttt ctgaccattc cgaggctgga ggagctctat aacacaagac  1620
   tgggtccacc aaacacactt catctgctgg tgagggatgt gaaaaagagc aaccttccgc  1680
   ctgattacca catcagcctc atagacatcg ggctcgtgct ggagtacctc atgggaggag  1740
   cctaccgctg caactacact cggaaaaact ttcggaccct ttacaacaac ttgtttggac  1800
   caaagaggcc taaagctctt aaacttctgg gaatggaaga tgatgagcct ccagctaaag  1860
   ggaagaaaaa aaaaaaaaag aaaaaggagg aagagatcga cattgatgtg gacgaccctg  1920
   ccgtgagtcg gttccagtat cccttccacg agctgatggt gtgggcagtg ctgatgaaac  1980
   gccagaaaat ggcagtgttc ctctggcagc gaggggaaga gagcatggcc aaggccctgg  2040
   tggcctgcaa gctctacaag gccatggccc acgagtcctc cgagagtgat ctggtggatg  2100
   acatctccca ggacttggat aacaattcca aagacttcgg ccagcttgct ttggagttat  2160
   tagaccagtc ctataagcat gacgagcaga tcgctatgaa actcctgacc tacgagctga  2220
   aaaactggag caactcgacc tgcctcaaac tggccgtggc agccaaacac cgggacttca  2280
   ttgctcacac ctgcagccag atgctgctga ccgatatgtg gatgggaaga ctgcggatgc  2340
   ggaagaaccc cggcctgaag gttatcatgg ggattcttct accccccacc atcttgtttt  2400
   tggaatttcg cacatatgat gatttctcgt atcaaacatc caaggaaaac gaggatggca  2460
   aagaaaaaga agaggaaaat acggatgcaa atgcagatgc tggctcaaga aagggggatg  2520
   aggagaacga gcataaaaaa cagagaagta ttcccatcgg aacaaagatc tgtgaattct  2580
   ataacgcgcc cattgtcaag ttctggtttt acacaatatc atacttgggc tacctgctgc  2640
   tgtttaacta cgtcatcctg gtgcggatgg atggctggcc gtccctccag gagtggatcg  2700
   tcatctccta catcgtgagc ctggcgttag agaagatacg agagatcctc atgtcagaac  2760
   caggcaaact cagccagaaa atcaaagttt ggcttcagga gtactggaac atcacagatc  2820
   tcgtggccat ttccacattc atgattggag caattcttcg cctacagaac cagccctaca  2880
   tgggctatgg ccgggtgatc tactgtgtgg atatcatctt ctggtacatc cgtgtcctgg    2940
   acatctttgg tgtcaacaag tatctggggc catacgtgat gatgattgga aagatgatga    3000
   tcgacatgct gtactttgtg gtcatcatgc tggtcgtgct catgagtttc ggagtagccc    3060
   gtcaagccat tctgcatcca gaggagaagc cctcttggaa actggcccga aacatcttct    3120
   acatgcccta ctggatgatc tatggagagg tgtttgcaga ccagatagac ctctacgcca    3180
   tggaaattaa tcctccttgt ggtgagaacc tatatgatga ggagggcaag cggcttcctc    3240
   cctgtatccc cggcgcctgg ctcactccag cactcatggc gtgctatcta ctggtcgcca    3300
   acatcctgct ggtgaacctg ctgattgctg tgttcaacaa tactttcttt gaagtaaaat    3360
   caatatccaa ccaggtgtgg aagttccagc gatatcagct gattatgaca tttcatgaca    3420
   ggccagtcct gcccccaccg atgatcattt taagccacat ctacatcatc attatgcgtc    3480
   tcagcggccg ctgcaggaaa aagagagaag gggaccaaga ggaacgggat cgtggattga    3540
   agctcttcct tagcgacgag gagctaaaga ggctgcatga gttcgaggag cagtgcgtgc    3600
   aggagcactt ccgggagaag gaggatgagc agcagtcgtc cagcgacgag cgcatccggg    3660
   tcacttctga aagagttgaa aatatgtcaa tgaggttgga agaaatcaat gaaagagaaa    3720
   cttttatgaa aacttccctg cagactgttg accttcgact tgctcagcta gaagaattat    3780
   ctaacagaat ggtgaatgct cttgaaaatc ttgcgggaat cgacaggtct gacctgatcc    3840
   aggcacggtc ccgggcttct tctgaatgtg aggcaacgta tcttctccgg caaagcagca    3900
   tcaatagcgc tgatggctac agcttgtatc gatatcattt taacggagaa gagttattat    3960
   ttgaggatac atctctctcc acgtcaccag ggacaggagt caggaaaaaa acctgttcct    4020
   tccgtataaa ggaagagaag gacgtgaaaa cgcacctagt cccagaatgt cagaacagtc    4080
   ttcacctttc actgggcaca agcacatcag caaccccaga tggcagtcac cttgcagtag    4140
   atgacttaaa gaacgctgaa gagtcaaaat taggtccaga tattgggatt tcaaaggaag    4200
   atgatgaaag acagacagac tctaaaaaag aagaaactat ttccccaagt ttaaataaaa    4260
   cagatgtgat acatggacag gacaaatcag atgttcaaaa cactcagcta acagtggaaa    4320
   cgacaaatat agaaggcact atttcctatc ccctggaaga aaccaaaatt acacgctatt    4380
   tccccgatga aacgatcaat gcttgtaaaa caatgaagtc cagaagcttc gtctattccc    4440
   ggggaagaaa gctggtcggt ggggttaacc aggatgtaga gtacagttca atcacggacc    4500
   agcaattgac gacggaatgg caatgccaag ttcaaaagat cacgcgctct catagcacag    4560
   atattcctta cattgtgtcg gaagctgcag tgcaagctga gcaaaaagag cagtttgcag    4620
   atatgcaaga tgaacaccat gtcgctgaag caattcctcg aatccctcgc ttgtccctaa    4680
   ccattactga cagaaatggg atggaaaact tactgtctgt gaagccagat caaactttgg    4740
   gattcccatc tctcaggtca aaaagtttac atggacatcc taggaatgtg aaatccattc    4800
   agggaaagtt agacagatct ggacatgcca gtagtgtaag cagcttagta attgtgtctg    4860
   gaatgacagc agaagaaaaa aaggttaaga aagagaaagc ttccacagaa actgaatgct    4920
   agtctgtttt gtttctttaa tttttttttt taacagtcag aaacccacta atgggtgtca    4980
   tcttggccca tcctaaacac atmtccaatt tcctaaaaac attttccctt aaaaaatttt    5040
   ggaaattcag acttgattta caatttaatg cactaaaagt agtattttgt tagnatatgt    5100
   tagtaggctt agttttttca gttgcagtag tatcaaatga aagtgatgat actgtaacga    5160
   agataaattg gctaatcagt atacaagatt atacaatctc tttattactg agggccacca    5220
   aatagcctag gaagtgccct cgagcactga agtcaccatt aggtcactca agaagtaagc    5280
   aactagctgg gcacagtggc tcatgcctgt aatcctagca ctttgggagg ccaaggcaga    5340
   aagatagctt gagtccagga gtttgagacc agcctgggca acatagtgat accccatctc    5400
   ttaaaaaaaa aaaaaaaaaa ctgccctcgt gcc                                 5433
<210> 10
   <211> 1533
   <212> PRT
   <213> Mus Musculus
<400> 10
<210> 11
   <211> 6220
   <212> DNA
   <213> Homo Sapiens
<400> 11
   tgtgcagaat tgtacagttg cgaaaccatg tcgctggcag ctggtgctgg cggtggagac 60
   ttccctgtgc ggtgctcagt gcatctgcac ccgtggggga gggagctctt tctctggccc 120
   tgcagtcacc tgaggttgtt accattatga acggccgctg ggacccccgc atgtgcatgt 180
   actcccccag agtgtccggg ggccccagcc aagggacaca tctcacgcag ctgggaacat 240
   gtgcaggctg atgaagagaa ccggatgagg gcttcacatg aggaagcatg tggccaggtc 300
   ctctcagaac atcagcctca tcttcctgtc tctgatctat ttcaccaacc accccatgtg 360
   tctctagaac cccagtgtag cgagctggag agaggactgt cctgagggca gcaggcctgg 420
   ttgcagctgg cgtgggggtc tcagaatgga gccctcagcc ctgaggaaag ctggctcgga 480
   gcaggaggag ggctttgagg ggctgcccag aagggtcact gacctgggga tggtctccaa 540
   tctccggcgc agcaacagca gcctcttcaa gagctggagg ctacagtgcc ccttcggcaa 600
   caatgacaag caagaaagcc tcagttcgtg gattcctgaa aacatcaaga agaaagaatg 660
   cgtgtatttt gtggaaagtt ccaaactgtc tgatgctggg aaggtggtgt gtcagtgtgg 720
   ctacacgcat gagcagcact tggaggaggc taccaagccc cacaccttcc agggcacaca 780
   gtgggaccca aagaaacatg tccaggagat gccaaccgat gcctttggcg acatcgtctt 840
   cacgggcctg agccagaagg tgaaaaagta cgtccgagtc tcccaggaca cgccctccag 900
   cgtgatctac cacctcatga cccagcactg ggggctggac gtccccaatc tcttgatctc 960
   ggtgaccggg ggggccaaga acttcaacat gaagccgcgg ctgaagagca ttttccgcag 1020
   aggcctggtc aaggtggctc agaccacagg ggcctggatc atcacagggg ggtcccacac 1080
   cggcgtcatg aagcaggtag gcgaggcggt gcgggacttc agcctgagca gcagctacaa 1140
   ggaaggcgag ctcatcacca tcggagtcgc cacctggggc actgtccacc gccgcgaggg 1200
   cctgatccat cccacgggca gcttccccgc cgagtacata ctggatgagg atggccaagg 1260
   gaacctgacc tgcctagaca gcaaccactc tcacttcatc ctcgtggacg acgggaccca 1320
   cggccagtac ggggtggaga ttcctctgag gaccaggctg gagaagttca tatcggagca 1380
   gaccaaggaa agaggaggtg tggccatcaa gatccccatc gtgtgcgtgg tgctggaggg 1440
   cggcccgggc acgttgcaca ccatcgacaa cgccaccacc aacggcaccc cctgtgtggt 1500
   tgtggagggc tcgggccgcg tggccgacgt cattgcccag gtggccaacc tgcctgtctc 1560
   ggacatcact atctccctga tccagcagaa actgagcgtg ttcttccagg agatgtttga 1620
   gaccttcacg gaaagcagga ttgtcgagtg gaccaaaaag atccaagata ttgtccggag 1680
   gcggcagctg ctgactgtct tccgggaagg caaggatggt cagcaggacg tggatgtggc 1740
   catcttgcag gccttgctga aagcctcacg gagccaagac cactttggcc acgagaactg 1800
   ggaccaccag ctgaaactgg cagtggcatg gaatcgcgtg gacattgccc gcagtgagat 1860
   cttcatggat gagtggcagt ggaagccttc agatctgcac cccacgatga cagctgcact 1920
   catctccaac aagcctgagt ttgtgaagct cttcctggaa aacggggtgc agctgaagga 1980
   gtttgtcacc tgggacacct tgctctacct gtacgagaac ctggacccct cctgcctgtt 2040
   ccacagcaag ctgcaaaagg tgctggtgga ggatcccgag cgcccggctt gcgcgcccgc 2100
   ggcgccccgc ctgcagatgc accacgtggc ccaggtgctg cgggagctgc tgggggactt 2160
   cacgcagccg ctttatcccc ggccccggca caacgaccgg ctgcggctcc tgctgcccgt 2220
   tccccacgtc aagctcaacg tgcagggagt gagcctccgg tccctctaca agcgttcctc 2280
   aggccatgtg accttcacca tggaccccat ccgtgacctt ctcatttggg ccattgtcca 2340
   gaaccgtcgg gagctggcag gaatcatctg ggctcagagc caggactgca tcgcagcggc 2400
   cttggcctgc agcaagatcc tgaaggaact gtccaaggag gaggaggaca cggacagctc 2460
   ggaggagatg ctggcgctgg cggaggagta tgagcacaga gccatcgggg tcttcaccga 2520
   gtgctaccgg aaggacgaag agagagccca gaaactgctc acccgcgtgt ccgaggcctg 2580
   ggggaagacc acctgcctgc agctcgccct ggaggccaag gacatgaagt ttgtgtctca 2640
   cgggggcatc caggccttcc tgaccaaggt gtggtggggc cagctctccg tggacaatgg 2700
   gctgtggcgt gtgaccctgt gcatgctggc cttcccgctg ctcctcaccg gcctcatctc 2760
   cttcagggag aagaggctgc aggatgtggg cacccccgcg gcccgcgccc gtgccttctt 2820
   caccgcaccc gtggtggtct tccacctgaa catcctctcc tacttcgcct tcctctgcct 2880
   gttcgcctac gtgctcatgg tggacttcca gcctgtgccc tcctggtgcg agtgtgccat 2940
   ctacctctgg ctcttctcct tggtgtgcga ggagatgcgg cagctcttct atgaccctga 3000
   cgagtgcggg ctgatgaaga aggcagcctt gtacttcagt gacttctgga ataagctgga 3060
   cgtcggcgca atcttgctct tcgtggcagg gctgacctgc aggctcatcc cggcgacgct 3120
   gtaccccggg cgcgtcatcc tctctctgga cttcatcctg ttctgcctcc ggctcatgca 3180
   catttttacc atcagtaaga cgctggggcc caagatcatc attgtgaagc ggatgatgaa 3240
   ggacgtcttc ttcttcctct tcctgctggc tgtgtgggtg gtgtccttcg gggtggccaa 3300
   gcaggccatc ctcatccaca acgagcgccg ggtggactgg ctgttccgag gggccgtcta 3360
   ccactcctac ctcaccatct tcgggcagat cccgggctac atcgacggtg tgaacttcaa 3420
   cccggagcac tgcagcccca atggcaccga cccctacaag cctaagtgcc ccgagagcga 3480
   cgcgacgcag cagaggccgg ccttccctga gtggctgacg gtcctcctac tctgcctcta 3540
   cctgctcttc accaacatcc tgctgctcaa cctcctcatc gccatgttca actacacctt 3600
   ccagcaggtg caggagcaca cggaccagat ttggaagttc cagcgccatg acctgatcga 3660
   ggagtaccac ggccgccccg ccgcgccgcc ccccttcatc ctcctcagcc acctgcagct 3720
   cttcatcaag agggtggtcc tgaagactcc ggccaagagg cacaagcagc tcaagaacaa 3780
   gctggagaag aacgaggagg cggccctgct atcctgggag atctacctga aggagaacta 3840
   cctccagaac cgacagttcc agcaaaagca gcggcccgag cagaagatcg aggacatcag 3900
   caataaggtt gacgccatgg tggacctgct ggacctggac ccactgaaga ggtcgggctc 3960
   catggagcag aggttggcct ccctggagga gcaggtggcc cagacagccc gagccctgca 4020
   ctggatcgtg aggacgctgc gggccagcgg cttcagctcg gaggcggacg tccccactct 4080
   ggcctcccag aaggccgcgg aggagccgga tgctgagccg ggaggcagga agaagacgga 4140
   ggagccgggc gacagctacc acgtgaatgc ccggcacctc ctctacccca actgccctgt 4200
   cacgcgcttc cccgtgccca acgagaaggt gccctgggag acggagttcc tgatctatga 4260
   cccacccttt tacacggcag agaggaagga cgcggccgcc atggacccca tgggagacac 4320
   cctggagcca ctgtccacga tccagtacaa cgtggtggat ggcctgaggg accgccggag 4380
   cttccacggg ccgtacacag tgcaggccgg gttgcccctg aaccccatgg gccgcacagg 4440
   actgcgtggg cgcgggagcc tcagctgctt cggacccaac cacacgctgt accccatggt 4500
   cacgcggtgg aggcggaacg aggatggagc catctgcagg aagagcataa agaagatgct 4560
   ggaagtgctg gtggtgaagc tccctctctc cgagcactgg gccctgcctg gggctcccgg 4620
   ggagccaggg gagatgctac ctcggaagct gaagcggatc ctccggcagg agcactggcc 4680
   gtcttttgaa aacttgctga agtgcggcat ggaggtgtac aaaggctaca tggatgaccc 4740
   gaggaacacg gacaatgcct ggatcgagac ggtggccgtc agcgtccact tccaggacca 4800
   gaatgacgtg gagctgaaca ggctgaactc taacctgcac gcctgcgact cgggggcctc 4860
   catccgatgg caggtggtgg acaggcgcat cccactctat gcgaaccaca agaccctcct 4920
   ccagaaggca gccgctgagt tcggggctca ctactgactg tgccctcagg ctgggcggct 4980
   ccagtccata gacgttcccc ccagaaacca gggcttctct ctcctgagcc tggccaggac 5040
   tcaggctgtt cctgggccct gcacatgatg gggtttggtg gacccagtgc ccctcacggc 5100
   tgccgcaagt ctgctgcaga tgacctcatg aactggaagg ggtcaaggtg acccgggagg 5160
   agagctcaag acagggcaca ggctactcag agctgagggg cccctgggac ccttggccat 5220
   caggcgaggg gctgggcctg tgcagctggg cccttggcca gagtccactc ccttcctggc 5280
   tgtgtcaccc cgagcagctc atccaccatg gaggtcattg gcctgaggca agttccccgg 5340
   agagtcggga tcccctgtgg ccccctcagg cctatgtctg tgaggaaggg gccctgccac 5400
   tctccccaag agggcctcca tgtttcgagg tgcctcaaca tggagccttg cctggcctgg 5460
   gctaggggca ctgtctgaac tcctgactgt caggataaac tccgtggggg tacaggagcc 5520
   cagacaaagc ccaggcctgt caagagacgc agagggcccc tgccagggtt ggccccaggg 5580
   accctgggac gaggctgcag aagctctccc tccctactcc ctgggagcca cgtgctggcc 5640
   atgtggccag ggacggcatg agcaggaggc ggggacgtgg gggccttctg gtttggtgtc 5700
   aacagctcac aggagcgtga accatgaggg ccctcaggag gggaacgtgg taaaacccaa 5760
   gacattaaat ctgccatctc aggcctggct ggctcttctg tgctttccac aaataaagtt 5820
   cctgacacgt ccagggccag gggctgtgtg acggctgcct gaagttctcc tcgatccccc 5880
   ggtgagcttc ctgcagcctg tggatgtcct gcagcccctc agccctaccc ccaagtttct 5940
   cctctgaccc atcagctccc tgtcttcatt ttcctaaacc tgggctccag catcgtcccc 6000
   aagcccacca ggccaggatg caggcatcca catgccctcc tccttggctt cccctgcgtg 6060
   gtggtgccaa tgtgccctgg cacccctgca gaggctccgg atggagcctg gggctgcctg 6120
   gccactgagc actggccgag gtgatgccca cccttccctg gacaggcctc tgtcttccac 6180
   ctgacccaaa gctctctagc cacccccttg tccccagtat 6220
<210> 12
   <211> 1503
   <212> PRT
   <213> Homo Sapiens
<400> 12
<210> 13
   <211> 1816
   <212> PRT
   <213> C. Elegans
<400> 13
<210> 14
   <211> 1387
   <212> PRT
   <213> C. Elegans
<400> 14
<210> 15
   <211> 1868
   <212> PRT
   <213> C. Elegans
<400> 15
<210> 16
   <211> 489
   <212> DNA
   <213> Mus Musculus
<400> 16
   ccctgaaaga ctcgacttct gctgctagcg ctggagctga gttagttttg agaaggtttc      60
   ccggggctgt ccttgttcgg tggcccgtgc caccgcctcc ggagacgctt tccgatagat     120
   ggctgcaggc cgcggaggtg gaggaggagc cgctgccctt ccggagtccg ccccgtgagg     180
   agaatgtccc agaaatcctg gatagagagc actttgacca agagggagtg tgtatatatt     240
   ataccaagct ccaaagaccc tcacagatgt cttccaggat gtcagatttg tcagcaactt     300
   gtcagatgtt tctgtggtcg tttggtcaag caacatgcat gctttactgc aagtcttgcc     360
   atgaaatact cagatgtgaa attgggtgaa cactttaacc aggcaataga agaatggtct     420
   gtggaaaagc acacggagca gagcccaaca gatgcttatg gagtcatcaa ttttcaaggg     480
   ggttctcat                                                             489
<210> 17
   <211> 102
   <212> PRT
   <213> Mus Musculus
<400> 17
   Met Ser Gln Lys Ser Trp Ile Glu Ser Thr Leu Thr Lys Arg Glu Cys
    1               5                  10                  15
   Val Tyr Ile Ile Pro Ser Ser Lys Asp Pro His Arg Cys Leu Pro Gly
               20                  25                  30
   Cys Gln Ile Cys Gln Gln Leu Val Arg Cys Phe Cys Gly Arg Leu Val
           35                  40                  45
   Lys Gln His Ala Cys Phe Thr Ala Ser Leu Ala Met Lys Tyr Ser Asp
       50                  55                  60
   Val Lys Leu Gly Glu His Phe Asn Gln Ala Ile Glu Glu Trp Ser Val
   65                  70                  75                  80
   Glu Lys His Thr Glu Gln Ser Pro Thr Asp Ala Tyr Gly Val Ile Asn
                   85                  90                  95
   Phe Gln Gly Gly Ser His
               100
<210> 18
   <211> 410
   <212> DNA
   <213> Homo Sapiens
<220>
   <221> unsure
   <222> (6)...(6)
<221> unsure
   <222> (58)...(58)
<221> unsure
   <222> (89)...(89)
<221> unsure
   <222> (406)...(406)
<400> 18
   gccgcnggag cctgagcgga gggtgtgcgc agcctcgcca gcgggggccc cgggctgngc      60
   cattgcctca ctgagccagc gcctgcctnc tacctcgccg acagctggaa ccagtgcgac     120
   ctagtggctc tcacctgctt cctcctgggc gtgggctgcc ggctgacccc gggtttgtac     180
   cacctgggcc gcactgtcct ctgcatcgac ttcatggttt tcacggtgcg gctgcttcac     240
   atcttcacgg tcaacaaaca gctggggccc aagatcgtca tcgtgagcaa gatgatgaag     300
   gacgtgttct tcttcctctt cttcctcggc gtgtggctgg tagctatggg ttgggccacg     360
   gaggggttcc tgaggccacg ggacagtgac ttcccaagta tcctgncgcc                410
<210> 19
   <211> 131
   <212> PRT
   <213> Homo Sapiens
<220>
   <221> UNSURE
   <222> (15)...(15)
   <223> UNKNOWN
<221> UNSURE
   <222> (25)...(25)
   <223> UNKNOWN
<221> UNSURE
   <222> (131)...(131)
   <223> UNKNOWN
<400> 19
   Ala Glu Gly Val Arg Ser Leu Ala Ser Gly Gly Pro Gly Leu Xaa His
   1 5 10 15
   Cys Leu Thr Glu Pro Ala Pro Ala Xaa Tyr Leu Ala Asp Ser Trp Asn
   20 25 30
   Gln Cys Asp Leu Val Ala Leu Thr Cys Phe Leu Leu Gly Val Gly Cys
   35 40 45
   Arg Leu Thr Pro Gly Leu Tyr His Leu Gly Arg Thr Val Leu Cys Ile
   50 55 60
   Asp Phe Met Val Phe Thr Val Arg Leu Leu His Ile Phe Thr Val Asn
   65 70 75 80
   Lys Gln Leu Gly Pro Lys Ile Val Ile Val Ser Lys Met Met Lys Asp
   85 90 95
   Val Phe Phe Phe Leu Phe Phe Leu Gly Val Trp Leu Val Ala Met Gly
   100 105 110
   Trp Ala Thr Glu Gly Phe Leu Arg Pro Arg Asp Ser Asp Phe Pro Ser
   115 120 125
   Ile Leu Xaa
   130
<210> 20
   <211> 389
   <212> DNA
   <213> Homo Sapiens
<400> 20
   caaatttttt gttagtacac catctcatcc aaattgcaaa agtcacatgg aaactggaac 60
   caaagatcaa gaaactgttt gctctaaagc tacagaagga gataatacag aatttggagc 120
   atttgtagga cacagagata gcatggattt acagaggttt aaagaaacat caaacaagat 180
   aaaaatacta tccaataaca atacttctga aaacactttg aaacgagtga gttctcttgc 240
   tggatttact gactgtcaca gaacttccat tcctgttcat tcaaaacgag aaaagatcag 300
   tagaaggcca tctaccgaag acactcatga agtagattcc aaagcagctt taataccggt 360
   ttgtagattt caactaaaca gatatatat 389
<210> 21
   <211> 415
   <212> DNA
   <213> Homo Sapiens
<400> 21
   atttctagtt tttcaaattt gccagtcttt ttgaatagta tctccttctt ttctcatgtt 60
   ttatatttaa aactttttta tgtccatcat cactttaaac atacttattt tgtcatctat 120
   aaccaataat tccactatct tatcagaaat caaataccgt ttatgtaagt tgactcccat 180
   gagttctaaa ttgccattgt gaggtcatct tcggttaggc tttaatttgt tgcaaagttg 240
   tgcagctcag ggtcaggaag agtccctcca gaaaggagga tttgttactg tgaatctctt 300
   tgttaactaa cctctttccc cactgaaata acttttttca ataacatgat tttaacaaca 360
   taatctctct atgccagaac agatatatat gaatgtaagt caatattttc ttgag 415
<210> 22
   <211> 405
   <212> DNA
   <213> Mus Musculus
<400> 22
   ttattatggc ttatcatgaa aaaccagtcc tgcctcctcc tcttatcatc ctcagccata      60
   tagtttcact gttttgctgt gtatgcaaaa gaagaaagaa agataagact tccgatgggc     120
   caaaactttt cttaacagaa gaagatcaaa agaaactcca tgattttgaa gagcagtgtg     180
   ttgagatgta ctttgatgag aaagatgaca aattcaattc tgggagtgaa gagagaatcc     240
   gggtcacttt tgaaagagtg gagcagatga gcattcagat taaagaagtt ggagatcgtg     300
   tcaactacat aaaaagatca ttacagtctt tagattctca aattggtcat ctgcaagatc     360
   tctcagccct aacagtagat acattgaaaa cacttacagc ccaga                     405
<210> 23
   <211> 5117
   <212> DNA
   <213> Homo Sapiens
<220>
   <221> unsure
   <222> (2382)...(2382)
   <223> unknown
<221> unsure
   <222> (4664)...(4664)
   <223> unknown
<221> unsure
   <222> (4682)...(4682)
   <223> unknown
<221> unsure
   <222> (4702)...(4702)
   <223> unknown
<221> unsure
   <222> (5038)...(5039)
   <223> unknown
<221> unsure
   <222> (5056)...(5056)
   <223> unknown
<221> unsure
   <222> (5071)...(5072)
<400> 23
<210> 24
   <211> 1224
   <212> PRT
   <213> Homo Sapiens
<220>
   <221> UNSURE
   <222> (794)...(794)
   <223> UNKNOWN
<400> 24
<210> 25
   <211> 2180
   <212> DNA
   <213> Homo Sapiens
<400> 25
   tcgaggccaa gaattcggca cgagggcctc gggcaggccc cctggagcga cctgcttctt      60
   tgggcactgt tgctgaacag ggcacagatg gccatgtact tctgggagat gggttccaat     120
   gcagtttcct cagctcttgg ggcctgtttg ctgctccggg tgatggcacg cctggagcct     180
   gacgctgagg aggcagcacg gaggaaagac ctggcgttca agtttgaggg gatgggcgtt     240
   gacctctttg gcgagtgcta tcgcagcagt gaggtgaggg ctgcccgcct cctcctccgt     300
   cgctgcccgc tctgggggga tgccacttgc ctccagctgg ccatgcaagc tgacgcccgt     360
   gccttctttg cccaggatgg ggtacagtct ctgctgacac agaagtggtg gggagatatg     420
   gccagcacta cacccatctg ggccctggtt ctcgccttct tttgccctcc actcatctac     480
   acccgcctca tcaccttcag gaaatcagaa gaggagccca cacgggagga gctagagttt     540
   gacatggata gtgtcattaa tggggaaggg cctgtcggga cggcggaccc agccgagaag     600
   acgccgctgg gggtcccgcg ccagtcgggc cgtccgggtt gctgcggggg ccgctgcggg     660
   gggcgccggt gcctacgccg ctggttccac ttctggggcg cgccggtgac catcttcatg     720
   ggcaacgtgg tcagctacct gctgttcctg ctgcttttct cgcgggtgct gctcgtggat     780
   ttccagccgg cgccgcccgg ctccctggag ctgctgctct atttctgggc tttcacgctg     840
   ctgtgcgagg aactgcgcca gggcctgagc ggaggcgggg gcagcctcgc cagcgggggc     900
   cccgggcctg gccatgcctc actgagccag cgcctgcgcc tctacctcgc cgacagctgg     960
   aaccagtgcg acctagtggc tctcacctgc ttcctcctgg gcgtgggctg ccggctgacc    1020
   ccgggtttgt accacctggg ccgcactgtc ctctgcatcg acttcatggt tttcacggtg    1080
   cggctgcttc acatcttcac ggtcaacaaa cagctggggc ccaagatcgt catcgtgagc    1140
   aagatgatga aggacgtgtt cttcttcctc ttcttcctcg gcgtgtggct ggtagcctat    1200
   ggcgtggcca cggaggggct cctgaggcca cgggacagtg acttcccaag tatcctgcgc    1260
   cgcgtcttct accgtcccta cctgcagatc ttcgggcaga ttccccagga ggacatggac    1320
   gtggccctca tggagcacag caactgctcg tcggagcccg gcttctgggc acaccctcct    1380
   ggggcccagg cgggcacctg cgtctcccag tatgccaact ggctggtggt gctgctcctc    1440
   gtcatcttcc tgctcgtggc caacatcctg ctggtcaact tgctcattgc catgttcagt    1500
   tacacattcg gcaaagtaca gggcaacagc gatctctact ggaaggcgca gcgttaccgc    1560
   ctcatccggg aattccactc tcggcccgcg ctggccccgc cctttatcgt catctcccac    1620
   ttgcgcctcc tgctcaggca attgtgcagg cgacccsgga gcccccagcc gtcctccccg    1680
   gccctcgagc atttccgggt ttacctttct aaggaagccg agcggaagct gctaacgtgg    1740
   gaatcggtgc ataaggagaa ctttctgctg gcacgcgcta gggacaagcg ggagagcgac    1800
   tccgagmgtc tgaagcgcac gtcccagaag gtggacttgg cactgaaaca gctgggacac    1860
   atccgcgagt acgaacagcg cctgaaagtg ctggagcggg aggtccagca gtgtacctcg    1920
   gcccccgcac ctggtggcct tgtccttgag gtgagcccca tgtccatctg ggccactgtc    1980
   aggaccacct ttgggagtgt catccttaca aaccacagca tgcccggctc ctcccagaac    2040
   cagtcccagc ctgggaggat caaggcctgg atcccrggcc gttatccatc tggaggctgc    2100
   agggtccttg gggtaacagg gaccacagac ccctcaccac tcacagattc ctcacactgg    2160
   ggaaataaag ccatttcaga                                                2180
<210> 26
   <211> 725
   <212> PRT
   <213> Homo Sapiens
<220>
   <221> UNSURE
   <222> (553)...(553)
   <223> UNKNOWN
<221> UNSURE
   <222> (603)...(603)
   <223> UNKNOWN
<400> 26
   Ser Arg Pro Arg Ile Arg His Glu Gly Leu Gly Gln Ala Pro Trp Ser
   1               5                   10                  15
   Asp Leu Leu Leu Trp Ala Leu Leu Leu Asn Arg Ala Gln Met Ala Met
               20                  25                  30
   Tyr Phe Trp Glu Met Gly Ser Asn Ala Val Ser Ser Ala Leu Gly Ala
           35                  40                  45
   Cys Leu Leu Leu Arg Val Met Ala Arg Leu Glu Pro Asp Ala Glu Glu
       50                  55                  60
   Ala Ala Arg Arg Lys Asp Leu Ala Phe Lys Phe Glu Gly Met Gly Val
   65                  70                  75                  80
   Asp Leu Phe Gly Glu Cys Tyr Arg Ser Ser Glu Val Arg Ala Ala Arg
                   85                  90                  95
   Leu Leu Leu Arg Arg Cys Pro Leu Trp Gly Asp Ala Thr Cys Leu Gln
               100                 105                 110
   Leu Ala Met Gln Ala Asp Ala Arg Ala Phe Phe Ala Gln Asp Gly Val
           115                 120                 125
   Gln Ser Leu Leu Thr Gln Lys Trp Trp Gly Asp Met Ala Ser Thr Thr
       130                 135                 140
   Pro Ile Trp Ala Leu Val Leu Ala Phe Phe Cys Pro Pro Leu Ile Tyr
   145                 150                 155                 160
   Thr Arg Leu Ile Thr Phe Arg Lys Ser Glu Glu Glu Pro Thr Arg Glu
                   165                 170                 175
   Glu Leu Glu Phe Asp Met Asp Ser Val Ile Asn Gly Glu Gly Pro Val
               180                 185                 190
   Gly Thr Ala Asp Pro Ala Glu Lys Thr Pro Leu Gly Val Pro Arg Gln
           195                 200                 205
   Ser Gly Arg Pro Gly Cys Cys Gly Gly Arg Cys Gly Gly Arg Arg Cys
       210                 215                 220
   Leu Arg Arg Trp Phe His Phe Trp Gly Ala Pro Val Thr Ile Phe Met
   225                 230                 235                 240
   Gly Asn Val Val Ser Tyr Leu Leu Phe Leu Leu Leu Phe Ser Arg Val
                   245                 250                 255
   Leu Leu Val Asp Phe Gln Pro Ala Pro Pro Gly Ser Leu Glu Leu Leu
               260                 265                 270
   Leu Tyr Phe Trp Ala Phe Thr Leu Leu Cys Glu Glu Leu Arg Gln Gly
           275                 280                 285
   Leu Ser Gly Gly Gly Gly Ser Leu Ala Ser Gly Gly Pro Gly Pro Gly
       290                 295                 300
   His Ala Ser Leu Ser Gln Arg Leu Arg Leu Tyr Leu Ala Asp Ser Trp
   305                 310                 315                 320
   Asn Gln Cys Asp Leu Val Ala Leu Thr Cys Phe Leu Leu Gly Val Gly
                   325                 330                 335
   Cys Arg Leu Thr Pro Gly Leu Tyr His Leu Gly Arg Thr Val Leu Cys
               340                 345                 350
   Ile Asp Phe Met Val Phe Thr Val Arg Leu Leu His Ile Phe Thr Val
           355                 360                 365
   Asn Lys Gln Leu Gly Pro Lys Ile Val Ile Val Ser Lys Met Met Lys
       370                 375                 380
   Asp Val Phe Phe Phe Leu Phe Phe Leu Gly Val Trp Leu Val Ala Tyr
   385                 390                 395                 400
   Gly Val Ala Thr Glu Gly Leu Leu Arg Pro Arg Asp Ser Asp Phe Pro
                   405                 410                 415
   Ser Ile Leu Arg Arg Val Phe Tyr Arg Pro Tyr Leu Gln Ile Phe Gly
               420                 425                 430
   Gln Ile Pro Gln Glu Asp Met Asp Val Ala Leu Met Glu His Ser Asn
           435                 440                 445
   Cys Ser Ser Glu Pro Gly Phe Trp Ala His Pro Pro Gly Ala Gln Ala
       450                 455                 460
   Gly Thr Cys Val Ser Gln Tyr Ala Asn Trp Leu Val Val Leu Leu Leu
   465                 470                 475                 480
   Val Ile Phe Leu Leu Val Ala Asn Ile Leu Leu Val Asn Leu Leu Ile
                   485                 490                 495
   Ala Met Phe Ser Tyr Thr Phe Gly Lys Val Gln Gly Asn Ser Asp Leu
               500                 505                 510
   Tyr Trp Lys Ala Gln Arg Tyr Arg Leu Ile Arg Glu Phe His Ser Arg
           515                 520                 525
   Pro Ala Leu Ala Pro Pro Phe Ile Val Ile Ser His Leu Arg Leu Leu
       530                 535                 540
   Leu Arg Gln Leu Cys Arg Arg Pro Xaa Ser Pro Gln Pro Ser Ser Pro
   545                 550                 555                 560
   Ala Leu Glu His Phe Arg Val Tyr Leu Ser Lys Glu Ala Glu Arg Lys
                   565                 570                 575
   Leu Leu Thr Trp Glu Ser Val His Lys Glu Asn Phe Leu Leu Ala Arg
               580                 585                 590
   Ala Arg Asp Lys Arg Glu Ser Asp Ser Glu Xaa Leu Lys Arg Thr Ser
           595                 600                 605
   Gln Lys Val Asp Leu Ala Leu Lys Gln Leu Gly His Ile Arg Glu Tyr
       610                 615                 620
   Glu Gln Arg Leu Lys Val Leu Glu Arg Glu Val Gln Gln Cys Thr Ser
   625                 630                 635                 640
   Ala Pro Ala Pro Gly Gly Leu Val Leu Glu Val Ser Pro Met Ser Ile
                   645                 650                 655
   Trp Ala Thr Val Arg Thr Thr Phe Gly Ser Val Ile Leu Thr Asn His
               660                 665                 670
   Ser Met Pro Gly Ser Ser Gln Asn Gln Ser Gln Pro Gly Arg Ile Lys
           675                 680                 685
   Ala Trp Ile Pro Gly Arg Tyr Pro Ser Gly Gly Cys Arg Val Leu Gly
       690                 695                 700
   Val Thr Gly Thr Thr Asp Pro Ser Pro Leu Thr Asp Ser Ser His Trp
   705                 710                 715                 720
   Gly Asn Lys Ala Ile
                   725
<210> 27
   <211> 7419
   <212> DNA
   <213> Homo Sapiens
<400> 27
   cggggaccga tccagcctcc ggactctagc ctaggctttt gcaaaaagct atttaggtga 60
   cactatagaa ggtacgcctg caggtaccgg tccggaattc ccgggtcgac ccacgcgtcc 120
   gcagccccgt cgccggcgga ggcgggcgcg ggcgcgtncc ctgtggccag tcacccggag 180
   gagttggtcg cacaattatg aaagactcgg cttctgctgc tagcgccgga gctgagttag 240
   ttctgagaag gtttccctgg gcgttccttg tccggcggcc tctgctgccg cctccggaga 300
   cgcttcccga tagatggcta caggccgcgg aggaggagga ggtggagttg ctgcccttcc 360
   ggagtccgcc ccgtgaggag aatgtcccag aaatcctgga tagaaagcac tttgaccaag 420
   agggaatgtg tatatattat accaagttcc aaggaccctc acagatgcct tccaggatgt 480
   caaatttgtc agcaactcgt caggtgtttt tgtggtcgct tggtcaagca acatgcttgt 540
   tttactgcaa gtcttgccat gaaatactca gatgtgaaat tgggtgacca ttttaatcag 600
   gcaatagaag aatggtctgt ggaaaagcat acagaacaga gcccaacgga tgcttatgga 660
   gtcataaatt ttcaaggggg ttctcattcc tacagagcta agtatgtgag gctatcatat 720
   gacaccaaac ctgaagtcat tctgcaactt ctgcttaaag aatggcaaat ggagttaccc 780
   aaacttgtta tctctgtaca tgggggcatg cagaaatttg agcttcaccc acgaatcaag 840
   cagttgcttg gaaaaggtct tattaaagct gcagttacaa ctggagcctg gattttaact 900
   ggaggagtaa acacaggtgt ggcaaaacat gttggagatg ccctcaaaga acatgcttcc 960
   agatcatctc gaaagatttg cactatcgga atagctccat ggggagtgat tgaaaacaga 1020
   aatgatcttg ttgggagaga tgtggttgct ccttatcaaa ccttattgaa ccccctgagc 1080
   aaattgaatg ttttgaataa tctgcattcc catttcatat tggtggatga tggcactgtt 1140
   ggaaagtatg gggcggaagt cagactgaga agagaacttg aaaaaactat taatcagcaa 1200
   agaattcatg ctaggattgg ccagggtgtc cctgtggtgg cacttatatt tgagggtggg 1260
   ccaaatgtta tcctcacagt tcttgaatac cttcaggaaa gcccccctgt tccagtagtt 1320
   gtgtgtgaag gaacaggcag agctgcagat ctgctagcgt atattcataa acaaacagaa 1380
   gaaggaggga atcttcctga tgcagcagag cccgatatta tttccactat caaaaaaaca 1440
   tttaactttg gccagaatga agcacttcat ttatttcaaa cactgatgga gtgcatgaaa 1500
   agaaaggagc ttatcactgt tttccatatt gggtcagatg aacatcaaga tatagatgta 1560
   gcaatactta ctgcactgct aaaaggtact aatgcatctg catttgacca gcttatcctt 1620
   acattggcat gggatagagt tgacattgcc aaaaatcatg tatttgttta tggacagcag 1680
   tggctggttg gatccttgga acaagctatg cttgatgctc ttgtaatgga tagagttgca 1740
   tttgtaaaac ttcttattga aaatggagta agcatgcata aattccttac cattccgaga 1800
   ctggaagaac tttacaacac taaacaaggt ccaactaatc caatgctgtt tcatcttgtt 1860
   cgagacgtca aacagggaaa tcttcctcca ggatataaga tcactctgat tgatatagga 1920
   cttgttattg aatatctcat gggaggaacc tacagatgca cctatactag gaaacgtttt 1980
   cgattaatat ataatagtct tggtggaaat aatcggaggt ctggccgaaa tacctccagc 2040
   agcactcctc agttgcgaaa gagtcatgaa tcttttggca atagggcaga taaaaaggaa 2100
   aaaatgaggc ataaccattt cattaagaca gcacagccct tccgaccaaa gattgataca 2160
   gttatggaag aaggaaagaa gaaaagaacc aaagatgaaa ttgtagacat tgatgatcca 2220
   gaaaccaagc gctttcctta tccacttaat gaacttttaa tttgggcttg ccttatgaag 2280
   aggcaggtca tggcccgttt tttatggcaa catggtgaag aatcaatggc taaagcatta 2340
   gttgcctgta agatctatcg ttcaatggca tatgaagcaa agcagagtga cctggtagat 2400
   gatacttcag aagaactaaa acagtattcc aatgattttg gtcagttggc cgttgaatta 2460
   ttagaacagt ccttcagaca agatgaaacc atggctatga aattgctcac ttatgaactg 2520
   aagaactgga gtaattcaac ctgccttaag ttagcagttt cttcaagact tagacctttt 2580
   gtagctcaca cctgtacaca aatgttgtta tctgatatgt ggatgggaag gctgaatatg 2640
   aggaaaaatt cctggtacaa ggtcatacta agcattttag ttccacctgc catattgctg 2700
   ttagagtata aaactaaggc tgaaatgtcc catatcccac aatctcaaga tgctcatcag 2760
   atgacaatgg atgacagcga aaacaacttt cagaacataa cagaagagat ccccatggaa 2820
   gtgtttaaag aagtacggat tttggatagt aatgaaggaa agaatgagat ggagatacaa 2880
   atgaaatcaa aaaagcttcc aattacgcga aagttttatg ccttttatca tgcaccaatt 2940
   gtaaaattct ggtttaacac gttggcatat ttaggatttc tgatgcttta tacatttgtg 3000
   gttcttgtac aaatggaaca gttaccttca gttcaagaat ggattgttat tgcttatatt 3060
   tttacttatg ccattgagaa agtccgtgag atctttatgt ctgaagctgg gaaagtaaac 3120
   cagaagatta aagtatggtt tagtgattac ttcaacatca gtgatacaat tgccataatt 3180
   tctttcttca ttggatttgg actaagattt ggagcaaaat ggaactttgc aaatgcatat 3240
   gataatcatg tttttgtggc tggaagatta atttactgtc ttaacataat attttggtat 3300
   gtgcgtttgc tagattttct agctgtaaat caacaggcag gaccttatgt aatgatgatt 3360
   ggaaaaatgg tggccaatat gttctacatt gtagtgatta tggctcttgt attacttagt 3420
   tttggtgttc ccagaaaggc aatactttat cctcatgaag caccatcttg gactcttgct 3480
   aaagatatag tttttcaccc atactggatg atttttggtg aagtttatgc atacgaaatt 3540
   gatgtgtgtg caaatgattc tgttatccct caaatctgtg gtcctgggac gtggttgact    3600
   ccatttcttc aagcagtcta cctctttgta cagtatatca ttatggttaa tcttcttatt    3660
   gcatttttca acaatgtgta tttacaagtg aaggcaattt ccaatattgt atggaagtac    3720
   cagcgttatc attttattat ggcttatcat gagaaaccag ttctgcctcc tccacttatc     3780
   attcttagcc atatagtttc tctgttttgc tgcatatgta agagaagaaa gaaagataag    3840
   acttccgatg gaccaaaact tttcttaaca gaagaagatc aaaagaaact tcatgatttt    3900
   gaagagcagt gtgttgaaat gtatttcaat gaaaaagatg acaaatttca ttctgggagt    3960
   gaagagagaa ttcgtgtcac ttttgaaaga gtggaacaga tgtgcattca gattaaagaa    4020
   gttggagatc gtgtcaacta cataaaaaga tcattacaat cattagattc tcaaattggc    4080
   catttgcaag atctttcagc cctgacggta gatacattaa aaacactcac tgcccagaaa    4140
   gcgtcggaag ctagcaaagt tcataatgaa atcacacgag aactgagcat ttccaaacac    4200
   ttggctcaaa accttattga tgatggtcct gtaagacctt ctgtatggaa aaagcatggt    4260
   gttgtaaata cacttagctc ctctcttcct caaggtgatc ttgaaagtaa taatcctttt    4320
   cattgtaata ttttaatgaa agatgacaaa gatccccagt gtaatatatt tggtcaagac    4380
   ttacctgcag taccccagag aaaagaattt aattttccag aggctggttc ctcttctggt     4440
   gccttattcc caagtgctgt ttcccctcca gaactgcgac agagactaca tggggtagaa    4500
   ctcttaaaaa tatttaataa aaatcaaaaa ttaggcagtt catctactag cataccacat    4560
   ctgtcatccc caccaaccaa attttttgtt agtacaccat ctcagccaag ttgcaaaagc    4620
   cacttggaaa ctggaaccaa agatcaagaa actgtttgct ctaaagctac agaaggagat    4680
   aatacagaat ttggagcatt tgtaggacac agagatagca tggatttaca gaggtttaaa    4740
   gaaacatcaa acaagataaa aatactatcc aataacaata cttctgaaaa cactttgaaa    4800
   cgagtgagtt ctcttgctgg atttactgac tgtcacagaa cttccattcc tgttcattca    4860
   aaacaagcag aaaaaatcag tagaaggcca tctaccgaag acactcatga agtagattcc    4920
   aaagcagctt taataccgga ttggttacaa gatagaccat caaacagaga aatgccatct     4980
   gaagaaggaa cattaaatgg tctcacttct ccatttaagc cagctatgga tacaaattac    5040
   tattattcag ctgtggaaag aaataacttg atgaggttat cacagagcat tccatttaca    5100
   cctgtgcctc caagagggga gcctgtcaca gtgtatcgtt tggaagagag ttcacccaac    5160
   atactaaata acagcatgtc ttcttggtca caactaggcc tctgtgccaa aatagagttt    5220
   ttaagcaaag aggagatggg aggaggttta cgaagagctg tcaaagtaca gtgtacctgg    5280
   tcagaacatg atatcctcaa atcagggcat ctttatatta tcaaatcttt tcttccagag    5340
   gtggttaata catggtcaag tatttataaa gaagatacag ttctgcatct ctgtctgaga    5400
   gaaattcaac aacagagagc agcacaaaag cttacgtttg cctttaatca aatgaaaccc    5460
   aaatccatac catattctcc aaggttcctt gaagttttcc tgctgtattg ccattcagca    5520
   ggacagtggt ttgctgtgga agaatgtatg actggagaat ttagaaaata caacaataat    5580
   aatggagatg agattattcc aactaatact ctggaagaga tcatgctagc ctttagccac    5640
   tggacttacg aatatacaag aggggagtta ctggtacttg atttgcaagg tgttggtgaa    5700
   aatttgactg acccatctgt gataaaagca gaagaaaaga gatcctgtga tatggttttt    5760
   ggcccagcaa atctaggaga agatgcaatt aaaaacttca gagcaaaaca tcactgtaat    5820
   tcttgctgta gaaagcttaa acttccagat ctgaagagga atgattatac gcctgataaa    5880
   attatatttc ctcaggatga gccttcagat ttgaatcttc agcctggaaa ttccaccaaa    5940
   gaatcagaat caactaattc tgttcgtctg atgttataat attaatatta ctgaatcatt    6000
   ggttttgcct gcacctcaca gaaatgttac tgtgtcactt ttccctcggg aggaaattgt    6060
   ttggtaatat agaaaggtgt atgcaagttg aatttgctga ctccagcaca gttaaaaggt    6120
   caatattctt ttgacctgat taatcagtca gaaagtccct ataggataga gctggcagct    6180
   gagaaatttt aaaggtaatt gataattagt atttgtaact ttttaaaggg ctctttgtat     6240
   agcagaggat ctcatttgac tttgttttga tgagggtgat gccctctctt atgtggtaca    6300
   ataccattaa ccaaaggtag gtgtccatgc agattttatt ggcagctgtt ttattgccat    6360
   tcaactaggg aaatgaagaa atcacgcagc cttttggtta aatggcagtc aaaattttcc    6420
   tcagtgtatt tagtgtgttc agtgatgata tcactggttc ccaactagat gcttgttggc    6480
   cacgggaagg gaaatgactt gttctaattc taggttcaca gaggtatgag aagcctgaac    6540
   tgaagaccat tttcaagagg gacggtattt atgaatcagg gttaggctcc atatttaaag    6600
   atagagccag tttttttttt aaatagaacc caaattgtgt aaaaatgtta attgggtttt    6660
   ttaaacattg ttttatcaag tcactgttaa gtagaagaaa gccatggtaa actgatacat    6720
   aacctaaatt ataaaagcag aaacctaact cactcgtcaa gggaagttac cttttgagga    6780
   aagttaaagt acttttttcc ctatctgtat ctatagcaac aacccagaac ttacaaactt   6840
   ctccaaagat tttattgatt gttatatcaa atcagaatgt aaacatgaac tcttgcatat    6900
   atttaaaatt gtgttggaac atttgaacat gaatgctgtt tgggtactta agaaattrat    6960
   tcagtnggat tatcattatg tganactggc agattgcagt gcanccttat gccaataaaa    7020
   tgtaatttar cagccccaga tattgttgaa tattcaacaa taacaagaaa agcttttcat    7080
   ctaagtttta tgctttaatt ttttttcttt ttttttcttt ttcttttgtt tccttggtac      7140
   taattttaat ttttatttgg aagggagcag tataaagctt atttgtattt agtagtgtat    7200
   ctcatagata cagacaaggc aagagatgat aagctgttta aatagtgktt aatattgatt    7260
   gggggtgggg agaaagaaaa agtgtattac ttaaagatac tatatacskt ttktatatca    7320
   ttaaatcttt aaaagaaatn naataaattt attgtttnca aaaaaaaaac ccnntaaaaa    7380
   aaaaagggcg gcccctctag aggatccctc gaggggccc                           7419
<210> 28
   <211> 1865
   <212> PRT
   <213> Homo Sapiens
<400> 28
   Met Ser Gln Lys Ser Trp Ile Glu Ser Thr Leu Thr Lys Arg Glu Cys
   1               5                   10                  15
   Val Tyr Ile Ile Pro Ser Ser Lys Asp Pro His Arg Cys Leu Pro Gly
               20                  25                  30
   Cys Gln Ile Cys Gln Gln Leu Val Arg Cys Phe Cys Gly Arg Leu Val
           35                  40                  45
   Lys Gln His Ala Cys Phe Thr Ala Ser Leu Ala Met Lys Tyr Ser Asp
       50                  55                  60
   Val Lys Leu Gly Asp His Phe Asn Gln Ala Ile Glu Glu Trp Ser Val
   65                  70                  75                  80
   Glu Lys His Thr Glu Gln Ser Pro Thr Asp Ala Tyr Gly Val Ile Asn
                   85                  90                  95
   Phe Gln Gly Gly Ser His Ser Tyr Arg Ala Lys Tyr Val Arg Leu Ser
               100                 105                 110
   Tyr Asp Thr Lys Pro Glu Val Ile Leu Gln Leu Leu Leu Lys Glu Trp
           115                 120                 125
   Gln Met Glu Leu Pro Lys Leu Val Ile Ser Val His Gly Gly Met Gln
       130                 135                 140
   Lys Phe Glu Leu His Pro Arg Ile Lys Gln Leu Leu Gly Lys Gly Leu
   145                 150                 155                 160
   Ile Lys Ala Ala Val Thr Thr Gly Ala Trp Ile Leu Thr Gly Gly Val
                   165                 170                 175
   Asn Thr Gly Val Ala Lys His Val Gly Asp Ala Leu Lys Glu His Ala
               180                 185                 190
   Ser Arg Ser Ser Arg Lys Ile Cys Thr Ile Gly Ile Ala Pro Trp Gly
           195                 200                 205
   Val Ile Glu Asn Arg Asn Asp Leu Val Gly Arg Asp Val Val Ala Pro
       210                 215                 220
   Tyr Gln Thr Leu Leu Asn Pro Leu Ser Lys Leu Asn Val Leu Asn Asn
   225                 230                 235                 240
   Leu His Ser His Phe Ile Leu Val Asp Asp Gly Thr Val Gly Lys Tyr
                   245                 250                 255
   Gly Ala Glu Val Arg Leu Arg Arg Glu Leu Glu Lys Thr Ile Asn Gln
               260                 265                 270
   Gln Arg Ile His Ala Arg Ile Gly Gln Gly Val Pro Val Val Ala Leu
           275                 280                 285
   Ile Phe Glu Gly Gly Pro Asn Val Ile Leu Thr Val Leu Glu Tyr Leu
       290                 295                 300
   Gln Glu Ser Pro Pro Val Pro Val Val Val Cys Glu Gly Thr Gly Arg
   305                 310                 315                 320
   Ala Ala Asp Leu Leu Ala Tyr Ile His Lys Gln Thr Glu Glu Gly Gly
                   325                 330                 335
   Asn Leu Pro Asp Ala Ala Glu Pro Asp Ile Ile Ser Thr Ile Lys Lys
               340                 345                 350
   Thr Phe Asn Phe Gly Gln Asn Glu Ala Leu His Leu Phe Gln Thr Leu
           355                 360                 365
   Met Glu Cys Met Lys Arg Lys Glu Leu Ile Thr Val Phe His Ile Gly
   370 375 380
   Ser Asp Glu His Gln Asp Ile Asp Val Ala Ile Leu Thr Ala Leu Leu
   385 390 395 400
   Lys Gly Thr Asn Ala Ser Ala Phe Asp Gln Leu Ile Leu Thr Leu Ala
   405 410 415
   Trp Asp Arg Val Asp Ile Ala Lys Asn His Val Phe Val Tyr Gly Gln
   420 425 430
   Gln Trp Leu Val Gly Ser Leu Glu Gln Ala Met Leu Asp Ala Leu Val
   435 440 445
   Met Asp Arg Val Ala Phe Val Lys Leu Leu Ile Glu Asn Gly Val Ser
   450 455 460
   Met His Lys Phe Leu Thr Ile Pro Arg Leu Glu Glu Leu Tyr Asn Thr
   465 470 475 480
   Lys Gln Gly Pro Thr Asn Pro Met Leu Phe His Leu Val Arg Asp Val
   485 490 495
   Lys Gln Gly Asn Leu Pro Pro Gly Tyr Lys Ile Thr Leu Ile Asp Ile
   500 505 510
   Gly Leu Val Ile Glu Tyr Leu Met Gly Gly Thr Tyr Arg Cys Thr Tyr
   515 520 525
   Thr Arg Lys Arg Phe Arg Leu Ile Tyr Asn Ser Leu Gly Gly Asn Asn
   530 535 540
   Arg Arg Ser Gly Arg Asn Thr Ser Ser Ser Thr Pro Gln Leu Arg Lys
   545 550 555 560
   Ser His Glu Ser Phe Gly Asn Arg Ala Asp Lys Lys Glu Lys Met Arg
   565 570 575
   His Asn His Phe Ile Lys Thr Ala Gln Pro Phe Arg Pro Lys Ile Asp
   580 585 590
   Thr Val Met Glu Glu Gly Lys Lys Lys Arg Thr Lys Asp Glu Ile Val
   595 600 605
   Asp Ile Asp Asp Pro Glu Thr Lys Arg Phe Pro Tyr Pro Leu Asn Glu
   610 615 620
   Leu Leu Ile Trp Ala Cys Leu Met Lys Arg Gln Val Met Ala Arg Phe
   625 630 635 640
   Leu Trp Gln His Gly Glu Glu Ser Met Ala Lys Ala Leu Val Ala Cys
   645 650 655
   Lys Ile Tyr Arg Ser Met Ala Tyr Glu Ala Lys Gln Ser Asp Leu Val
   660 665 670
   Asp Asp Thr Ser Glu Glu Leu Lys Gln Tyr Ser Asn Asp Phe Gly Gln
   675 680 685
   Leu Ala Val Glu Leu Leu Glu Gln Ser Phe Arg Gln Asp Glu Thr Met
   690 695 700
   Ala Met Lys Leu Leu Thr Tyr Glu Leu Lys Asn Trp Ser Asn Ser Thr
   705 710 715 720
   Cys Leu Lys Leu Ala Val Ser Ser Arg Leu Arg Pro Phe Val Ala His
   725 730 735
   Thr Cys Thr Gln Met Leu Leu Ser Asp Met Trp Met Gly Arg Leu Asn
   740 745 750
   Met Arg Lys Asn Ser Trp Tyr Lys Val Ile Leu Ser Ile Leu Val Pro
   755 760 765
   Pro Ala Ile Leu Leu Leu Glu Tyr Lys Thr Lys Ala Glu Met Ser His
   770 775 780
   Ile Pro Gln Ser Gln Asp Ala His Gln Met Thr Met Asp Asp Ser Glu
   785 790 795 800
   Asn Asn Phe Gln Asn Ile Thr Glu Glu Ile Pro Met Glu Val Phe Lys
   805 810 815
   Glu Val Arg Ile Leu Asp Ser Asn Glu Gly Lys Asn Glu Met Glu Ile
   820 825 830
   Gln Met Lys Ser Lys Lys Leu Pro Ile Thr Arg Lys Phe Tyr Ala Phe
   835 840 845
   Tyr His Ala Pro Ile Val Lys Phe Trp Phe Asn Thr Leu Ala Tyr Leu
   850 855 860
   Gly Phe Leu Met Leu Tyr Thr Phe Val Val Leu Val Gln Met Glu Gln
   865 870 875 880
   Leu Pro Ser Val Gln Glu Trp Ile Val Ile Ala Tyr Ile Phe Thr Tyr
   885 890 895
   Ala Ile Glu Lys Val Arg Glu Ile Phe Met Ser Glu Ala Gly Lys Val
   900 905 910
   Asn Gln Lys Ile Lys Val Trp Phe Ser Asp Tyr Phe Asn Ile Ser Asp
   915 920 925
   Thr Ile Ala Ile Ile Ser Phe Phe Ile Gly Phe Gly Leu Arg Phe Gly
   930 935 940
   Ala Lys Trp Asn Phe Ala Asn Ala Tyr Asp Asn His Val Phe Val Ala
   945 950 955 960
   Gly Arg Leu Ile Tyr Cys Leu Asn Ile Ile Phe Trp Tyr Val Arg Leu
   965 970 975
   Leu Asp Phe Leu Ala Val Asn Gln Gln Ala Gly Pro Tyr Val Met Met
   980 985 990
   Ile Gly Lys Met Val Ala Asn Met Phe Tyr Ile Val Val Ile Met Ala
   995 1000 1005
   Leu Val Leu Leu Ser Phe Gly Val Pro Arg Lys Ala Ile Leu Tyr Pro
   1010 1015 1020
   His Glu Ala Pro Ser Trp Thr Leu Ala Lys Asp Ile Val Phe His Pro
   1025 1030 1035 1040
   Tyr Trp Met Ile Phe Gly Glu Val Tyr Ala Tyr Glu Ile Asp Val Cys
   1045 1050 1055
   Ala Asn Asp Ser Val Ile Pro Gln Ile Cys Gly Pro Gly Thr Trp Leu
   1060 1065 1070
   Thr Pro Phe Leu Gln Ala Val Tyr Leu Phe Val Gln Tyr Ile Ile Met
   1075 1080 1085
   Val Asn Leu Leu Ile Ala Phe Phe Asn Asn Val Tyr Leu Gln Val Lys
   1090 1095 1100
   Ala Ile Ser Asn Ile Val Trp Lys Tyr Gln Arg Tyr His Phe Ile Met
   1105 1110 1115 1120
   Ala Tyr His Glu Lys Pro Val Leu Pro Pro Pro Leu Ile Ile Leu Ser
   1125 1130 1135
   His Ile Val Ser Leu Phe Cys Cys Ile Cys Lys Arg Arg Lys Lys Asp
   1140 1145 1150
   Lys Thr Ser Asp Gly Pro Lys Leu Phe Leu Thr Glu Glu Asp Gln Lys
   1155 1160 1165
   Lys Leu His Asp Phe Glu Glu Gln Cys Val Glu Met Tyr Phe Asn Glu
   1170 1175 1180
   Lys Asp Asp Lys Phe His Ser Gly Ser Glu Glu Arg Ile Arg Val Thr
   1185 1190 1195 1200
   Phe Glu Arg Val Glu Gln Met Cys Ile Gln Ile Lys Glu Val Gly Asp
   1205 1210 1215
   Arg Val Asn Tyr Ile Lys Arg Ser Leu Gln Ser Leu Asp Ser Gln Ile
   1220 1225 1230
   Gly His Leu Gln Asp Leu Ser Ala Leu Thr Val Asp Thr Leu Lys Thr
   1235 1240 1245
   Leu Thr Ala Gln Lys Ala Ser Glu Ala Ser Lys Val His Asn Glu Ile
   1250 1255 1260
   Thr Arg Glu Leu Ser Ile Ser Lys His Leu Ala Gln Asn Leu Ile Asp
   1265 1270 1275 1280
   Asp Gly Pro Val Arg Pro Ser Val Trp Lys Lys His Gly Val Val Asn
   1285 1290 1295
   Thr Leu Ser Ser Ser Leu Pro Gln Gly Asp Leu Glu Ser Asn Asn Pro
   1300 1305 1310
   Phe His Cys Asn Ile Leu Met Lys Asp Asp Lys Asp Pro Gln Cys Asn
   1315 1320 1325
   Ile Phe Gly Gln Asp Leu Pro Ala Val Pro Gln Arg Lys Glu Phe Asn
       1330                1335                1340
   Phe Pro Glu Ala Gly Ser Ser Ser Gly Ala Leu Phe Pro Ser Ala Val
   1345                1350                1355                1360
   Ser Pro Pro Glu Leu Arg Gln Arg Leu His Gly Val Glu Leu Leu Lys
                   1365                1370                1375
   Ile Phe Asn Lys Asn Gln Lys Leu Gly Ser Ser Ser Thr Ser Ile Pro
               1380                1385                1390
   His Leu Ser Ser Pro Pro Thr Lys Phe Phe Val Ser Thr Pro Ser Gln
           1395                1400                1405
   Pro Ser Cys Lys Ser His Leu Glu Thr Gly Thr Lys Asp Gln Glu Thr
       1410                1415                1420
   Val Cys Ser Lys Ala Thr Glu Gly Asp Asn Thr Glu Phe Gly Ala Phe
   1425                1430                1435                1440
   Val Gly His Arg Asp Ser Met Asp Leu Gln Arg Phe Lys Glu Thr Ser
                   1445                1450                1455
   Asn Lys Ile Lys Ile Leu Ser Asn Asn Asn Thr Ser Glu Asn Thr Leu
               1460                1465                1470
   Lys Arg Val Ser Ser Leu Ala Gly Phe Thr Asp Cys His Arg Thr Ser
           1475                1480                1485
   Ile Pro Val His Ser Lys Gln Ala Glu Lys Ile Ser Arg Arg Pro Ser
       1490                1495                1500
   Thr Glu Asp Thr His Glu Val Asp Ser Lys Ala Ala Leu Ile Pro Asp
   1505                1510                1515                1520
   Trp Leu Gln Asp Arg Pro Ser Asn Arg Glu Met Pro Ser Glu Glu Gly
                   1525                1530                1535
   Thr Leu Asn Gly Leu Thr Ser Pro Phe Lys Pro Ala Met Asp Thr Asn
               1540                1545                1550
   Tyr Tyr Tyr Ser Ala Val Glu Arg Asn Asn Leu Met Arg Leu Ser Gln
           1555                1560                1565
   Ser Ile Pro Phe Thr Pro Val Pro Pro Arg Gly Glu Pro Val Thr Val
       1570                1575                1580
   Tyr Arg Leu Glu Glu Ser Ser Pro Asn Ile Leu Asn Asn Ser Met Ser
   1585                1590                1595                1600
   Ser Trp Ser Gln Leu Gly Leu Cys Ala Lys Ile Glu Phe Leu Ser Lys
                   1605                1610                1615
   Glu Glu Met Gly Gly Gly Leu Arg Arg Ala Val Lys Val Gln Cys Thr
               1620                1625                1630
   Trp Ser Glu His Asp Ile Leu Lys Ser Gly His Leu Tyr Ile Ile Lys
           1635                1640                1645
   Ser Phe Leu Pro Glu Val Val Asn Thr Trp Ser Ser Ile Tyr Lys Glu
       1650                1655                1660
   Asp Thr Val Leu His Leu Cys Leu Arg Glu Ile Gln Gln Gln Arg Ala
   1665                1670                1675                1680
   Ala Gln Lys Leu Thr Phe Ala Phe Asn Gln Met Lys Pro Lys Ser Ile
                   1685                1690                1695
   Pro Tyr Ser Pro Arg Phe Leu Glu Val Phe Leu Leu Tyr Cys His Ser
               1700                1705                1710
   Ala Gly Gln Trp Phe Ala Val Glu Glu Cys Met Thr Gly Glu Phe Arg
           1715                1720                1725
   Lys Tyr Asn Asn Asn Asn Gly Asp Glu Ile Ile Pro Thr Asn Thr Leu
       1730                1735                1740
   Glu Glu Ile Met Leu Ala Phe Ser His Trp Thr Tyr Glu Tyr Thr Arg
   1745                1750                1755                1760
   Gly Glu Leu Leu Val Leu Asp Leu Gln Gly Val Gly Glu Asn Leu Thr
                   1765                1770                1775
   Asp Pro Ser Val Ile Lys Ala Glu Glu Lys Arg Ser Cys Asp Met Val
               1780                1785                1790
   Phe Gly Pro Ala Asn Leu Gly Glu Asp Ala Ile Lys Asn Phe Arg Ala
           1795                1800                1805
   Lys His His Cys Asn Ser Cys Cys Arg Lys Leu Lys Leu Pro Asp Leu
       1810                1815                1820
   Lys Arg Asn Asp Tyr Thr Pro Asp Lys Ile Ile Phe Pro Gln Asp Glu
   1825                1830                1835                1840
   Pro Ser Asp Leu Asn Leu Gln Pro Gly Asn Ser Thr Lys Glu Ser Glu
                   1845                1850                1855
   Ser Thr Asn Ser Val Arg Leu Met Leu
               1860                1865
<210> 29
   <211> 4061
   <212> DNA
   <213> Homo Sapiens
<400> 29
<210> 30
   <211> 1214
   <212> PRT
   <213> Homo Sapiens
<400> 30
<210> 31
   <211> 4646
   <212> DNA
   <213> Homo Sapiens
<400> 31
   tcgacccacg cgtccgccca cgcgtccgcc cacgcgtccg cccacgcgtc cgcccacgcg 60
   tccgcccacg cgtccggggt gaaagmramy cmygcktsms aaaaaccgtc acttaggaaa 120
   agatgtcctt tcgggcagcc aggctcagca tgaggaacag aaggaatgac actctggaca 180
   gcacccggac cctgtactcc agcgcgtctc ggagcacaga cttgtcttac agtgaaagcg 240
   acttggtgaa ttttattcaa gcaaatttta agaaacgaga atgtgtcttc tttaccaaag 300
   attccaaggc cacggagaat gtgtgcaagt gtggctatgc ccagagccag cacatggaag 360
   gcacccagat caaccaaagt gagaaatgga actacaagaa acacaccaag gaatttccta 420
   ccgacgcctt tggggatatt cagtttgaga cactggggaa gaaagggaag tatatacgtc 480
   tgtcctgcga cacggacgcg gaaatccttt acgagctgct gacccagcac tggcacctga 540
   aaacacccaa cctggtcatt tctgtgaccg ggggcgccaa gaacttcgcc ctgaagccgc 600
   gcatgcgcaa gatcttcagc cggctcatct acatcgcgca gtccaaaggt gcttggattc 660
   tcacgggagg cacccattat ggcctgatga agtacatcgg ggaggtggtg agagataaca 720
   ccatcagcag gagttcagag gagaatattg tggccattgg catagcagct tggggcatgg 780
   tctccaaccg ggacaccctc atcaggaatt gcgatgctga gggctatttt ttagcccagt 840
   accttatgga tgacttcaca agagatccac tgtgtatcct ggacaacaac cacacacatt 900
   tgctgctcgt ggacaatggc tgtcatggac atcccactgt cgaagcaaag ctccggaatc 960
   agctagagaa gtatatctct gagcgcacta ttcaagattc caactatggt ggcaagatcc 1020
   ccattgtgtg ttttgcccaa ggaggtggaa aagagacttt gaaagccatc aatacctcca 1080
   tcaaaaataa aattccttgt gtggtggtgg aaggctcggg ccagatcgct gatgtgatcg 1140
   ctagcctggt ggaggtggag gatgccctga catcttctgc cgtcaaggag aagctggtgc 1200
   gctttttacc ccgcacggtg tcccggctgc ctgaggagga gactgagagt tggatcaaat 1260
   ggctcaaaga aattctcgaa tgttctcacc tattaacagt tattaaaatg gaagaagctg 1320
   gggatgaaat tgtgagcaat gccatctcct acgctctata caaagccttc agcaccagtg 1380
   agcaagacaa ggataactgg aatgggcagc tgaagcttct gctggagtgg aaccagctgg 1440
   acttagccaa tgatgagatt ttcaccaatg accgccgatg ggagtctgct gaccttcaag 1500
   aagtcatgtt tacggctctc ataaaggaca gacccaagtt tgtccgcctc tttctggaga 1560
   atggcttgaa cctacggaag tttctcaccc atgatgtcct cactgaactc ttctccaacc 1620
   acttcagcac gcttgtgtac cggaatctgc agatcgccaa gaattcctat aatgatgccc 1680
   tcctcacgtt tgtctggaaa ctggttgcga acttccgaag aggcttccgg aaggaagaca 1740
   gaaatggccg ggacgagatg gacatagaac tccacgacgt gtctcctatt actcggcacc 1800
   ccctgcaagc tctcttcatc tgggccattc ttcagaataa gaaggaactc tccaaagtca 1860
   tttgggagca gaccaggggc tgcactctgg cagccctggg agccagcaag cttctgaaga 1920
   ctctggccaa agtgaagaac gacatcaatg ctgctgggga gtccgaggag ctggctaatg 1980
   agtacgagac ccgggctgtt gagctgttca ctgagtgtta cagcagcgat gaagacttgg 2040
   cagaacagct gctggtctat tcctgtgaag cttggggtgg aagcaactgt ctggagctgg 2100
   cggtggaggc cacagaccag catttcatcg cccagcctgg ggtccagaat tttctttcta 2160
   agcaatggta tggagagatt tcccgagaca ccaagaactg gaagattatc ctgtgtctgt 2220
   ttattatacc cttggtgggc tgtggctttg tatcatttag gaagaaacct gtcgacaagc 2280
   acaagaagct gctttggtac tatgtggcgt tcttcacctc cccttcgtgg gtcttctcct 2340
   ggaatgtggt cttctacatc gccttcctcc tgctgtttgc ctacgtgctg ctcatggatt 2400
   tccattcggt gccacacccc cccgagctgg tcctgtactc gctggtcttt gtcctcttct 2460
   gtgatgaagt gagacagtgg tacgtaaatg gggtgaatta ttttactgac ctgtggaatg 2520
   tgatggacac gctggggctt ttttacttca tagcaggaat tgtatttcgg ctccactctt 2580
   ctaataaaag ctctttgtat tctggacgag tcattttctg tctggactac attattttca 2640
   ctctaagatt gatccacatt tttactgtaa gcagaaactt aggacccaag attataatgc 2700
   tgcagaggat gctgatcgat gtgttcttct tcctgttcct ctttgcggtg tggatggtgg 2760
   cctttggcgt ggccaggcaa gggatcctta ggcagaatga gcagcgctgg aggtggatat 2820
   tccgttcggt catctacgag ccctacctgg ccatgttcgg ccaggtgccc agtgacgtgg 2880
   atggtaccac gtatgacttt gcccactgca ccttcactgg gaatgagtcc aagccactgt 2940
   gtgtggagct ggatgagcac aacctgcccc ggttccccga gtggatcacc atccccctgg 3000
   tgtgcatcta catgttatcc accaacatcc tgctggtcaa cctgctggtc gccatgtttg 3060
   gctacacggt gggcaccgtc caggagaaca atgaccaggt ctggaagttc cagaggtact    3120
   tcctggtgca ggagtactgc agccgcctca atatcccctt ccccttcatc gtcttcgctt    3180
   acttctacat ggtggtgaag aagtgcttca agtgttgctg caaggagaaa aacatggagt    3240
   cttctgtctg ctgtttcaaa aatgaagaca atgagactct ggcatgggag ggtgtcatga    3300
   aggaaaacta ccttgtcaag atcaacacaa aagccaacga cacctcagag gaaatgaggc    3360
   atcgatttag acaactggat acaaagctta atgatctcaa gggtcttctg aaagagattg    3420
   ctaataaaat caaataaaac tgtatgaact ctaatggaga aaaatctaat tatagcaaga    3480
   tcatattaag gaatgctgat gaacaatttt gctatcgact actaaatgag agattttcag    3540
   acccctgggt acatggtgga tgattttaaa tcaccctagt gtgctgagac cttgagaata    3600
   aagtgtgtga ttggtttcat acttgaagac ggatataaag gaagaatatt tcctttatgt    3660
   gtttctccag aatggtgcct gtttctctct gtgtctcaat gcctgggact ggaggttgat    3720
   agtttaagtg tgttcttacc gcctcctttt tcctttaatc ttatttttga tgaacacata    3780
   tataggagaa catctatcct atgaataaga acctggtcat gctttactcc tgtattgtta    3840
   ttttgttcat ttccaattga ttctctactt ttcccttttt tgtattatgt gactaattag    3900
   ttggcatatt gtwaaaagtc tctcaaatta ggccagattc taaaacatgc tgcagcaaga    3960
   ggaccccgct ctcttcagga aaagtgtttt catttctcag gatgcttctt acctgtcaga    4020
   ggaggtgaca aggcagtctc ttgctctctt ggactcacca ggctcctatt gaaggaacca    4080
   cccccattcc taaatatgtg aaaagtcgcc caaaatgcaa ccttgaaagg cactactgac    4140
   tttgttctta ttggatactc ctcttattta ttatttttcc attaaaaata atagctggct    4200
   attatagaaa atttagacca tacagagatg tagaaagaac ataaattgtc cccattacct    4260
   taaggtaatc actgctaaca atttctggat ggtttttcaa gtctattttt tttctatgta    4320
   tgtctcaatt ctctttcaaa attttacaga atgttatcat actacatata tactttttat    4380
   gtaagctttt tcacttagta ttttatcaaa tatgttttta ttatattcat agccttctta    4440
   aacattatat caataattgc ataataggca acctctagcg attaccataa ttttgctcat    4500
   tgaaggctat ctccagttga tcattgggat gagcatcttt gtgcatgaat cctattgctg    4560
   tatttgggaa aattttccaa ggttagattc caataaatat ctatttatta ttaaaaaaaa    4620
   aaaaaaaagg gcggccgctc tagagt                                         4646
<210> 32
   <211> 1104
   <212> PRT
   <213> Homo Sapiens
<400> 32